# EUROPEAN PATENT SPECIFICATION

(11) **EP 2 921 545 B1**
(45) Date of publication and mention of the grant of the patent: **21.08.2019**
(21) Application number: 15000075.0
(22) Date of filing: 14.01.2015
(51) Int. Cl.: C09K 19/32, C09K 19/34, C09K 19/04, C09K 19/12, C09K 19/30

(54) **POLYMERISABLE COMPOUNDS AND THE USE THEREOF IN LIQUID-CRYSTAL DISPLAYS**
POLYMERISIERBARE VERBINDUNGEN UND DEREN VERWENDUNG IN FLÜSSIGKRISTALLANZEIGEN
COMPOSÉS POLYMÉRISABLES ET LEUR UTILISATION DANS DES AFFICHAGES À CRISTAUX LIQUIDES

(30) Priority: 21.03.2014 EP 14001061
(43) Date of publication of application: 23.09.2015
(73) Proprietor: Merck Patent GmbH, 64293 Darmstadt (DE)
(72) Inventor: Baron, Eveline, 64285 Darmstadt (DE); Vogt, Julian, 64347 Griesheim (DE); Tong, Qiong, 64291 Darmstadt (DE); Brocke, Constanze, 64521 Gross-Gerau (DE); Haas, Helga, 68623 Lampertheim (DE); Hahn, Alexander, 64584 Biebesheim (DE)

(56) References cited:
- WO-A1-2013/077343
- WO-A1-2013/178333
- WO-A1-2014/079517
- US-A1- 2006 172 090
- US-A1- 2007 284 556
- FRANK HENTRICH ET AL: "Molecular design of amphotropic materials: double-headed diol-based mesogens incorporating rigid structural units", JOURNAL OF MATERIALS CHEMISTRY, vol. 4, no. 10, 1 January 1994 (1994-01-01), pages 1547-1558, XP055229177, GB ISSN: 0959-9428, DOI: 10.1039/jm9940401547

## Description

The present invention relates to polymerisable compounds, to processes and intermediates for the preparation thereof, to liquid-crystal (LC) media comprising them, and to the use of the polymerisable compounds and LC media for optical, electro-optical and electronic purposes, in particular in LC displays, especially in LC displays of the polymer sustained alignment type.

### Background of the Invention

The liquid-crystal displays (LC displays) used at present are usually those of the TN ("twisted nematic") type. However, these have the disadvantage of a strong viewing-angle dependence of the contrast. In addition, so-called VA ("vertically aligned") displays are known which have a broader viewing angle. The LC cell of a VA display contains a layer of an LC medium between two transparent electrodes, where the LC medium usually has a negative dielectric anisotropy. In the switched-off state, the molecules of the LC layer are aligned perpendicular to the electrode surfaces (homeotropically) or have a tilted homeotropic alignment. On application of an electrical voltage to the two electrodes, a realignment of the LC molecules parallel to the electrode surfaces takes place.

Furthermore, OCB ("optically compensated bend") displays are known which are based on a birefringence effect and have an LC layer with a so-called "bend" alignment and usually positive dielectric anisotropy. On application of an electrical voltage, a realignment of the LC molecules perpendicular to the electrode surfaces takes place. In addition, OCB displays normally contain one or more birefringent optical retardation films in order to prevent undesired transparency to light of the bend cell in the dark state. OCB displays have a broader viewing angle and shorter response times compared with TN displays.

Also known are so-called IPS ("in-plane switching") displays, which contain an LC layer between two substrates, where the two electrodes are arranged on only one of the two substrates and preferably have intermeshed, comb-shaped structures. On application of a voltage to the electrodes, an electric field which has a significant component parallel to the LC layer is thereby generated between them. This causes realignment of the LC molecules in the layer plane.

Furthermore, so-called FFS ("fringe-field switching") displays have been reported (see, inter alia, S.H. Jung et al., Jpn. J. Appl. Phys., Volume 43, No. 3, 2004, 1028), which contain two electrodes on the same substrate, one of which structured in a comb-shaped manner and the other is unstructured. A strong, so-called "fringe field" is thereby generated, i.e. a strong electric field close to the edge of the electrodes, and, throughout the cell, an electric field which has both a strong vertical component and also a strong horizontal component. FFS displays have a low viewing-angle dependence of the contrast. FFS displays usually contain an LC medium with positive dielectric anisotropy, and an alignment layer, usually of polyimide, which provides planar alignment to the molecules of the LC medium.

FFS displays can be operated as active-matrix or passive-matrix displays. In the case of active-matrix displays, individual pixels are usually addressed by integrated, non-linear active elements, such as, for example, transistors (for example thin-film transistors ("TFTs")), while in the case of passive-matrix displays, individual pixels are usually addressed by the multiplex method, as known from the prior art.

Furthermore, FFS displays have been disclosed (see S.H. Lee et al., Appl. Phys. Lett. 73(20), 1998, 2882-2883 and S.H. Lee et al., Liquid Crystals 39(9), 2012, 1141-1148), which have similar electrode design and layer thickness as FFS displays, but comprise a layer of an LC medium with negative dielectric anisotropy instead of an LC medium with positive dielectric anisotropy. The LC medium with negative dielectric ansiotropy shows a more favourable director orientation that has less tilt and more twist orientation compared to the LC medium with positive dielectric anisotropy, as a result of which these displays have a higher transmission. The displays further comprise an alignment layer, preferably of polyimide provided on at least one of the substrates that is in contact with the LC medium and induces planar alignment of the LC molecules of the LC medium. These displays are also known as "Ultra Brightness FFS (UB-FFS)" mode displays. These displays require an LC medium with high reliability.

The term "reliability" as used hereinafter means the quality of the performance of the display during time and with different stress loads, such as light load, temperature, humidity, voltage, and comprises display effects such as image sticking (area and line image sticking), mura, yogore etc. which are known to the skilled person in the field of LC displays. As a standard parameter for categorising the reliability usually the voltage holding ration (VHR) value is used, which is a measure for maintaining a constant electrical voltage in a test display. The higher the VHR value, the better the reliability of the LC medium.

In VA displays of the more recent type, uniform alignment of the LC molecules is restricted to a plurality of relatively small domains within the LC cell. Disclinations may exist between these domains, also known as tilt domains. VA displays having tilt domains have, compared with conventional VA displays, a greater viewing-angle independence of the contrast and the grey shades. In addition, displays of this type are simpler to produce since additional treatment of the electrode surface for uniform alignment of the molecules in the switched-on state, such as, for example, by rubbing, is no longer necessary. Instead, the preferential direction of the tilt or pretilt angle is controlled by a special design of the electrodes.

In so-called MVA ("multidomain vertical alignment") displays, this is usually achieved by the electrodes having protrusions which cause a local pretilt. As a consequence, the LC molecules are aligned parallel to the electrode surfaces in different directions in different, defined regions of the cell on application of a voltage. "Controlled" switching is thereby achieved, and the formation of interfering disclination lines is prevented. Although this arrangement improves the viewing angle of the display, it results, however, in a reduction in its transparency to light. A further development of MVA uses protrusions on only one electrode side, while the opposite electrode has slits, which improves the transparency to light. The slitted electrodes generate an inhomogeneous electric field in the LC cell on application of a voltage, meaning that controlled switching is still achieved. For further improvement of the transparency to light, the separations between the slits and protrusions can be increased, but this in turn results in a lengthening of the response times. In so-called PVA ("patterned VA") displays, protrusions are rendered completely superfluous in that both electrodes are structured by means of slits on the opposite sides, which results in increased contrast and improved transparency to light, but is technologically difficult and makes the display more sensitive to mechanical influences ("tapping", etc.). For many applications, such as, for example, monitors and especially TV screens, however, a shortening of the response times and an improvement in the contrast and luminance (transmission) of the display are demanded.

A further development are displays of the so-called PS ("polymer sustained") or PSA ("polymer sustained alignment") type, for which the term "polymer stabilised" is also occasionally used. In these, a small amount (for example 0.3% by weight, typically < 1% by weight) of one or more polymerisable, compound(s), preferably polymerisable monomeric compound(s), is added to the LC medium and, after filling the LC medium into the display, is polymerised or crosslinked in situ, usually by UV photopolymerisation, optionally while a voltage is applied to the electrodes of the display. The polymerisation is carried out at a temperature where the LC medium exhibits a liquid crystal phase, usually at room temperature. The addition of polymerisable mesogenic or liquid-crystalline compounds, also known as reactive mesogens or "RMs", to the LC mixture has proven particularly suitable.

Unless indicated otherwise, the term "PSA" is used hereinafter when referring to displays of the polymer sustained alignment type in general, and the term "PS" is used when referring to specific display modes, like PS-VA, PS-TN and the like.

Also, unless indicated otherwise, the term "RM" is used hereinafter when referring to a polymerisable mesogenic or liquid-crystalline compound.

In the meantime, the PS(A) principle is being used in various conventional LC display modes. Thus, for example, PS-VA, PS-OCB, PS-IPS, PS-FFS, PS-UB-FFS and PS-TN displays are known. The polymerisation of the RMs preferably takes place with an applied voltage in the case of PS-VA and PS-OCB displays, and with or without, preferably without, an applied voltage in the case of PS-IPS displays. As can be demonstrated in test cells, the PS(A) method results in a pretilt in the cell. In the case of PS-OCB displays, for example, it is possible for the bend structure to be stabilised so that an offset voltage is unnecessary or can be reduced. In the case of PS-VA displays, the pretilt has a positive effect on response times. For PS-VA displays, a standard MVA or PVA pixel and electrode layout can be used. In addition, however, it is also possible, for example, to manage with only one structured electrode side and no protrusions, which significantly simplifies production and at the same time results in very good contrast at the same time as very good transparency to light.

Furthermore, the so-called posi-VA displays ("positive VA") have proven to be a particularly suitable mode. Like in classical VA displays, the initial orientation of the LC molecules in posi-VA displays is homeotropic, i.e. substantially perpendicular to the substrates, in the initial state when no voltage is applied. However, in contrast to classical VA displays, in posi-VA displays LC media with positive dielectric anisotropy are used. Like in the usually used IPS displays, the two electrodes in posi-VA displays are arranged on only one of the two substrates, and preferably exhibit intermeshed and comb-shaped (interdigital) structures. By application of a voltage to the interdigital electrodes, which create an electrical field that is substantially parallel to the layer of the LC medium, the LC molecules are transferred into an orientation that is substantially parallel to the substrates. In posi-VA displays polymer stabilisation, by addition of RMs to the LC medium which are polymerised in the display, has also proven to be advantageous, as a significant reduction of the switching times could thereb< be realised.

PS-VA displays are described, for example, in EP 1 170 626 A2, US 6,861,107, US 7,169,449, US 2004/0191428 A1, US 2006/0066793 A1 and US 2006/0103804 A1. PS-OCB displays are described, for example, in T.-J-Chen et al., Jpn. J. Appl. Phys. 45, 2006, 2702-2704 and S. H. Kim, L.-C-Chien, Jpn. J. Appl. Phys. 43, 2004, 7643-7647. PS-IPS displays are described, for example, in US 6,177,972 and Appl. Phys. Lett. 1999, 75(21), 3264. PS-TN displays are described, for example, in Optics Express 2004, 12(7), 1221.

Like the conventional LC displays described above, PSA displays can be operated as active-matrix or passive-matrix displays. In the case of active-matrix displays, individual pixels are usually addressed by integrated, non-linear active elements, such as, for example, transistors (for example thin-film transistors ("TFTs")), while in the case of passive-matrix displays, individual pixels are usually addressed by the multiplex method, as known from the prior art.

The PSA display may also comprise an alignment layer on one or both of the substrates forming the display cell. The alignment layer is usually applied on the electrodes (where such electrodes are present) such that it is in contact with the LC medium and induces initial alignment of the LC molecules. The alignment layer may comprise or consist of, for example, a polyimide, which may also be rubbed, or may be prepared by a photoalignment method.

In particular for monitor and especially TV applications, optimisation of the response times, but also of the contrast and luminance (thus also transmission) of the LC display continues to be demanded. The PSA method can provide significant advantages here. In particular in the case of PS-VA, PS-IPS, PS-FFS and PS-posi-VA displays, a shortening of the response times, which correlate with a measurable pretilt in test cells, can be achieved without significant adverse effects on other parameters.

Prior art has suggested biphenyl diacrylates or dimethacrylates, which are optionally fluorinated as RMs for use in PSA displays

However, the problem arises that not all combinations consisting of an LC mixture and one or more RMs are suitable for use in PSA displays because, for example, an inadequate tilt or none at all becomes established or since, for example, the so-called "voltage holding ratio" (VHR or HR) is inadequate for TFT display applications. In addition, it has been found that, on use in PSA displays, the LC mixtures and RMs known from the prior art do still have some disadvantages. Thus, not every known RM which is soluble in LC mixtures is suitable for use in PSA displays. In addition, it is often difficult to find a suitable selection criterion for the RM besides direct measurement of the pretilt in the PSA display. The choice of suitable RMs becomes even smaller if polymerisation by means of UV light without the addition of photoinitiators is desired, which may be advantageous for certain applications.

In addition, the selected combination of LC host mixture/RM should have the lowest possible rotational viscosity and the best possible electrical properties. In particular, it should have the highest possible VHR. In PSA displays, a high VHR after irradiation with UV light is particularly necessary since UV exposure is a requisite part of the display production process, but also occurs as normal exposure during operation of the finished display.

In particular, it would be desirable to have available novel materials for PSA displays which produce a particularly small pretilt angle. Preferred materials here are those which produce a lower pretilt angle during polymerisation for the same exposure time than the materials known to date, and/or through the use of which the (higher) pretilt angle that can be achieved with known materials can already be achieved after a shorter exposure time. The production time ("tact time") of the display could thus be shortened and the costs of the production process reduced.

A further problem in the production of PSA displays is the presence or removal of residual amounts of unpolymerised RMs, in particular after the polymerisation step for production of the pretilt angle in the display. For example, unreacted RMs of this type may adversely affect the properties of the display by, for example, polymerising in an uncontrolled manner during operation after finishing of the display.

Thus, the PSA displays known from the prior art often exhibit the undesired effect of so-called "image sticking" or "image burn", i.e. the image produced in the LC display by temporary addressing of individual pixels still remains visible even after the electric field in these pixels has been switched off or after other pixels have been addressed.

This "image sticking" can occur on the one hand if LC host mixtures having a low VHR are used. The UV component of daylight or the backlighting can cause undesired decomposition reactions of the LC molecules therein and thus initiate the production of ionic or free-radical impurities. These may accumulate, in particular, at the electrodes or the alignment layers, where they may reduce the effective applied voltage. This effect can also be observed in conventional LC displays without a polymer component.

In addition, an additional "image sticking" effect caused by the presence of unpolymerised RMs is often observed in PSA displays. Uncontrolled polymerisation of the residual RMs is initiated here by UV light from the environment or by the backlighting. In the switched display areas, this changes the tilt angle after a number of addressing cycles. As a result, a change in transmission in the switched areas may occur, while it remains unchanged in the unswitched areas.

It is therefore desirable for the polymerisation of the RMs to proceed as completely as possible during production of the PSA display and for the presence of unpolymerised RMs in the display to be excluded as far as possible or reduced to a minimum. Thus, RMs and LC mixtures are required which enable or support highly effective and complete polymerisation of the RMs. In addition, controlled reaction of the residual RM amounts would be desirable. This would be simpler if the RM polymerised more rapidly and effectively than the compounds known to date.

A further problem that has been observed in the operation of PSA displays is the stability of the pretilt angle. Thus, it was observed that the pretilt angle, which was generated during display manufacture by polymerising the RM as described above, does not remain constant but can deteriorate after the display was subjected to voltage stress during its operation. This can negatively affect the display performance, e.g. by increasing the black state transmission and hence lowering the contrast.

Another problem to be solved is that the RMs of prior art do often have high melting points, and do only show limited solubility in many currently common LC mixtures, and therefore frequently tend to spontaneously crystallise out of the mixture. In addition, the risk of spontaneous polymerisation prevents the LC host mixture being warmed in order to dissolve the polymerisable component, meaning that the best possible solubility even at room temperature is necessary. In addition, there is a risk of separation, for example on introduction of the LC medium into the LC display (chromatography effect), which may greatly impair the homogeneity of the display. This is further increased by the fact that the LC media are usually introduced at low temperatures in order to reduce the risk of spontaneous polymerisation (see above), which in turn has an adverse effect on the solubility.

Another problem observed in prior art is that LC media for use in PSA displays, including but not limited to displays of the PSA type, do often exhibit high viscosities and, as a consequence, high switching times. In order to reduce the viscosity and switching time of the LC medium, it has been suggested in prior art to add LC compounds with an alkenyl group. However, it was observed that LC media containing alkenyl compounds often show a decrease of the reliability and stability, and a decrease of the VHR especially after exposure to UV radiation. Especially for use in PSA displays this is a considerable disadvantage, because the photo-polymerisation of the RMs in the PSA display is usually carried out by exposure to UV radiation, which may cause a VHR drop in the LC medium.

There is thus still a great demand for PSA displays and LC media and polymerisable compounds for use in such displays, which do not show the drawbacks as described above, or only do so to a small extent, and have improved properties. In particular, there is a great demand for PSA displays, and LC media and polymerisable compounds for use in such PSA displays, which enable a high specific resistance at the same time as a large working-temperature range, short response times, even at low temperatures, and a low threshold voltage, a low pretilt angle, a multiplicity of grey shades, high contrast and a broad viewing angle, have high reliability and high values for the "voltage holding ratio" (VHR) after UV exposure, and, in case of the polymerisable compounds, have low melting points and a high solubility in the LC host mixtures.

The invention is based on the object of providing novel suitable materials, in particular RMs and LC media comprising same, for use in PSA displays, which do not have the disadvantages indicated above or do so to a reduced extent, polymerise as rapidly and completely as possible, enable a low pretilt angle to be established as quickly as possible, reduce or prevent the occurrence of "image sticking" in the display, and preferably at the same time enable very high specific resistance values, high VHR values, low threshold voltages and short response times, and have a high solubility in the LC media which are typically used as host mixtures in PSA displays.

A further object of the invention is the provision of novel RMs, in particular for optical, electro-optical and electronic applications, and of suitable processes and intermediates for the preparation thereof.

In particular, the invention is based on the object of providing polymerisable compounds like RMs which produce a lower pretilt after photopolymerisation, which results in the desired pretilt being achieved more quickly and thus in significantly shortened times for production of the LC display, and which are easily processable in an LC mixture.

This object has been achieved in accordance with the present invention by compounds, mixtures and processes as described in the present application. In particular, it has been found, surprisingly, that the use of multireactive polymerisable compounds of formula I as described hereinafter, which contain at least three polymerisable groups, and contain at least one branched polymerisable group, in PSA displays facilitates particularly low pretilt angles and fast establishment of the desired tilt angles.

This has been demonstrated in connection with an LC medium by means of pretilt measurements. In particular, a pretilt has been achieved without the addition of photoinitiator. In addition, the polymerisable compounds according to the present invention exhibit significantly faster generation of the pretilt angle compared with the compounds known from prior art, as demonstrated by exposure time-dependent measurements of the pretilt angle.

It has also been demonstrated that the polymerisable compounds according to the present invention are especially suitable for use in LC host mixtures containing mesogenic or LC compounds with an alkenyl group. The use of the polymerisable compounds according to the present invention in such LC host mixtures enables LC media with high VHR values and high reliability.

In addition, the polymerisable compounds according to the invention exhibit a high polymerisation rate, causing smaller unreacted residual amounts to remain in the cell. The electro-optical properties of the cell are thus improved, and in addition controlled reaction of these residual amounts becomes simpler. The polymerisable compounds are therefore suitable for creating a high pretilt in PSA type displays.

Also, the polymerisable compounds according to the invention show a low tendency towards crystallisation and high solubility in typical commercially available LC host mixtures.

US 7,060,200 B1 and US 2006/0172090 A1 disclose multireactive compounds with branched polymerisable groups for use in polymerisable LC materials and LC polymers, but do not disclose polymerisable compounds as disclosed or claimed hereinafter, or their use in LC media for PSA type LC displays.

WO2014/079517A1 discloses polymerisable compounds of a generic formula I, which comprise a spacer group Sp⁴ that is directly adjacent to a cyclic group A² and may also denote a single bond.

US2007/0284556A1 discloses polymerisable compounds comprising a polymerisable diene group.

US2006/0172090A1 discloses polymerisable compounds of formula (Ia) having a branched polymerisable group on one side of the mesogenic core and no polymerisable group on the other side, and further discloses polymerisable compounds of formula (Ib) having a branched polymerisable group on both sides of the mesogenic core.

Hentrich et al., J. Mater. Chem. 1994, 4(10), 1547 discloses diol-based biphenyls with three or four terminal OH groups.

WO2013/077343A1 discloses a biphenyl compound (S-13) with an acrylate group and two terminal hydroxy groups.

WO2013/178333A1 discloses an LC medium for use in PSA displays, wherein the LC host mixture contains fluorinated biphenyl compounds, and further discloses that the LC medium may contain trireactive polymerisable compounds with a branched polymerisable group that is attached to the mesogenic core via an alkylene spacer, but without an ether bond.

However, the above cited documents do not disclose polymerisable compounds as disclosed and claimed hereinafter.

### Summary of the Invention

The invention relates to compounds of formula I

P¹-Sp¹-(A¹-Z¹)ₙ-A²-O-Sp⁴-CH(Sp²-P²)(Sp³-P³) I

in which the individual radicals have the following meanings:
- P¹, P², P³ and Sp¹⁻⁴: are as defined in claim 1
- A¹, A²: independently of each other, and on each occurrence identically or differently, denote an aromatic, heteroaromatic, alicyclic or heterocyclic group having 4 to 25 C atoms, which may also contain fused rings, and which is optionally mono- or polysubstituted by L,
- Z¹: denotes, on each occurrence identically or differently, -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -CR⁰⁰R⁰⁰⁰-, or a single bond,
- L: denotes P¹-, P¹-Sp¹-, F, Cl, Br, I, -CN, -NO₂, -NCO, -NCS,-OCN, -SCN, -C(=O)N(R^{x})₂, -C(=O)Y¹, -C(=O)R^{x}, -N(R^{x})₂, optionally substituted silyl, optionally substituted aryl or heteroaryl having 5 to 20 ring atoms, or straight-chain or branched alkyl having 1 to 25, particularly preferably 1 to 10, C atoms, in which, in addition, one or more non-adjacent CH₂ groups may each be replaced, independently of one another, by -C(R⁰⁰)=C(R⁰⁰⁰)-, -C≡C-, -N(R⁰⁰)-, -O-, -S-, -CO-, -CO-O-, -O-CO-, -O-CO-O- in such a way that O and/or S atoms are not linked directly to one another, and in which, in addition, one or more H atoms may be replaced by F, Cl, CN, P¹ or P¹-Sp¹-,
- R⁰⁰ and R⁰⁰⁰: each, independently of one another, denote H or alkyl having 1 to 12 C atoms,
- Y¹: is halogen,
- R^{x}: denotes P¹, P¹-Sp¹-, H, halogen, straight chain, branched or cyclic alkyl having 1 to 25 C atoms, wherein one or more non-adjacent CH₂-groups are optionally replaced by -O-, -S-, -CO-,-CO-O-, -O-CO-, -O-CO-O- in such a manner that O- and/or S-atoms are not directly connected with each other, and wherein one or more H atoms are optionally replaced by F, Cl, P¹- or P¹-Sp¹-, optionally substituted aryl, aryloxy, heteroaryl or heteroaryloxy having 5 to 20 ring atoms,
- n: is 1, 2, 3 or 4.

The invention further relates to the use of compounds of formula I as polymerisable compounds in LC media and LC displays, especially in the LC medium, active layer or alignment layer of an LC display, wherein the LC displays are preferably PSA displays.

The invention further relates to methods for preparing compounds of formula I, and to novel intermediates used or obtained in these methods.

The invention furthermore relates to an LC medium comprising one or more compounds of formula I.

The invention furthermore relates to an LC medium comprising one or more polymerisable compounds, at least one of which is a compound of formula I.

The invention furthermore relates to an LC medium comprising
- a polymerisable component A) comprising, preferably consisting of, one or more polymerisable compounds, at least one of which is a compound of formula I, and
- a liquid-crystalline component B), hereinafter also referred to as "LC host mixture", comprising, preferably consisting of, one or more mesogenic or liquid-crystalline compounds.

The liquid-crystalline component B) of an LC medium according to the present invention is hereinafter also referred to as "LC host mixture", and preferably comprises, or consists of, one or more, preferably at least two mesogenic or LC compounds selected from low-molecular-weight compounds which are unpolymerisable.

The invention furthermore relates to an LC medium as described above and below, wherein the LC host mixture or component B comprise at least one mesogenic or LC compound comprising an alkenyl group.

The invention furthermore relates to an LC medium or LC display as described above, wherein the compounds of formula I are polymerised.

The invention furthermore relates to a process for preparing an LC medium as described above and below, comprising the steps of mixing one or more mesogenic or LC compounds, or an LC host mixture or LC component B) as described above and below, with one or more compounds of formula I, and optionally with further LC compounds and/or additives.

The invention furthermore relates to the use of compounds of formula I and LC media according to the invention in PSA displays, in particular the use in PSA displays containing an LC medium, for the production of a tilt angle in the LC medium by in-situ polymerisation of the compound(s) of the formula I in the PSA display, preferably in an electric or magnetic field.

The invention furthermore relates to an LC display comprising one or more compounds of formula I or an LC medium according to the invention, in particular a PSA display, particularly preferably a PS-VA, PS-OCB, PS-IPS, PS-FFS, PS-UB-FFS, PS-posi-VA or PS-TN display.

The invention furthermore relates to an LC display comprising a polymer obtainable by polymerisation of one or more compounds of formula I or of a polymerisable component A) as described above, or comprising an LC medium according to the invention, which is preferably a PSA display, very preferably a PS-VA, PS-OCB, PS-IPS, PS-FFS, PS-UB-FFS, PS-posi-VA or PS-TN display.

The invention furthermore relates to an LC display of the PSA type comprising two substrates, at least one which is transparent to light, an electrode provided on each substrate or two electrodes provided on only one of the substrates, and located between the substrates a layer of an LC medium that comprises one or more polymerisable compounds and an LC component as described above and below, wherein the polymerisable compounds are polymerised between the substrates of the display.

The invention furthermore relates to a process for manufacturing an LC display as described above and below, comprising the steps of filling or otherwise providing an LC medium, which comprises one or more polymerisable compounds as described above and below, between the substrates of the display, and polymerising the polymerisable compounds.

The PSA displays according to the invention have two electrodes, preferably in the form of transparent layers, which are applied to one or both of the substrates. In some displays, for example in PS-VA, PS-OCB or PS-TN displays, one electrode is applied to each of the two substrates. In other displays, for example in PS-posi-VA, PS-IPS or PS-FFS or PS-UB-FFS displays, both electrodes are applied to only one of the two substrates.

In a preferred embodiment the polymerisable component is polymerised in the LC display while a voltage is applied to the electrodes of the display.

The polymerisable compounds of the polymerisable compoment are preferably polymerised by photo-polymerisation, very preferably by UV photo-polymerisation.

### Detailed Description of the Invention

As used herein, the terms "active layer" and "switchable layer" mean a layer in an electrooptical display, for example an LC display, that comprises one or more molecules having structural and optical anisotropy, like for example LC molecules, which change their orientation upon an external stimulus like an electric or magnetic field, resulting in a change of the transmission of the layer for polarized or unpolarized light.

As used herein, the terms "tilt" and "tilt angle" will be understood to mean a tilted alignment of the LC molecules of an LC medium relative to the surfaces of the cell in an LC display (here preferably a PSA display). The tilt angle here denotes the average angle (< 90°) between the longitudinal molecular axes of the LC molecules (LC director) and the surface of the plane-parallel outer plates which form the LC cell. A low value for the tilt angle (i.e. a large deviation from the 90° angle) corresponds to a large tilt here. A suitable method for measurement of the tilt angle is given in the examples. Unless indicated otherwise, tilt angle values disclosed above and below relate to this measurement method.

As used herein, the terms "reactive mesogen" and "RM" will be understood to mean a compound containing a mesogenic or liquid crystalline skeleton, and one or more functional groups attached thereto which are suitable for polymerisation and are also referred to as "polymerisable group" or "P".

Unless stated otherwise, the term "polymerisable compound" as used herein will be understood to mean a polymerisable monomeric compound.

As used herein, the term "low-molecular-weight compound" will be understood to mean to a compound that is monomeric and/or is not prepared by a polymerisation reaction, as opposed to a "polymeric compound" or a "polymer".

As used herein, the term "unpolymerisable compound" will be understood to mean a compound that does not contain a functional group that is suitable for polymerisation under the conditions usually applied for the polymerisation of the RMs.

The term "mesogenic group" as used herein is known to the person skilled in the art and described in the literature, and means a group which, due to the anisotropy of its attracting and repelling interactions, essentially contributes to causing a liquid-crystal (LC) phase in low-molecular-weight or polymeric substances. Compounds containing mesogenic groups (mesogenic compounds) do not necessarily have to have an LC phase themselves. It is also possible for mesogenic compounds to exhibit LC phase behaviour only after mixing with other compounds and/or after polymerisation. Typical mesogenic groups are, for example, rigid rod- or disc-shaped units. An overview of the terms and definitions used in connection with mesogenic or LC compounds is given in Pure Appl. Chem. 73(5), 888 (2001) and C. Tschierske, G. Pelzl, S. Diele, Angew. Chem. 2004, 116, 6340-6368.

The term "spacer group", hereinafter also referred to as "Sp", as used herein is known to the person skilled in the art and is described in the literature, see, for example, Pure Appl. Chem. 73(5), 888 (2001) and C. Tschierske, G. Pelzl, S. Diele, Angew. Chem. 2004, 116, 6340-6368. As used herein, the terms "spacer group" or "spacer" mean a flexible group, for example an alkylene group, which connects the mesogenic group and the polymerisable group(s) in a polymerisable mesogenic compound.

Above and below, 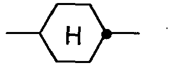 denotes a trans-1,4-cyclohexylene ring, and 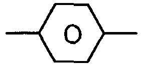 denotes a 1,4-phenylene ring.

Above and below "organic group" denotes a carbon or hydrocarbon group.

"Carbon group" denotes a mono- or polyvalent organic group containing at least one carbon atom, where this either contains no further atoms (such as, for example, -C≡C-) or optionally contains one or more further atoms, such as, for example, N, O, S, B, P, Si, Se, As, Te or Ge (for example carbonyl, etc.). The term "hydrocarbon group" denotes a carbon group which additionally contains one or more H atoms and optionally one or more heteroatoms, such as, for example, N, O, S, B, P, Si, Se, As, Te or Ge.

"Halogen" denotes F, CI, Br or I.

-CO-, -C(=O)- and -C(O)- denote a carbonyl group, i.e.

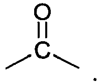

A carbon or hydrocarbon group can be a saturated or unsaturated group. Unsaturated groups are, for example, aryl, alkenyl or alkynyl groups. A carbon or hydrocarbon radical having more than 3 C atoms can be straight-chain, branched and/or cyclic and may also contain spiro links or condensed rings.

The terms "alkyl", "aryl", "heteroaryl", etc., also encompass polyvalent groups, for example alkylene, arylene, heteroarylene, etc.

The term "aryl" denotes an aromatic carbon group or a group derived therefrom. The term "heteroaryl" denotes "aryl" as defined above, containing one or more heteroatoms, preferably selected from N, O, S, Se, Te, Si and Ge.

Preferred carbon and hydrocarbon groups are optionally substituted, straight-chain, branched or cyclic, alkyl, alkenyl, alkynyl, alkoxy, alkylcarbonyl, alkoxycarbonyl, alkylcarbonyloxy and alkoxycarbonyloxy having 1 to 40, preferably 1 to 20, very preferably 1 to 12, C atoms, optionally substituted aryl or aryloxy having 5 to 30, preferably 6 to 25, C atoms, or optionally substituted alkylaryl, arylalkyl, alkylaryloxy, arylalkyloxy, arylcarbonyl, aryloxycarbonyl, arylcarbonyloxy and aryloxycarbonyloxy having 5 to 30, preferably 6 to 25, C atoms, wherein one or more C atoms may also be replaced by hetero atoms, preferably selected from N, O, S, Se, Te, Si and Ge.

Further preferred carbon and hydrocarbon groups are C₁-C₂₀ alkyl, C₂-C₂₀ alkenyl, C₂-C₂₀ alkynyl, C₃-C₂₀ allyl, C₄-C₂₀ alkyldienyl, C₄-C₂₀ polyenyl, C₆-C₂₀ cycloalkyl, C₄-C₁₅ cycloalkenyl, C₆-C₃₀ aryl, C₆-C₃₀ alkylaryl, C₆-C₃₀ arylalkyl, C₆-C₃₀ alkylaryloxy, C₆-C₃₀ arylalkyloxy, C₂-C₃₀ heteroaryl, C₂-C₃₀ heteroaryloxy.

Particular preference is given to C₁-C₁₂ alkyl, C₂-C₁₂ alkenyl, C₂-C₁₂ alkynyl, C₆-C₂₅ aryl and C₂-C₂₅ heteroaryl.

Further preferred carbon and hydrocarbon groups are straight-chain, branched or cyclic alkyl having 1 to 20, preferably 1 to 12, C atoms, which are unsubstituted or mono- or polysubstituted by F, Cl, Br, I or CN and in which one or more non-adjacent CH₂ groups may each be replaced, independently of one another, by -C(R^{x})=C(R^{x})-, -C≡C-, -N(R^{x})-, -O-, -S-, -CO-, -CO-O-, -O-CO-, -O-CO-O- in such a way that O and/or S atoms are not linked directly to one another.

R^{x} preferably denotes H, halogen, a straight-chain, branched or cyclic alkyl chain having 1 to 15 C atoms, in which, in addition, one or more non-adjacent C atoms may be replaced by -O-, -S-, -CO-, -CO-O-, -O-CO-, -O-CO-O- and in which one or more H atoms may be replaced by fluorine, an optionally substituted aryl or aryloxy group having 6 to 30 C atoms, or an optionally substituted heteroaryl or heteroaryloxy group having 2 to 30 C atoms.

Preferred alkoxy groups are, for example, methoxy, ethoxy, 2-methoxy-ethoxy, n-propoxy, i-propoxy, n-butoxy, i-butoxy, s-butoxy, t-butoxy, 2-methylbutoxy, n-pentoxy, n-hexoxy, n-heptoxy, n-octoxy, n-nonoxy, n-decoxy, n-undecoxy, n-dodecoxy, etc.

Preferred alkyl groups are, for example, methyl, ethyl, n-propyl, isopropyl, n-butyl, isobutyl, s-butyl, t-butyl, 2-methylbutyl, n-pentyl, s-pentyl, cyclopentyl, n-hexyl, cyclohexyl, 2-ethylhexyl, n-heptyl, cycloheptyl, n-octyl, cyclooctyl, n-nonyl, n-decyl, n-undecyl, n-dodecyl, dodecanyl, trifluoromethyl, perfluoro-n-butyl, 2,2,2-trifluoroethyl, perfluorooctyl, perfluorohexyl, etc.

Preferred alkenyl groups are, for example, ethenyl, propenyl, butenyl, pentenyl, cyclopentenyl, hexenyl, cyclohexenyl, heptenyl, cycloheptenyl, octenyl, cyclooctenyl, etc.

Preferred alkynyl groups are, for example, ethynyl, propynyl, butynyl, pentynyl, hexynyl, octynyl, etc.

Preferred alkoxy groups are, for example, methoxy, ethoxy, 2-methoxy-ethoxy, n-propoxy, i-propoxy, n-butoxy, i-butoxy, s-butoxy, t-butoxy, 2-methylbutoxy, n-pentoxy, n-hexoxy, n-heptoxy, n-octoxy, n-nonoxy, n-decoxy, n-undecoxy, n-dodecoxy, etc.

Preferred amino groups are, for example, dimethylamino, methylamino, methylphenylamino, phenylamino, etc.

Aryl and heteroaryl groups can be monocyclic or polycyclic, i.e. they can contain one ring (such as, for example, phenyl) or two or more rings, which may also be fused (such as, for example, naphthyl) or covalently bonded (such as, for example, biphenyl), or contain a combination of fused and linked rings. Heteroaryl groups contain one or more heteroatoms, preferably selected from O, N, S and Se.

Particular preference is given to mono-, bi- or tricyclic aryl groups having 6 to 25 C atoms and mono-, bi- or tricyclic heteroaryl groups having 5 to 25 ring atoms, which optionally contain fused rings and are optionally substituted. Preference is furthermore given to 5-, 6- or 7-membered aryl and heteroaryl groups, in which, in addition, one or more CH groups may be replaced by N, S or O in such a way that O atoms and/or S atoms are not linked directly to one another.

Preferred aryl groups are, for example, phenyl, biphenyl, terphenyl, [1,1':3',1"]terphenyl-2'-yl, naphthyl, anthracene, binaphthyl, phenanthrene, 9,10-dihydro-phenanthrene, pyrene, dihydropyrene, chrysene, perylene, tetracene, pentacene, benzopyrene, fluorene, indene, indenofluorene, spirobifluorene, etc.

Preferred heteroaryl groups are, for example, 5-membered rings, such as pyrrole, pyrazole, imidazole, 1,2,3-triazole, 1,2,4-triazole, tetrazole, furan, thiophene, selenophene, oxazole, isoxazole, 1,2-thiazole, 1,3-thiazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 6-membered rings, such as pyridine, pyridazine, pyrimidine, pyrazine, 1,3,5-triazine, 1,2,4-triazine, 1,2,3-triazine, 1,2,4,5-tetrazine, 1,2,3,4-tetrazine, 1,2,3,5-tetrazine, or condensed groups, such as indole, isoindole, indolizine, indazole, benzimidazole, benzotriazole, purine, naphthimidazole, phenanthrimidazole, pyridimidazole, pyrazinimidazole, quinoxalinimidazole, benzoxazole, naphthoxazole, anthroxazole, phenanthroxazole, isoxazole, benzothiazole, benzofuran, isobenzofuran, dibenzofuran, quinoline, isoquinoline, pteridine, benzo-5,6-quinoline, benzo-6,7-quinoline, benzo-7,8-quinoline, benzoisoquinoline, acridine, phenothiazine, phenoxazine, benzopyridazine, benzopyrimidine, quinoxaline, phenazine, naphthyridine, azacarbazole, benzocarboline, phenanthridine, phenanthroline, thieno[2,3b]thiophene, thieno[3,2b]thiophene, dithienothiophene, isobenzothiophene, dibenzothiophene, benzothiadiazothiophene, or combinations of these groups.

The aryl and heteroaryl groups mentioned above and below may also be substituted by alkyl, alkoxy, thioalkyl, fluorine, fluoroalkyl or further aryl or heteroaryl groups.

The (non-aromatic) alicyclic and heterocyclic groups encompass both saturated rings, i.e. those containing exclusively single bonds, and also partially unsaturated rings, i.e. those which may also contain multiple bonds. Heterocyclic rings contain one or more heteroatoms, preferably selected from Si, O, N, S and Se.

The (non-aromatic) alicyclic and heterocyclic groups can be monocyclic, i.e. contain only one ring (such as, for example, cyclohexane), or polycyclic, i.e. contain a plurality of rings (such as, for example, decahydronaphthalene or bicyclooctane). Particular preference is given to saturated groups. Preference is furthermore given to mono-, bi- or tricyclic groups having 5 to 25 ring atoms, which optionally contain fused rings and are optionally substituted. Preference is furthermore given to 5-, 6-, 7- or 8-membered carbocyclic groups, in which, in addition, one or more C atoms may be replaced by Si and/or one or more CH groups may be replaced by N and/or one or more non-adjacent CH₂ groups may be replaced by -O- and/or -S-.

Preferred alicyclic and heterocyclic groups are, for example, 5-membered groups, such as cyclopentane, tetrahydrofuran, tetrahydrothiofuran, pyrrolidine, 6-membered groups, such as cyclohexane, silinane, cyclohexene, tetrahydropyran, tetrahydrothiopyran, 1,3-dioxane, 1,3-dithiane, piperidine, 7-membered groups, such as cycloheptane, and fused groups, such as tetrahydronaphthalene, decahydronaphthalene, indane, bicyclo[1.1.1]-pentane-1,3-diyl, bicyclo[2.2.2]octane-1,4-diyl, spiro[3.3]heptane-2,6-diyl, octahydro-4,7-methanoindane-2,5-diyl.

Preferred substituents are, for example, solubility-promoting groups, such as alkyl or alkoxy, electron-withdrawing groups, such as fluorine, nitro or nitrile, or substituents for increasing the glass transition temperature (Tg) in the polymer, in particular bulky groups, such as, for example, t-butyl or optionally substituted aryl groups.

Preferred substituents, hereinafter also referred to as "L", are, for example, F, Cl, Br, I, -CN, -NO₂, -NCO, -NCS, -OCN, -SCN, -C(=O)N(R^{x})₂,-C(=O)Y¹, -C(=O)R^{x}, -N(R^{x})₂, straight-chain or branched alkyl, alkoxy, alkylcarbonyl, alkoxycarbonyl, alkylcarbonyloxy or alkoxycarbonyloxy each having 1 to 25 C atoms, in which one or more H atoms may optionally be replaced by F or CI, optionally substituted silyl having 1 to 20 Si atoms, or optionally substituted aryl having 6 to 25, preferably 6 to 15, C atoms,
wherein R^{x} denotes H, F, Cl, CN, or straight chain, branched or cyclic alkyl having 1 to 25 C atoms, wherein one or more non-adjacent CH₂-groups are optionally replaced by -O-, -S-, -CO-, -CO-O-, -O-CO-, -O-CO-O- in such a manner that O- and/or S-atoms are not directly connected with each other, and wherein one or more H atoms are each optionally replaced by F, CI, P- or P-Sp-, and
Y¹ denotes halogen.

"Substituted silyl or aryl" preferably means substituted by halogen, -CN, R⁰, -OR⁰, -CO-R⁰, -CO-O-R⁰, -O-CO-R⁰ or -O-CO-O-R⁰, wherein R⁰ denotes H or alkyl with 1 to 20 C atoms.

Particularly preferred substituents L are, for example, F, CI, CN, NO₂, CH₃, C₂H₅, OCH₃, OC₂H₅, COCH₃, COC₂H₅, COOCH₃, COOC₂H₅, CF₃, OCF₃, OCHF₂, OC₂F₅, furthermore phenyl. 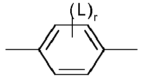 is preferably 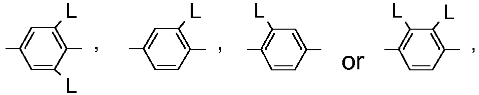 in which L has one of the meanings indicated above.

The polymerisable groups P¹⁻³ are selected from the group consisting of vinyl, vinyloxy, acrylate, methacrylate, fluoroacrylate, chloroacrylate, oxetane and epoxide groups, very preferably from acrylate and methacrylate groups.

In the compounds of formula I
- Sp¹ is a single bond, or
- Sp¹ is -(CH₂)ₚ₂- or -(CH₂)ₚ₁-O-, in which p1 is 1, 2 or 3,
- Sp² and Sp³ denote -(CH₂)ₚ₂-, in which p2 is 1, 2 or 3,
- Sp⁴ is -(CH₂)ₚ₄-, in which p4 is 1, 2 or 3.

Preferred compounds of formula I are those in which A¹, A² each, independently of one another, denote 1,4-phenylene, naphthalene-1,4-diyl or naphthalene-2,6-diyl, where one or more CH groups in these groups are optionally replaced by N, cyclohexane-1,4-diyl, in which, in addition, one or more non-adjacent CH₂ groups are optionally replaced by O and/or S, 1,4-cyclohexenylene, bicyclo[1.1.1]pentane-1,3-diyl, bicyclo[2.2.2]octane-1,4-diyl, spiro[3.3]heptane-2,6-diyl, piperidine-1,4-diyl, decahydronaphthalene-2,6-diyl, 1,2,3,4-tetrahydronaphthalene-2,6-diyl, indane-2,5-diyl, octahydro-4,7-methanoindane-2,5-diyl, anthracene-2,7-diyl, fluorene-2,7-diyl, phenanthrene-2,7-diyl or 9,10-dihydro-phenanthrene-2,7-diyl, where all these groups are unsubstituted or mono- or polysubstituted by L.

Further preferred compounds of formula I are those in which
- P¹, P² and P³ are selected from the group consisting of acrylate, methacrylate and oxetane,
- Sp¹ is a single bond,
- Sp¹ is -(CH₂)ₚ₁- or -(CH₂)ₚ₁-O-, in which p1 is 1, 2 or 3,
- Sp² and Sp³ denote
   methylene,
- Sp⁴ is
   methylene,
- Sp¹ is a single bond, and Sp², Sp³ and Sp⁴ are methylene,
- Sp¹ and Sp⁴ are a single bond, and Sp² and Sp³ are methylene,
- Sp¹, Sp² and Sp³ are a single bond, and Sp⁴ is ethylene,
- L does not denote or contain a polymerisable group,
- A¹ and A² are selected from the group consisting of 1,4-phenylene, naphthalene-2,6-diyl, phenanthrene-2,7-diyl and 9,10-dihydro-phenanthrene-2,7-diyl, where, in addition, one or two CH groups in these rings are optionally replaced by N, and where these rings are optionally mono- or polysubstituted by L, as described above and below,
- Z¹ is selected from the group consisting of -O-, -CO-O-, -OCO-, -OCH₂-, -CH₂O-, -CF₂O-, -OCF₂-, -CH₂CH₂-, -CH=CH-, -CF=CF-, -CH=CF-, -CF=CH-, -C≡C-, and a single bond,
- Z¹ is a single bond,
- n is 1 or 2,
- L is an unpolymerisable group, preferably selected from F, CI, -CN and straight-chain or branched alkyl having 1 to 25, particularly preferably 1 to 10, C atoms, in which, in addition, one or more non-adjacent CH₂ groups may each be replaced, independently of one another, by -C(R⁰⁰)=C(R⁰⁰⁰)-, -C≡C-, -N(R⁰⁰)-, -O-, -S-, -CO-, -CO-O-, -O-CO-, -O-CO-O- in such a way that O and/or S atoms are not linked directly to one another, and in which, in addition, one or more H atoms may be replaced by F, Cl, Br, I or CN.

Very preferred compounds of formula I are selected from the following subformulae 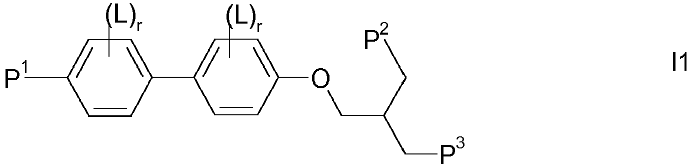 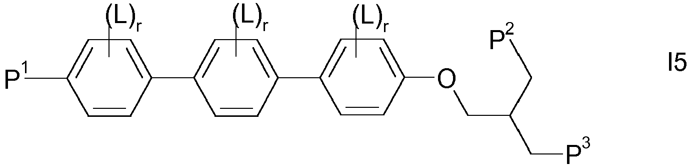 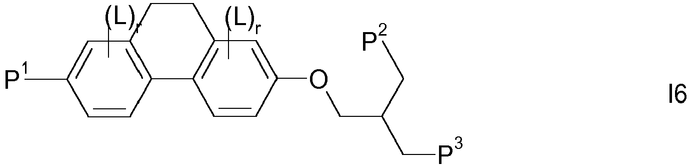 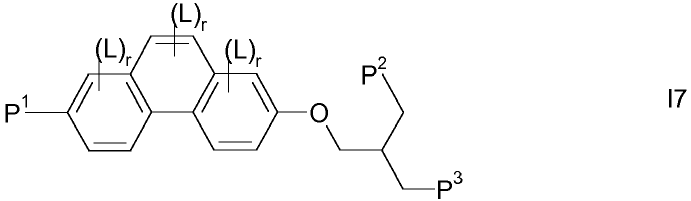 wherein P¹, P², P³ and L are as defined in formula I and r is 0, 1, 2, 3 or 4.

The compounds of the invention can be prepared from compounds of formula II

Pg¹-Sp¹-(A¹-Z¹)ₙ-A²-O-Sp⁴-CH(Sp²-Pg²)(Sp³-Pg³) II

in which Sp¹, Sp², Sp³, Sp⁴, A¹, A², Z¹ and n have the meaning indicated in formula I or above and below, and Pg¹, Pg² and Pg³ denote independently of each other OH, a protected hydroxyl group or a masked hydroxyl group.

The compounds of formula II are suitable as intermediates for the preparation of compounds of the formula I and its subformulae.

The invention further relates to the use of the compounds of formula II as intermediates for the preparation of compounds of the formula I and its subformulae.

Suitable protected hydroxyl groups Pg¹⁻³ are known to the person skilled in the art. Preferred protecting groups for hydroxyl groups are alkyl, alkoxyalkyl, acyl, alkylsilyl, arylsilyl and arylmethyl groups, especially 2-tetrahydropyranyl, methoxymethyl, methoxyethoxymethyl, acetyl, triisopropylsilyl, *tert*-butyldimethylsilyl or benzyl.

The term "masked hydroxyl group" is understood to mean any functional group that can be chemically converted into a hydroxyl group. Suitable masked hydroxyl groups Pg¹⁻³ are known to the person skilled in the art. A preferred masking group for CH(CH₂-Pg²)(CH₂-Pg³) is a malonate group - CH(CO₂Et)₂.

Preferred compounds of formula II are selected from the following subformulae: 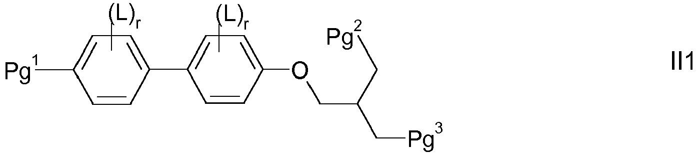 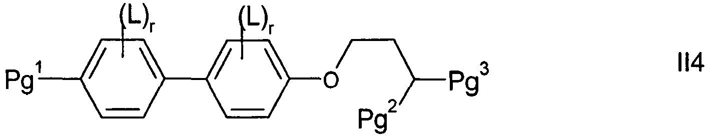 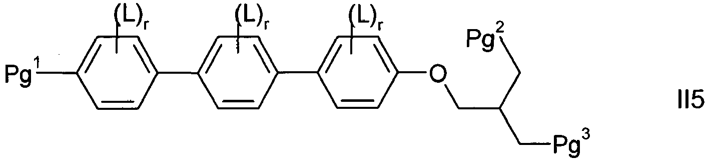 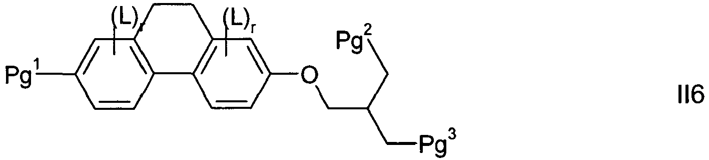 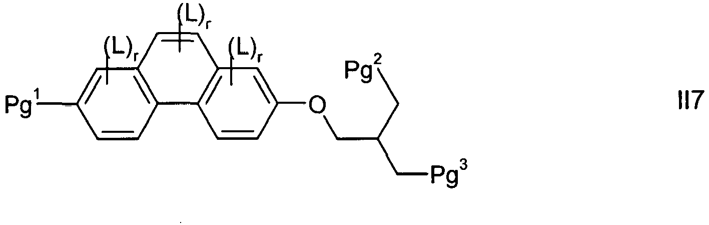 wherein Pg¹, Pg² and Pg³ are as defined in formula II or have one of the preferred meanings given above, and L and r are as defined in formula I1.

The compounds and intermediates of the formulae I and II and sub-formulae thereof can be prepared analogously to processes known to the person skilled in the art and described in standard works of organic chemistry, such as, for example, in Houben-Weyl, Methoden der organischen Chemie [Methods of Organic Chemistry], Thieme-Verlag, Stuttgart.

Particularly suitable and preferred processes for the preparation of compounds and intermediates of the formulae I and II are depicted by way of example in the following schemes and preferably comprise one or more of the steps described below.

For example, compounds of formula I can be synthesised by esterification or etherification of the intermediates of formula II, wherein Pg¹⁻³ denote OH, using corresponding acids, acid derivatives, or halogenated compounds containing a polymerisable group P¹.

As exemplarily shown in Scheme 1, acrylic or methacrylic esters (wherein Sp¹⁻⁴, A¹⁻², Z¹ and n have the meanings given above, and "Acr" denotes an acrylate or methacrylate group) can be prepared by esterification of the corresponding alcohols with acid derivatives like, for example, (meth)acryloyl chloride or (meth)acrylic anhydride in the presence of a base like pyridine or triethyl amine, and 4-(*N*,*N*-dimethylamino)pyridine (DMAP). Alternatively the esters can be prepared by esterification of the alcohols with (meth)acrylic acid in the presence of a dehydrating reagent, for example according to Steglich with dicyclohexylcarbodiimide (DCC), *N*-(3-dimethylaminopropyl)-*N*'-ethylcarbodiimide (EDC) or *N*-(3-dimethylaminopropyl)-*N'*-ethylcarbodiimide hydrochloride and DMAP.

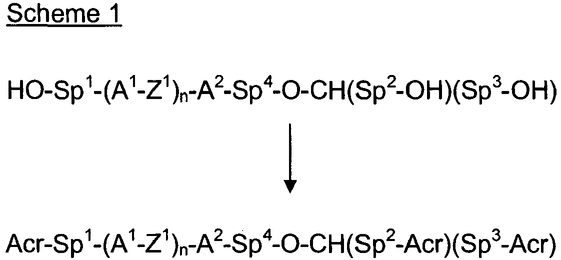

Suitable reaction schemes for the synthesis of compounds of formula I are exemplarily illustrated below. Other compounds of formula I with different mesogenic cores and/or different spacer groups can be prepared by the person skilled in the art in analogy thereto without inventive contribution.

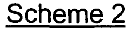

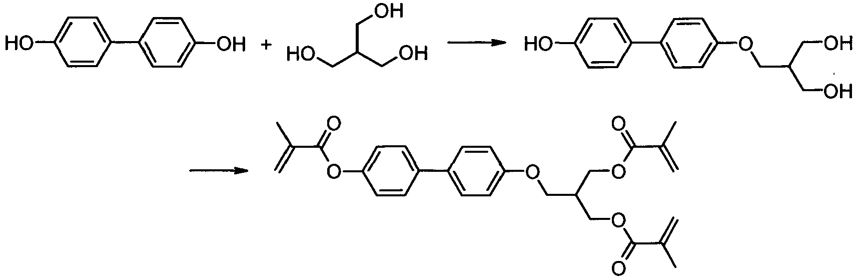

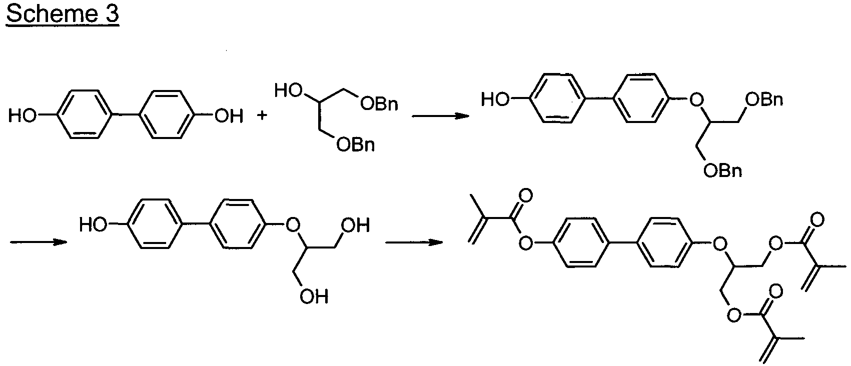

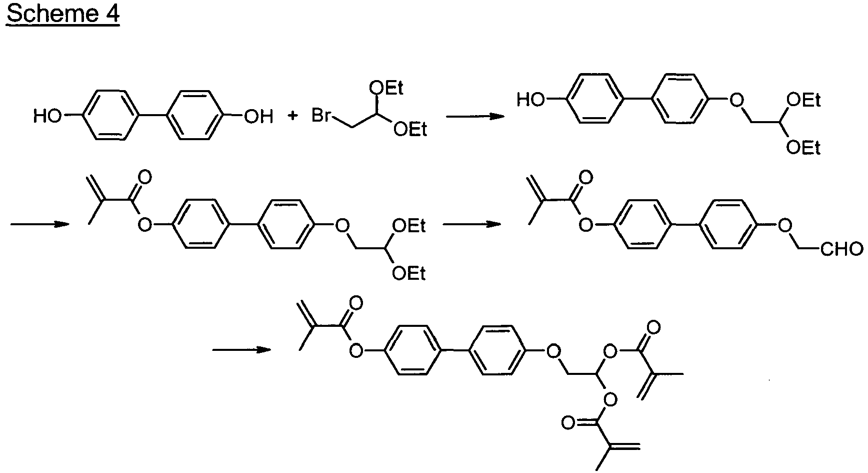

For the production of PSA displays, the polymerisable compounds cointained in the LC medium are polymerised or crosslinked (if one compound contains two or more polymerisable groups) by in-situ polymerisation in the LC medium between the substrates of the LC display, optionally while a voltage is applied to the electrodes.

The structure of the PSA displays according to the invention corresponds to the usual geometry for PSA displays, as described in the prior art cited at the outset. Geometries without protrusions are preferred, in particular those in which, in addition, the electrode on the colour filter side is unstructured and only the electrode on the TFT side has slots. Particularly suitable and preferred electrode structures for PS-VA displays are described, for example, in US 2006/0066793 A1.

A preferred PSA type LC display of the present invention comprises:
- a first substrate including a pixel electrode defining pixel areas, the pixel electrode being connected to a switching element disposed in each pixel area and optionally including a micro-slit pattern, and optionally a first alignment layer disposed on the pixel electrode,
- a second substrate including a common electrode layer, which may be disposed on the entire portion of the second substrate facing the first substrate, and optionally a second alignment layer,
- an LC layer disposed between the first and second substrates and including an LC medium comprising a polymerisable component A and a liquid crystal component B as described above and below, wherein the polymerisable component A may also be polymerised.

The first and/or second alignment layer controls the alignment direction of the LC molecules of the LC layer. For example, in PS-VA displays the alignment layer is selected such that it imparts to the LC molecules homeotropic (or vertical) alignment (i.e. perpendicular to the surface) or tilted alignment. Such an alignment layer may for example comprise a polyimide, which may also be rubbed, or may be prepared by a photoalignment method.

The LC layer with the LC medium can be deposited between the substrates of the display by methods that are conventionally used by display manufacturers, for example the so-called one-drop-filling (ODF) method. The polymerisable component of the LC medium is then polymerised for example by UV photopolymerisation. The polymerisation can be carried out in one step or in two or more steps.

The PSA display may comprise further elements, like a colour filter, a black matrix, a passivation layer, optical retardation layers, transistor elements for addressing the individual pixels, etc., all of which are well known to the person skilled in the art and can be employed without inventive skill.

The electrode structure can be designed by the skilled person depending on the individual display type. For example for PS-VA displays a multi-domain orientation of the LC molecules can be induced by providing electrodes having slits and/or bumps or protrusions in order to create two, four or more different tilt alignment directions.

Upon polymerisation the polymerisable compounds form a crosslinked polymer, which causes a certain pretilt of the LC molecules in the LC medium. Without wishing to be bound to a specific theory, it is believed that at least a part of the crosslinked polymer, which is formed by the polymerisable compounds, will phase-separate or precipitate from the LC medium and form a polymer layer on the substrates or electrodes, or the alignment layer provided thereon. Microscopic measurement data (like SEM and AFM) have confirmed that at least a part of the formed polymer accumulates at the LC/substrate interface.

The polymerisation can be carried out in one step. It is also possible firstly to carry out the polymerisation, optionally while applying a voltage, in a first step in order to produce a pretilt angle, and subsequently, in a second polymerisation step without an applied voltage, to polymerise or crosslink the compounds which have not reacted in the first step ("end curing").

Suitable and preferred polymerisation methods are, for example, thermal or photopolymerisation, preferably photopolymerisation, in particular UV induced photopolymerisation, which can be achieved by exposure of the polymerisable compounds to UV radiation.

Optionally one or more polymerisation initiators are added to the polymerisable compounds. Suitable conditions for the polymerisation and suitable types and amounts of initiators are known to the person skilled in the art and are described in the literature. Suitable for free-radical polymerisation are, for example, the commercially available photoinitiators Irgacure651®, Irgacure184®, Irgacure907®, Irgacure369® or Darocure1173® (Ciba AG). If a polymerisation initiator is employed, its proportion is preferably 0.001 to 5% by weight, particularly preferably 0.001 to 1% by weight.

The polymerisable compounds according to the invention are also suitable for polymerisation without an initiator, which is accompanied by considerable advantages, such, for example, lower material costs and in particular less contamination of the LC medium by possible residual amounts of the initiator or degradation products thereof. The polymerisation can thus also be carried out without the addition of an initiator. In a preferred embodiment, the LC medium thus does not contain a polymerisation initiator.

The polymerisable component (component A) or the LC medium may also comprise one or more stabilisers in order to prevent undesired spontaneous polymerisation of the RMs, for example during storage or transport. Suitable types and amounts of stabilisers are known to the person skilled in the art and are described in the literature. Particularly suitable are, for example, the commercially available stabilisers from the Irganox® series (Ciba AG), such as, for example, Irganox® 1076. If stabilisers are employed, their proportion, based on the total amount of RMs or the polymerisable component (component A), is preferably 10-500,000 ppm, particularly preferably 50-50,000 ppm.

The polymerisable compounds of formula I do in particular show good UV absorption in, and are therefore especially suitable for, a process of preparing a PSA display including one or more of the following features:
- the polymerisable medium is exposed to UV light in the display in a 2-step process, including a first UV exposure step ("UV-1 step") to generate the tilt angle, and a second UV exposure step ("UV-2 step") to finish polymerization,
- the polymerisable medium is exposed to UV light in the display generated by an energy-saving UV lamp (also known as "green UV lamps"). These lamps are characterized by a relative low intensity (1/100-1/10 of a conventional UV1 lamp) in their absorption spectra from 300-380nm, and are preferably used in the UV2 step, but are optionally also used in the UV1 step when avoiding high intensity is necessary for the process.
- the polymerisable medium is exposed to UV light in the display generated by a UV lamp with a radiation spectrum that is shifted to longer wavelengths, preferably 340nm or more, to avoid short UV light exposure in the PS-VA process.

Both using lower intensity and a UV shift to longer wavelengths protect the organic layer against damage that may be caused by the UV light.

A preferred embodiment of the present invention relates to a process for preparing a PSA display as described above and below, comprising one or more of the following features:
- the polymerisable LC medium is exposed to UV light in a 2-step process, including a first UV exposure step ("UV-1 step") to generate the tilt angle, and a second UV exposure step ("UV-2 step") to finish polymerization,
- the polymerisable LC medium is exposed to UV light generated by a UV lamp having an intensity of from 0.5 mW/cm² to 10 mW/cm² in the wavelength range from 300-380nm, preferably used in the UV2 step, and optionally also in the UV1 step,
- the polymerisable LC medium is exposed to UV light having a wavelength of 340 nm or more, and preferably 400 nm or less.

This preferred process can be carried out for example by using the desired UV lamps or by using a band pass filter and/or a cut-off filter, which are substantially transmissive for UV light with the respective desired wavelength(s) and are substantially blocking light with the respective undesired wavelengths. For example, when irradiation with UV light of wavelengths λ of 300-400nm is desired, UV exposure can be carried out using a wide band pass filter being substantially transmissive for wavelengths 300nm < λ < 400nm. When irradiation with UV light of wavelength λ of more than 340 nm is desired, UV exposure can be carried out using a cut-off filter being substantially transmissive for wavelengths λ > 340 nm.

"Substantially transmissive" means that the filter transmits a substantial part, preferably at least 50% of the intensity, of incident light of the desired wavelength(s). "Substantially blocking" means that the filter does not transmit a substantial part, preferably at least 50% of the intensity, of incident light of the undesired wavelengths. "Desired (undesired) wavelength" e.g. in case of a band pass filter means the wavelengths inside (outside) the given range of λ, and in case of a cut-off filter means the wavelengths above (below) the given value of λ.

This preferred process enables the manufacture of displays by using longer UV wavelengths, thereby reducing or even avoiding the hazardous and damaging effects of short UV light components.

UV radiation energy is in general from 6 to 100 J, depending on the production process conditions.

Preferably the LC medium according to the present invention does essentially consist of one or more polymerisable compounds of formula I and an LC host mixture as described above and below. However, the LC medium or LC host mixture may additionally comprise one or more further components or additives, preferably selected from the list including but not limited to co-monomers, chiral dopants, polymerisation initiators, inhibitors, stabilizers, surfactants, wetting agents, lubricating agents, dispersing agents, hydrophobing agents, adhesive agents, flow improvers, defoaming agents, deaerators, diluents, reactive diluents, auxiliaries, colourants, dyes, pigments and nanoparticles.

Particular preference is given to LC media comprising one, two or three polymerisable compounds of formula I.

Preference is furthermore given to LC media in which the polymerisable component (component A) comprises exclusively polymerisable compounds of formula I.

Preference is furthermore given to LC media in which the liquid-crystalline component (component B) or the LC host mixture has a nematic LC phase, and preferably has no chiral liquid crystal phase.

Preference is furthermore given to achiral compounds of formula I, and to LC media in which the compounds of component A and/or B are selected exclusively from the group consisting of achiral compounds.

Preferably the proportion of the polymerisable component or component A) in the LC medium is from > 0 to < 5%, very preferably from > 0 to < 1%, most preferably from 0.01 to 0.5%.

Preferably the proportion of compounds of formula I in the LC medium is from >0 to < 5%, very preferably from >0 to < 1%, most preferably from 0.01 to 0.5%.

Preferably the proportion of the liquid-crystalline component or component B) in the LC medium is from 95 to < 100%, very preferably from 99 to <100%.

In a preferred embodiment the polymerisable compounds of the polymerisable component (component B) are exclusively selected from formula I.

In another preferred embodiment the polymerisable component (component B) comprises, in addition to the compounds of formula I, one or more further polymerisable compounds ("co-monomers"), preferably selected from RMs.

Suitable and preferred mesogenic comonomers are selected from the following formulae: 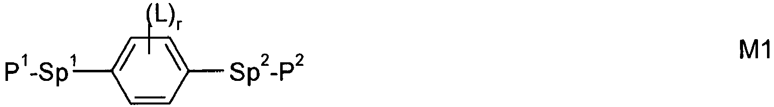 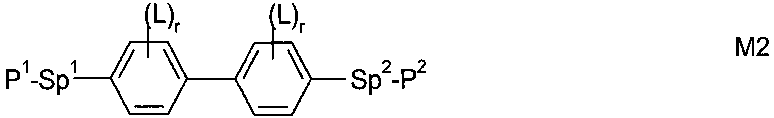 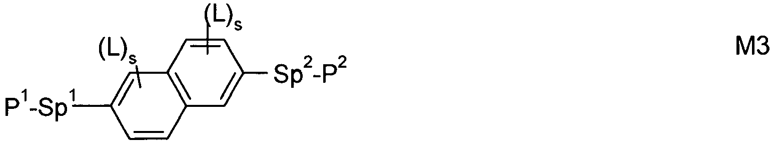 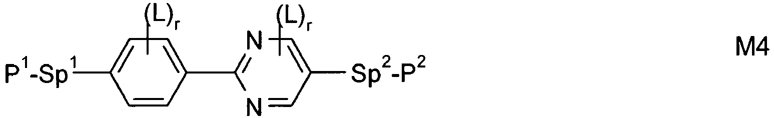 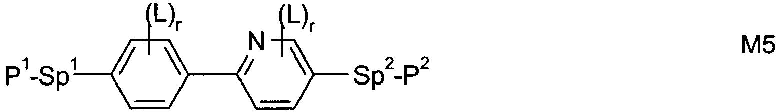 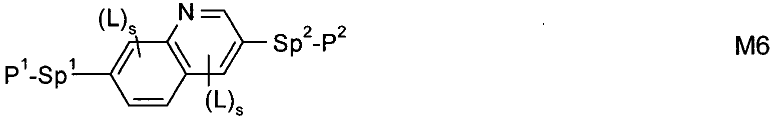 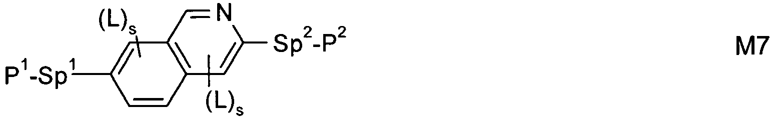 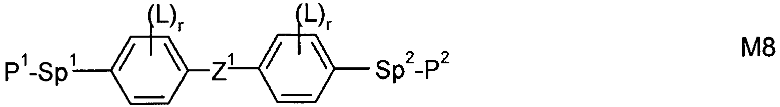 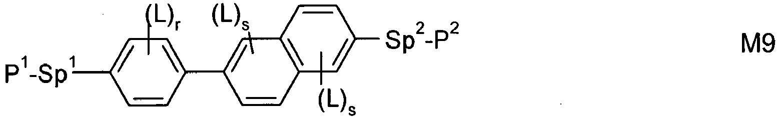 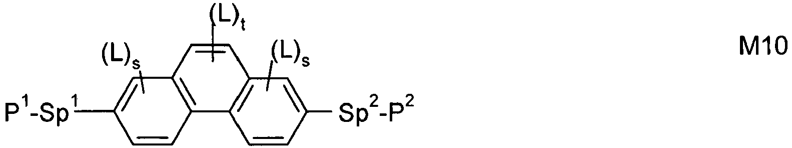 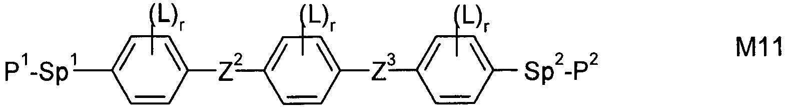 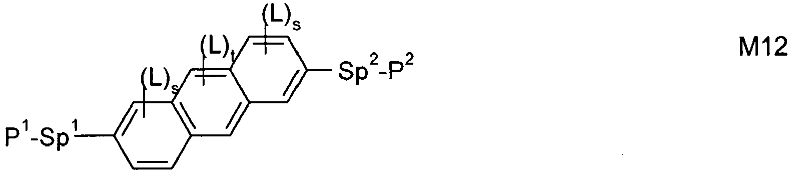 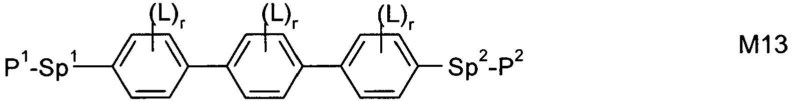 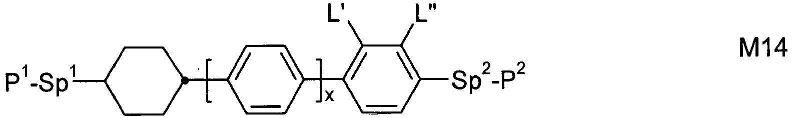 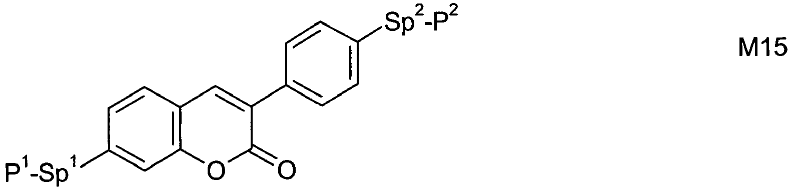 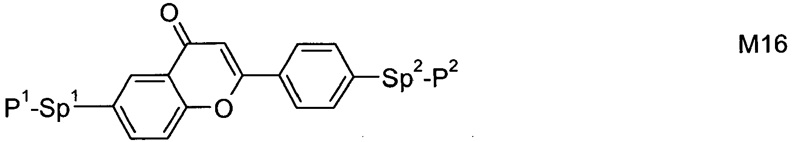 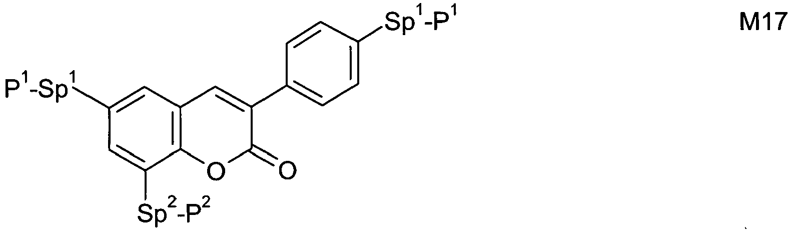 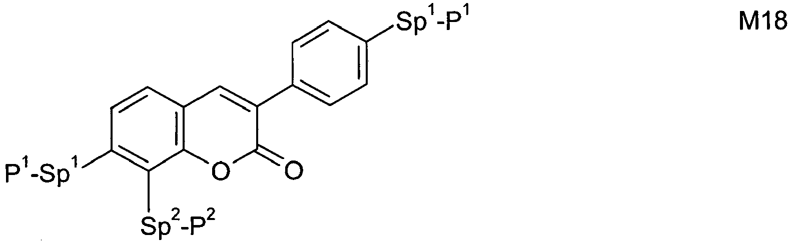 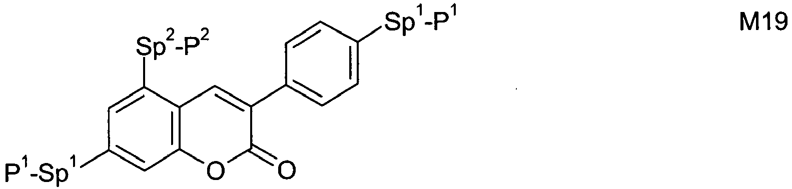 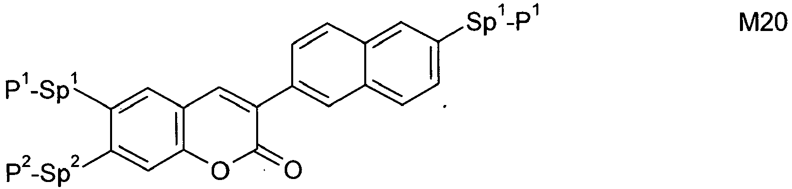 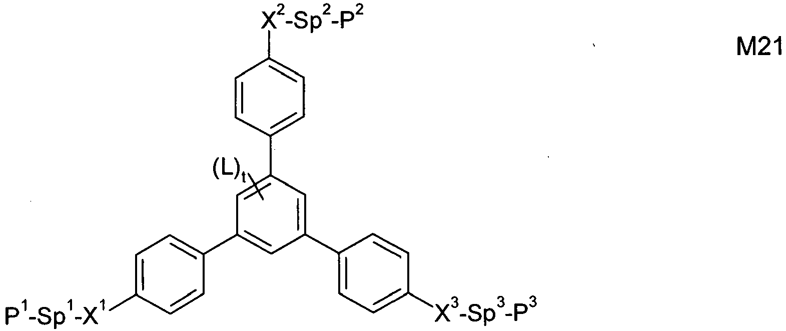 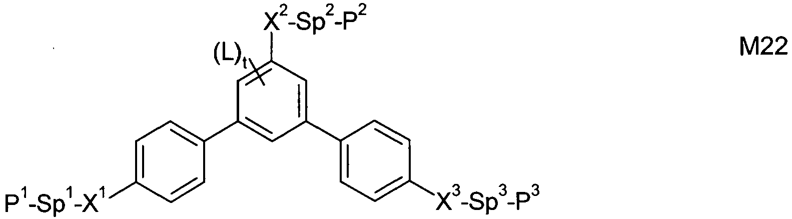 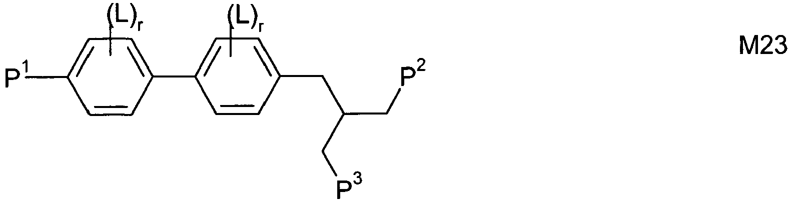 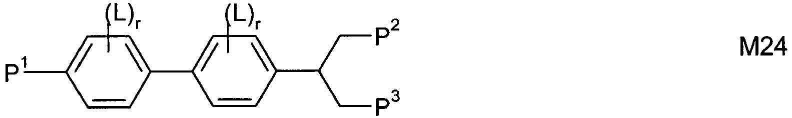 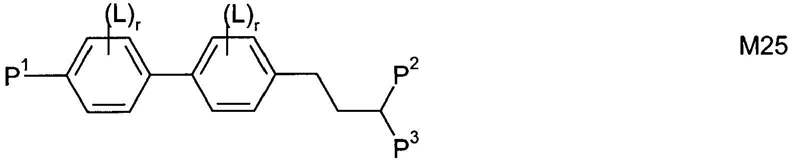 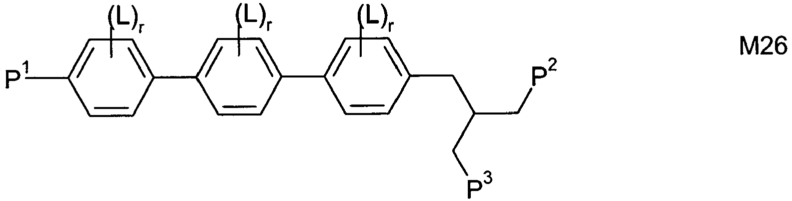 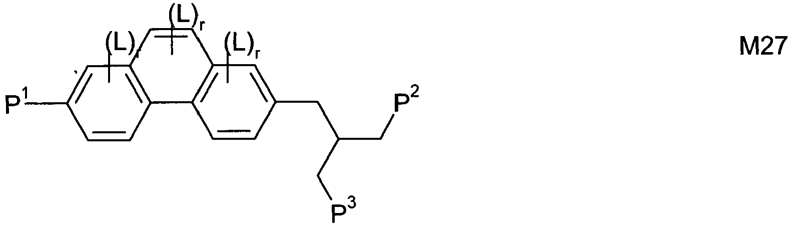 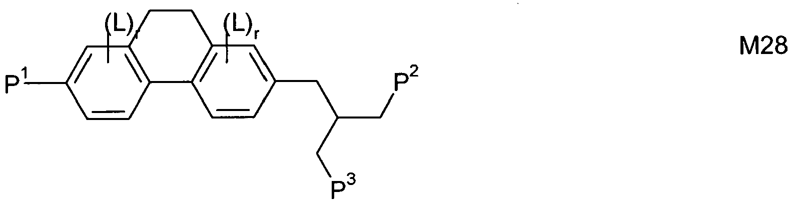 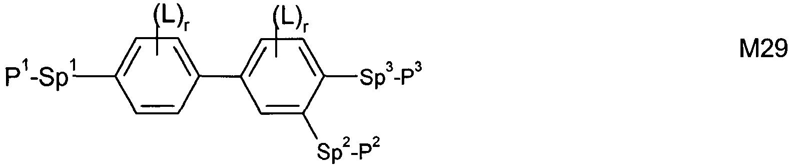 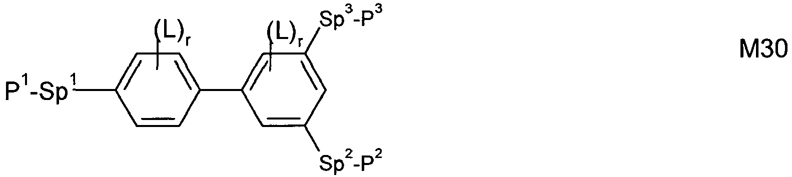 in which the individual radicals have the following meanings:
- P¹, P² and P³: each, independently of one another, denote an acrylate or methacrylate group,
- Sp¹, Sp² and Sp³: each, independently of one another, denote a single bond or a spacer group having one of the meanings indicated above and below for Sp¹, and particularly preferably denote -(CH₂)ₚ₁-, -(CH₂)ₚ₁-O-, -(CH₂)ₚ₁-CO-O- -, -(CH₂)ₚ₁-O-CO- or -(CH₂)ₚ₁-O-CO-O-, in which p1 is an integer from 1 to 12, where, in addition, one or more of the radicals P¹-Sp¹-, P¹-Sp²- and P³-Sp³- may denote R^{aa}, with the proviso that at least one of the radicals P¹-Sp¹-, P²-Sp² and P³-Sp³- present is different from R^{aa},
- R^{aa}: denotes H, F, Cl, CN or straight-chain or branched alkyl having 1 to 25 C atoms, in which, in addition, one or more non-adjacent CH₂ groups may each be replaced, independently of one another, by C(R⁰)=C(R⁰⁰)-, -C≡C-, -N(R⁰)-, -O-, -S-, -CO-, -CO-O-, -O-CO-, -O-CO-O- in such a way that O and/or S atoms are not linked directly to one another, and in which, in addition, one or more H atoms may be replaced by F, CI, CN or P¹-Sp¹-, particularly preferably straight-chain or branched, optionally mono- or polyfluorinated alkyl, alkoxy, alkenyl, alkynyl, alkylcarbonyl, alkoxycarbonyl, alkylcarbonyloxy or alkoxycarbonyloxy having 1 to 12 C atoms (where the alkenyl and alkynyl radicals have at least two C atoms and the branched radicals have at least three C atoms),
- R⁰, R⁰⁰: each, independently of one another and identically or differently on each occurrence, denote H or alkyl having 1 to 12 C atoms,
- R^{y} and R^{z}: each, independently of one another, denote H, F, CH₃ or CF₃,
- X¹, X² and X³: each, independently of one another, denote -CO-O-, -O-CO- or a single bond,
- Z¹: denotes -O-, -CO-, -C(R^{y}R^{z})- or -CF₂CF₂-,
- Z² and Z³: each, independently of one another, denote -CO-O-, -O-CO-, -CH₂O-, -OCH₂-, -CF₂O-, -OCF₂- or -(CH₂)ₙ-, where n is 2, 3 or 4,
- L: on each occurrence, identically or differently, denotes F, CI, CN or straight-chain or branched, optionally mono- or poly-fluorinated alkyl, alkoxy, alkenyl, alkynyl, alkylcarbonyl, alkoxycarbonyl, alkylcarbonyloxy or alkoxycarbonyloxy having 1 to 12 C atoms, preferably F,
- L' and L": each, independently of one another, denote H, F or Cl,
- r: denotes 0, 1, 2, 3 or 4,
- s: denotes 0, 1, 2 or 3,
- t: denotes 0, 1 or 2,
- x: denotes 0 or 1.

Especially preferred are compounds of formulae M2, M13, M17, M23 and M29.

Further preferred are trireactive compounds M15 to M30, in particular M17, M18, M19, M23, M24, M25, M29 and M30.

In the compounds of formulae M1 to M30 the group 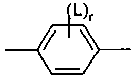 is preferably 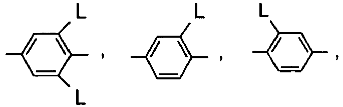 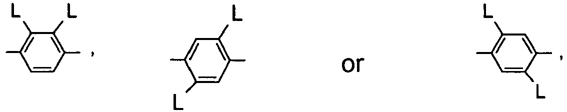 wherein L on each occurrence, identically or differently, has one of the meanings given above or below, and is preferably F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ or P-Sp-, very preferably F, Cl, CN, CH₃, C₂H₅, OCH₃, COCH₃, OCF₃ or P-Sp-, more preferably F, Cl, CH₃, OCH₃, COCH₃ oder OCF₃, especially F or CH₃.

Besides the polymerisable compounds described above, the LC media for use in the LC displays according to the invention comprise an LC mixture ("host mixture") comprising one or more, preferably two or more LC compounds which are selected from low-molecular-weight compounds that are unpolymerisable. These LC compounds are selected such that they stable and/or unreactive to a polymerisation reaction under the conditions applied to the polymerisation of the polymerisable compounds.

In principle, any LC mixture which is suitable for use in conventional displays is suitable as host mixture. Suitable LC mixtures are known to the person skilled in the art and are described in the literature, for example mixtures in VA displays in EP 1 378 557 A1 and mixtures for OCB displays in EP 1 306 418 A1 and DE 102 24 046 A1.

In addition to the polymerisable compounds the LC medium according to the present invention comprises one or more mesogenic or liquid crystalline compounds comprising an alkenyl group, ("alkenyl compound"), where this alkenyl group is preferably stable to a polymerisation reaction under the conditions used for the polymerisation of the polymerisable compounds of formula I or of the other polymerisable compounds contained in the LC medium.

The polymerisable compounds of formula I are especially suitable for use in an LC host mixture that comprises one or more mesogenic or LC compounds comprising an alkenyl group (hereinafter also referred to as "alkenyl compounds"), wherin said alkenyl group is stable to a polymerisation reaction under the conditions used for polymerisation of the compounds of formula I and of the other polymerisable compounds contained in the LC medium. Compared to RMs known from prior art the compounds of formula I do in such an LC host mixture exhibit improved properties, like solubility, reactivity or capability of generating a tilt angle.

The LC host mixture is preferably a nematic LC mixture.

The alkenyl groups in the alkenyl compounds are preferably selected from straight-chain, branched or cyclic alkenyl, in particular having 2 to 25 C atoms, particularly preferably having 2 to 12 C atoms, in which, in addition, one or more non-adjacent CH₂ groups may be replaced by -O-, -S-, -CO-, -CO-O-, -O-CO-, -O-CO-O- in such a way that O and/or S atoms are not linked directly to one another, and in which, in addition, one or more H atoms may be replaced by F and/or CI.

Preferred alkenyl groups are straight-chain alkenyl having 2 to 7 C atoms and cyclohexenyl, in particular ethenyl, propenyl, butenyl, pentenyl, hexenyl, heptenyl, 1,4-cyclohexen-1-yl and 1,4-cyclohexen-3-yl.

The concentration of compounds containing an alkenyl group in the LC host mixture (i.e. without any polymerisable compounds) is preferably from 5% to 100%, very preferably from 20% to 60%.

Especially preferred are LC mixtures containing 1 to 5, preferably 1, 2 or 3 compounds having an alkenyl group.

The mesogenic and LC compounds containing an alkenyl group are preferably selected from the following formulae: 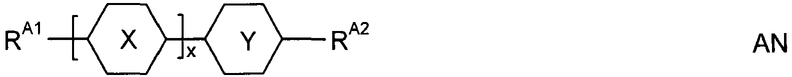 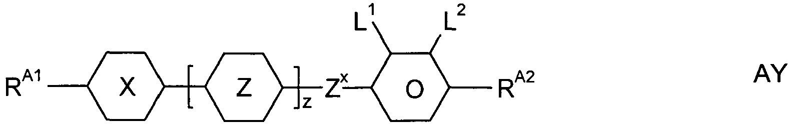 in which the individual radicals, on each occurrence identically or differently, each, independently of one another, have the following meaning: 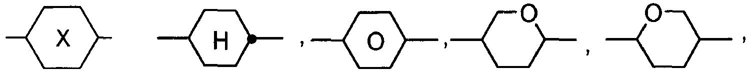 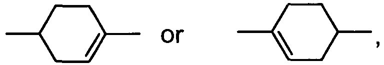 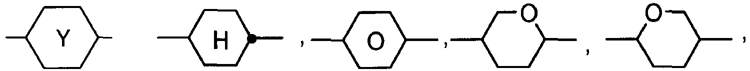 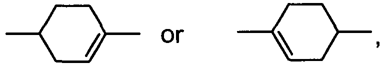 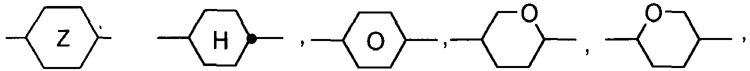 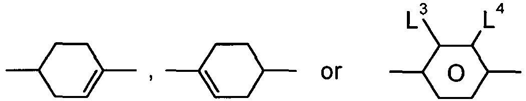
- R^{A1}: alkenyl having 2 to 9 C atoms or, if at least one of the rings X, Y and Z denotes cyclohexenyl, also one of the meanings of R^{A2},
- R^{A2}: alkyl having 1 to 12 C atoms, in which, in addition, one or two non-adjacent CH₂ groups may be replaced by -O-, -CH=CH-, -CO-, -OCO- or -COO- in such a way that O atoms are not linked directly to one another,
- Z^{x}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, or a single bond, preferably a single bond,

- L¹⁻⁴: each, independently of one another, H, F, Cl, OCF₃, CF₃, CH₃, CH₂F or CHF₂H, preferably H, F or CI,
- x: 1 or 2,
- z: 0 or 1.
R^{A2} is preferably straight-chain alkyl or alkoxy having 1 to 8 C atoms or straight-chain alkenyl having 2 to 7 C atoms.

The LC medium preferably comprises no compounds containing a terminal vinyloxy group (-O-CH=CH₂), in particular no compounds of the formula AN or AY in which R^{A1} or R^{A2} denotes or contains a terminal vinyloxy group (-O-CH=CH₂).

Preferably, L¹ and L² denote F, or one of L¹ and L² denotes F and the other denotes Cl, and L³ and L⁴ denote F, or one of L³ and L⁴ denotes F and the other denotes Cl.

The compounds of the formula AN are preferably selected from the following sub-formulae: 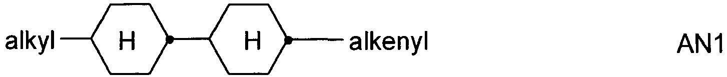 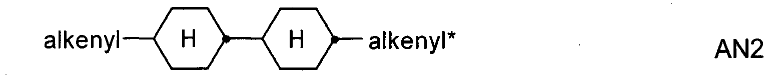 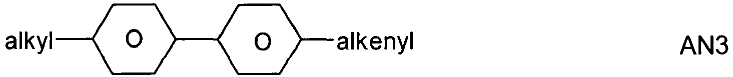 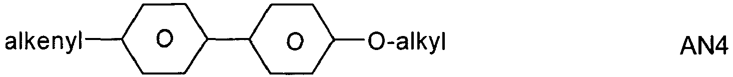 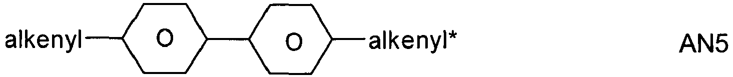 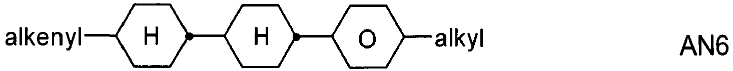 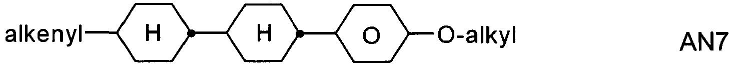 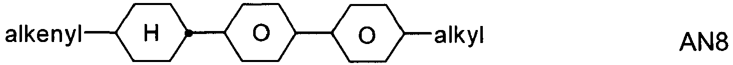 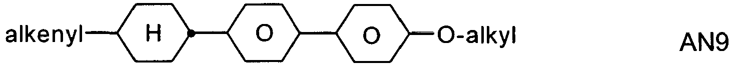 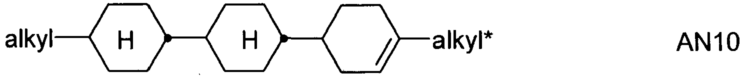 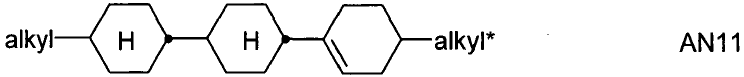 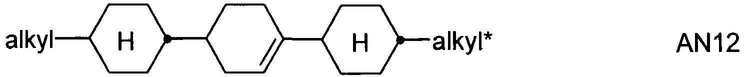 in which alkyl and alkyl* each, independently of one another, denote a straight-chain alkyl radical having 1-6 C atoms, and alkenyl and alkenyl* each, independently of one another, denote a straight-chain alkenyl radical having 2-7 C atoms. Alkenyl and alkenyl* preferably denote CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.

The compounds of the formula AY are preferably selected from the following sub-formulae: 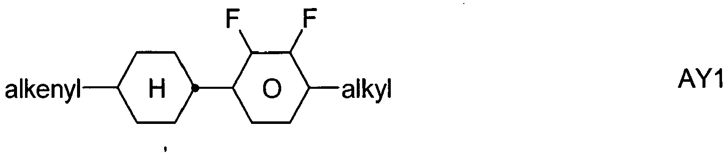 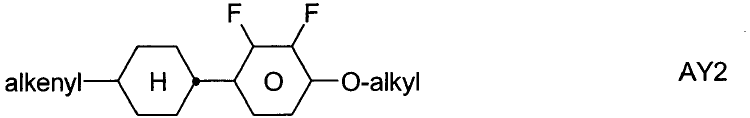 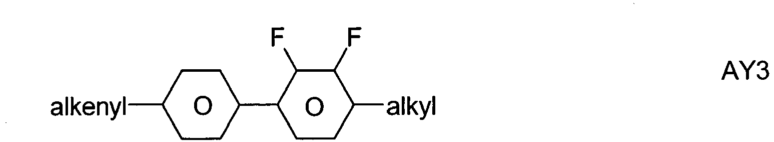 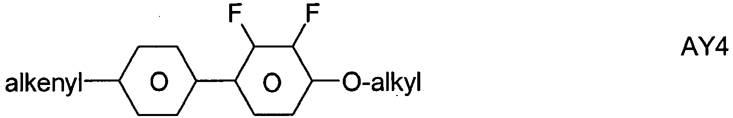 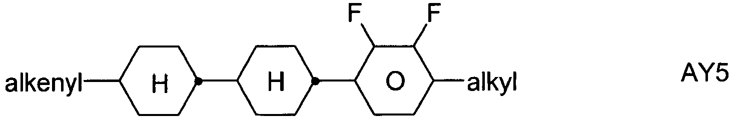 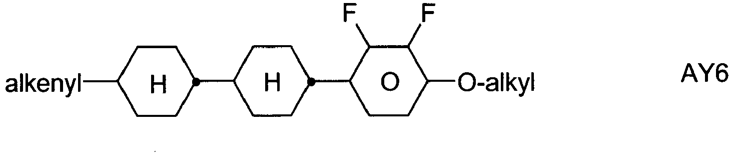 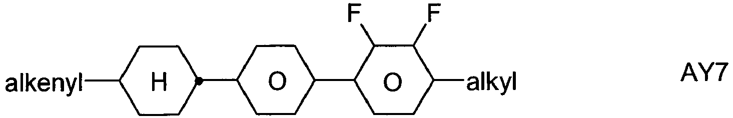 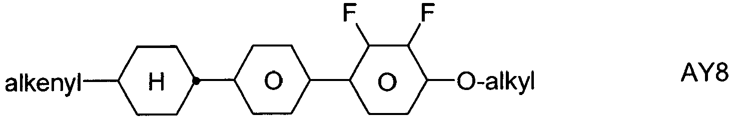 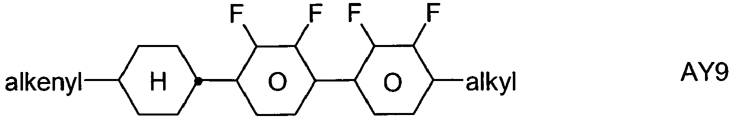 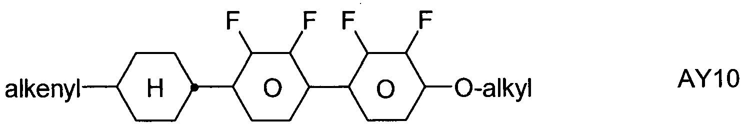 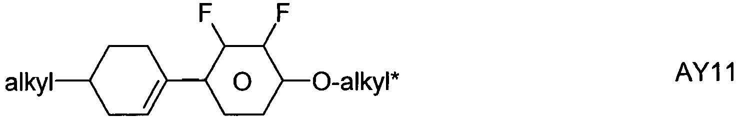 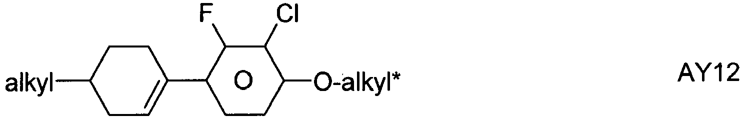 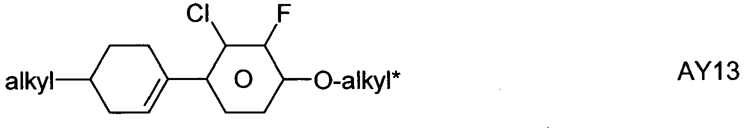 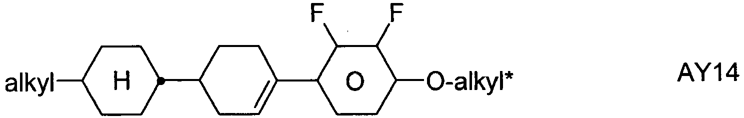 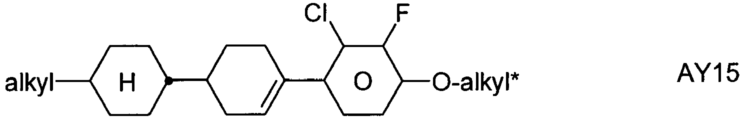 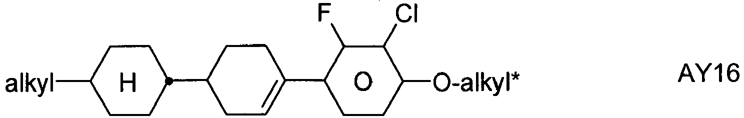 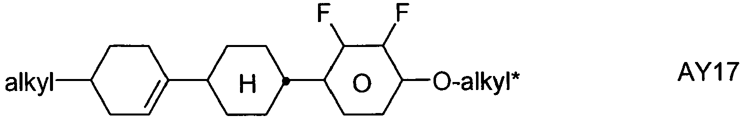 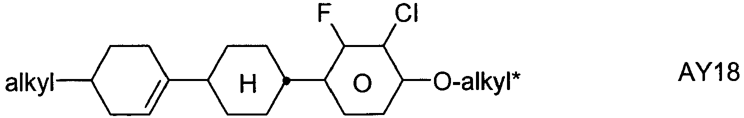 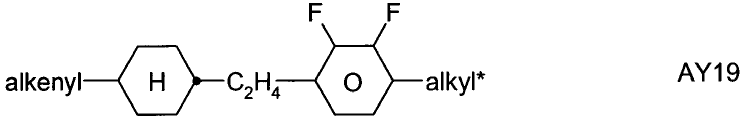 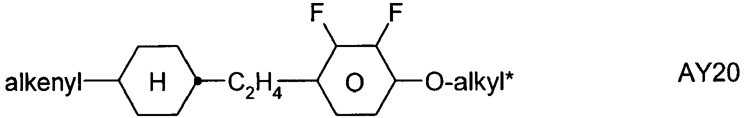 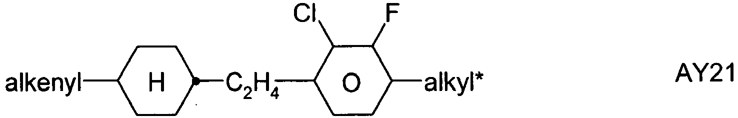 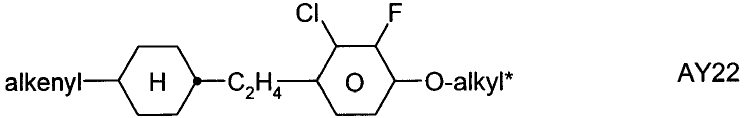 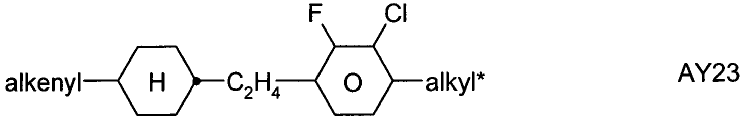 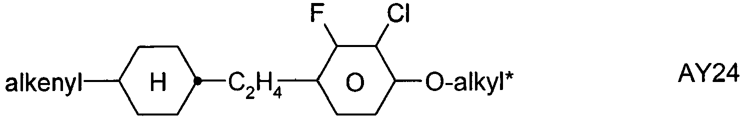 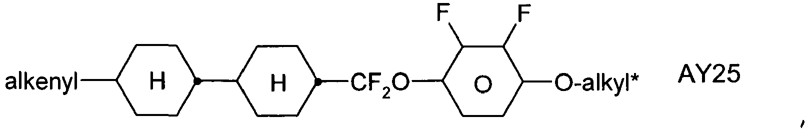 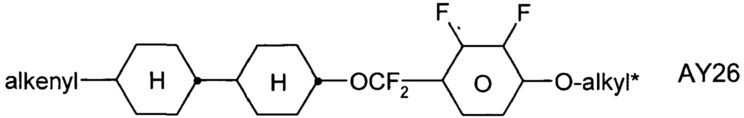 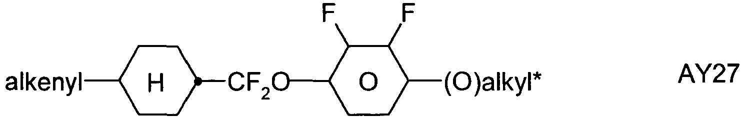 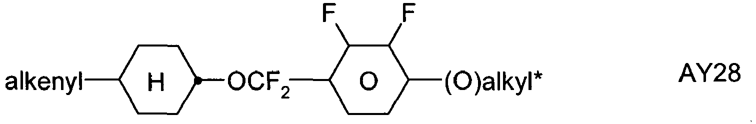 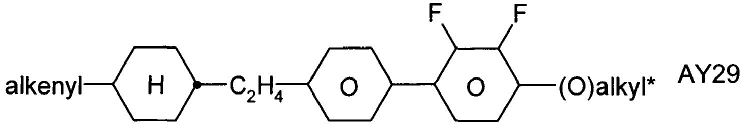 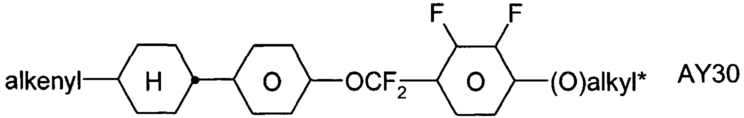 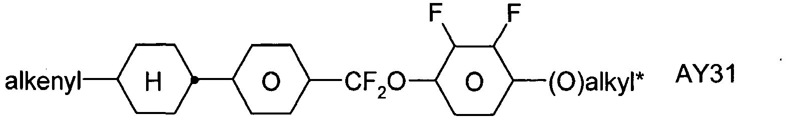 in which alkyl and alkyl* each, independently of one another, denote a straight-chain alkyl radical having 1-6 C atoms, and alkenyl and alkenyl* each, independently of one another, denote a straight-chain alkenyl radical having 2-7 C atoms. Alkenyl and alkenyl* preferably denote CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.

Very preferred compounds of the formula AN are selected from the following sub-formulae: 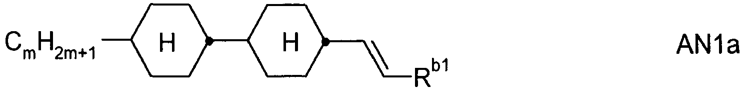 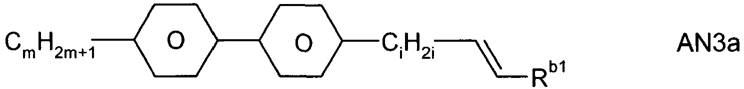 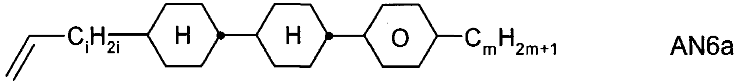 in which m denotes 1, 2, 3, 4, 5 or 6, i denotes 0, 1, 2 or 3, and R^{b1} denotes H, CH₃ or C₂H₅.

Very particularly preferred compounds of the formula AN are selected from the following sub-formulae:

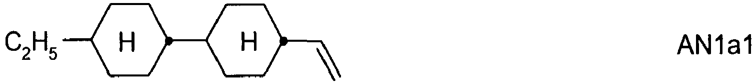

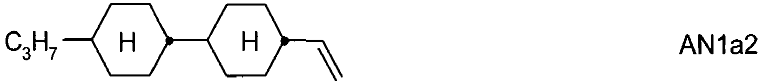

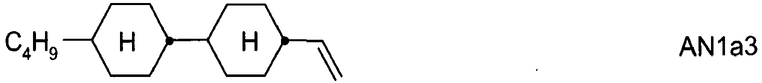

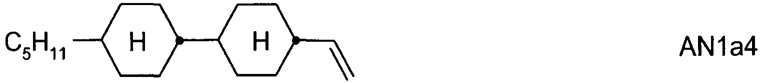

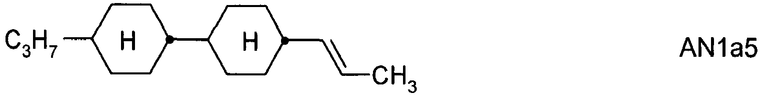

Most preferred are compounds of formula AN1a2 and AN1a5.

Very particularly preferred compounds of the formula AY are selected from the following sub-formulae: 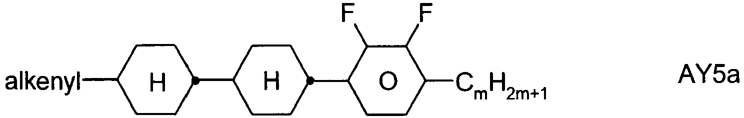 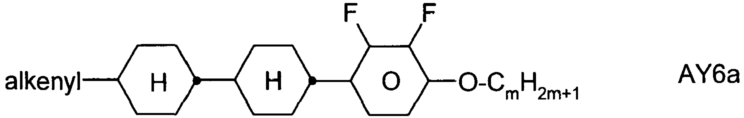 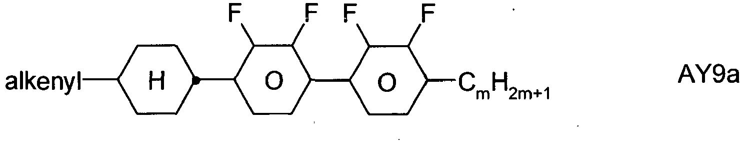 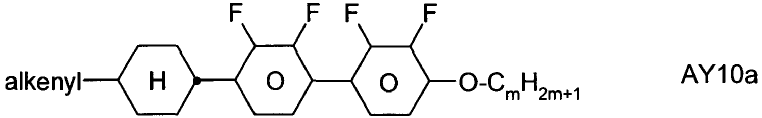 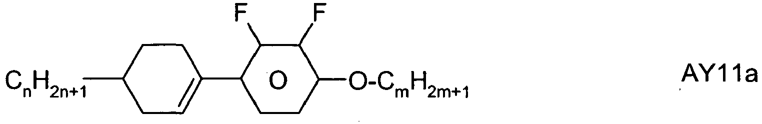 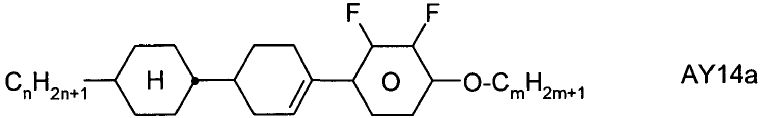 in which m and n each, independently of one another, denote 1, 2, 3, 4, 5 or 6, and alkenyl denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.

In a first preferred embodiment the LC medium contains an LC host mixture based on compounds with negative dielectric anisotropy. Such LC media are especially suitable for use in PS-VA and PS-UB-FFS displays. Particularly preferred embodiments of such an LC medium are those of sections a)-z) below:
a) LC medium which comprises one or more compounds of the formulae CY and/or PY: 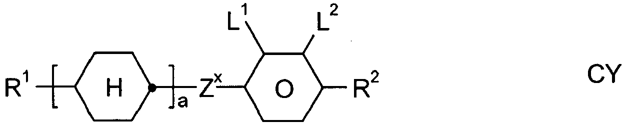 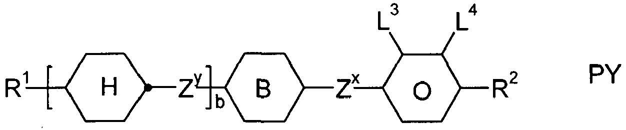 wherein
   - a: denotes 1 or 2,
   - b: denotes 0 or 1,

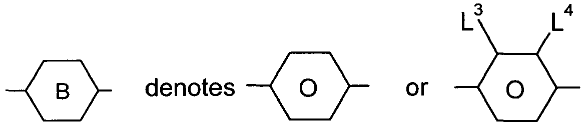

   - R¹ and R²: each, independently of one another, denote alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, preferably alkyl or alkoxy having 1 to 6 C atoms,
   - Z^{x} and Z^{y}: each, independently of one another, denote -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O- or a single bond, preferably a single bond,
   - L¹⁻⁴: each, independently of one another, denote F, Cl, OCF₃, CF₃, CH₃, CH₂F, CHF₂.

   Preferably, both L¹ and L² denote F or one of L¹ and L² denotes F and the other denotes Cl, or both L³ and L⁴ denote F or one of L³ and L⁴ denotes F and the other denotes Cl.
   The compounds of the formula CY are preferably selected from the group consisting of the following sub-formulae: 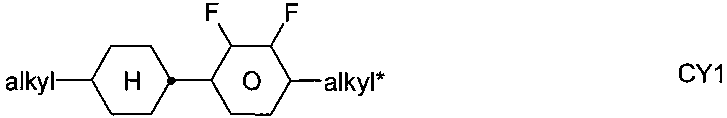 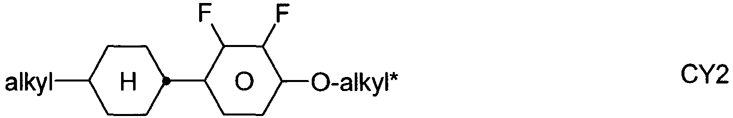 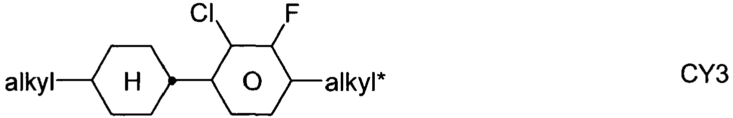 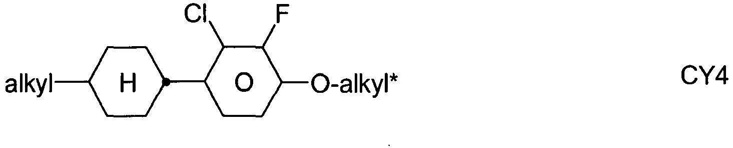 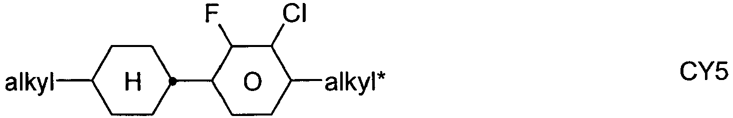 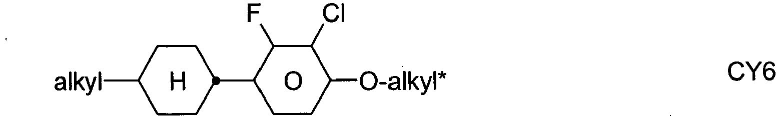 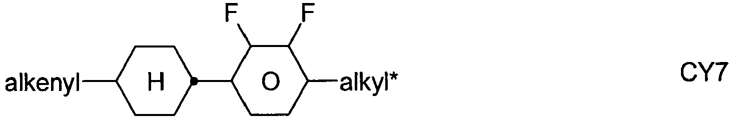 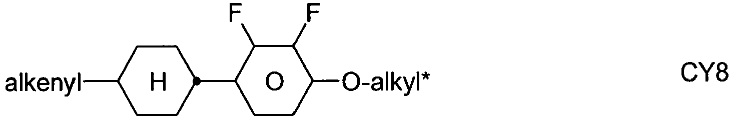 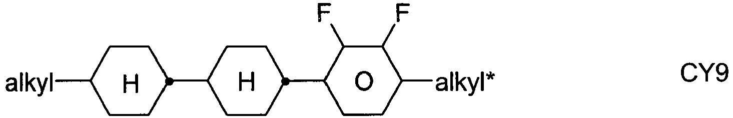 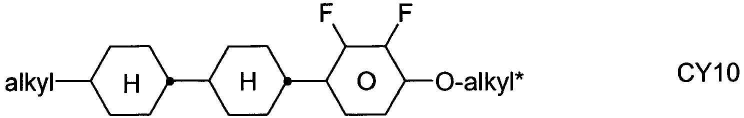 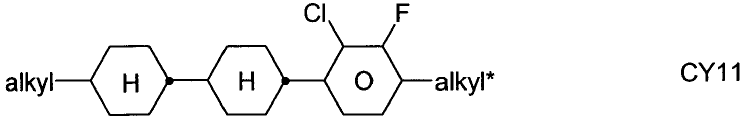 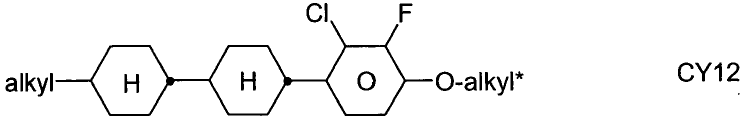 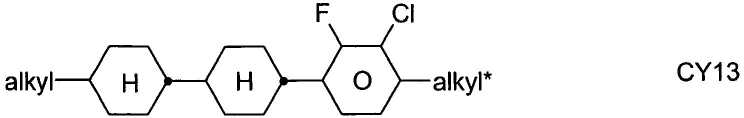 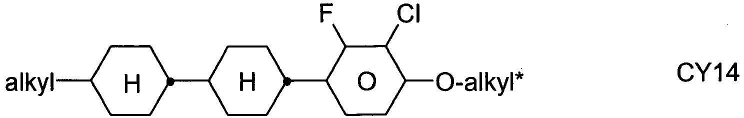 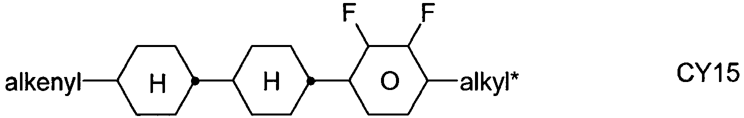 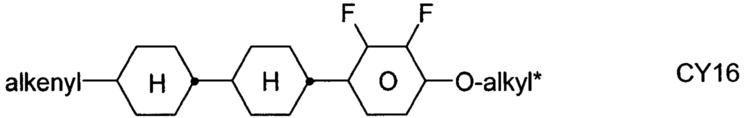 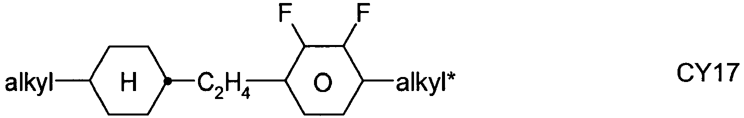 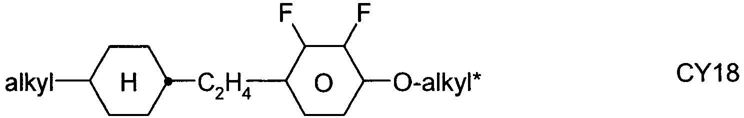 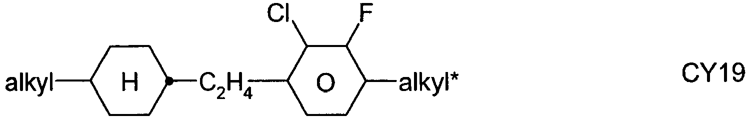 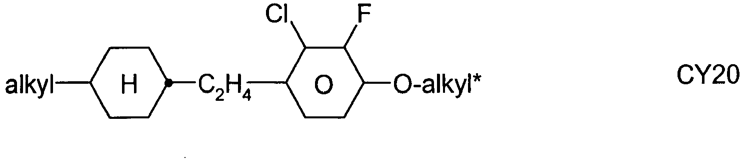 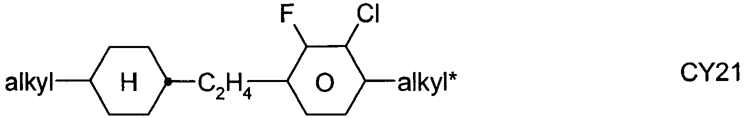 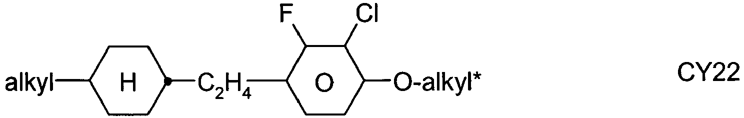 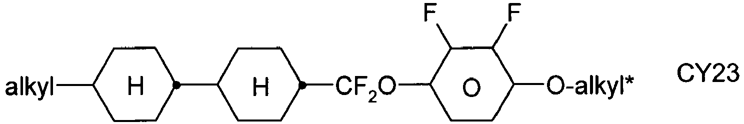 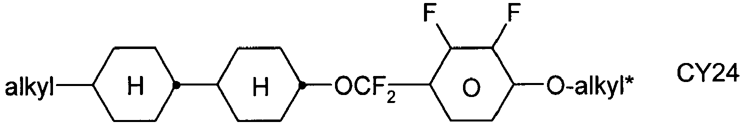 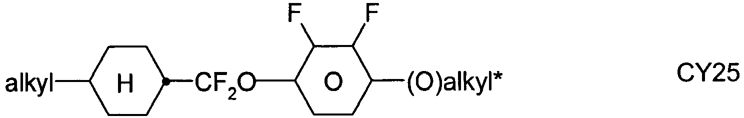 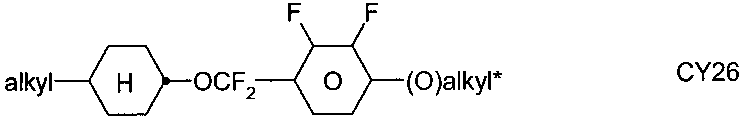 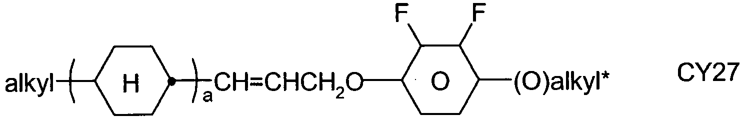 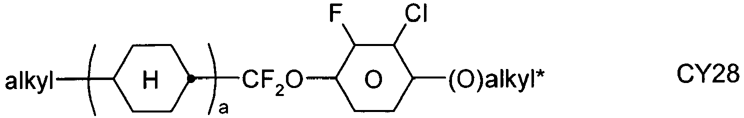 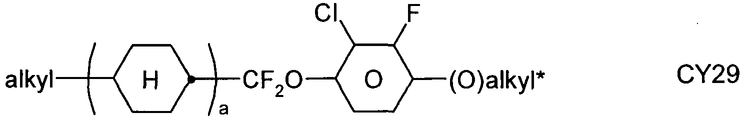 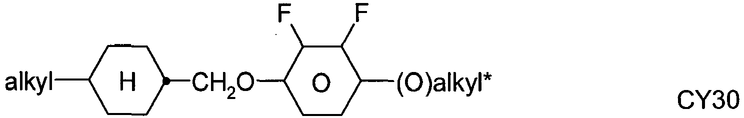 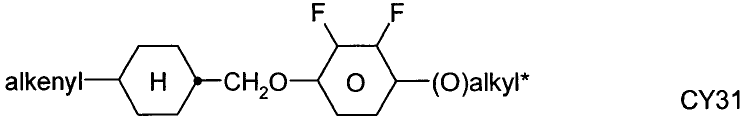 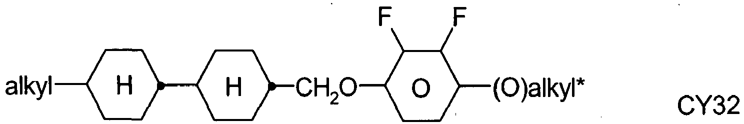 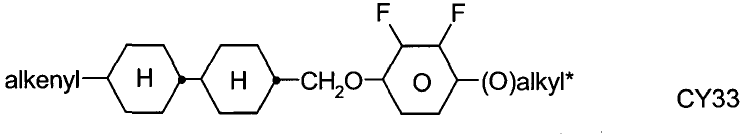 in which a denotes 1 or 2, alkyl and alkyl* each, independently of one another, denote a straight-chain alkyl radical having 1-6 C atoms, and alkenyl denotes a straight-chain alkenyl radical having 2-6 C atoms, and (O) denotes an oxygen atom or a single bond. Alkenyl preferably denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   The compounds of the formula PY are preferably selected from the group consisting of the following sub-formulae: 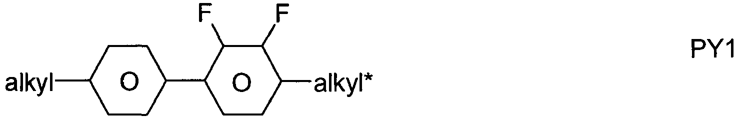 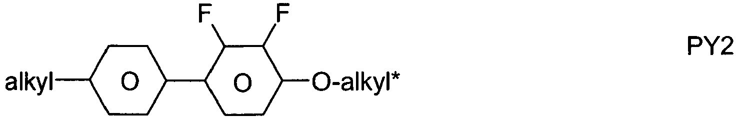 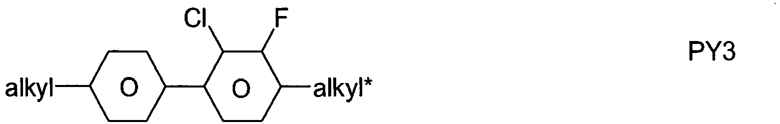 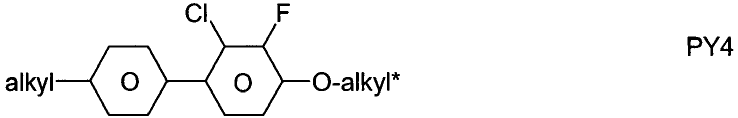 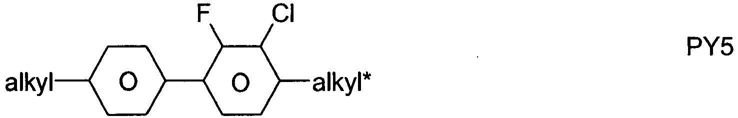 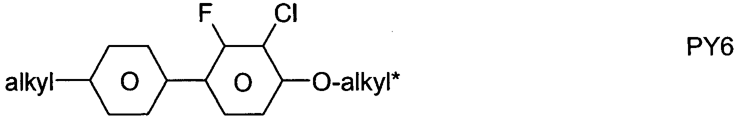 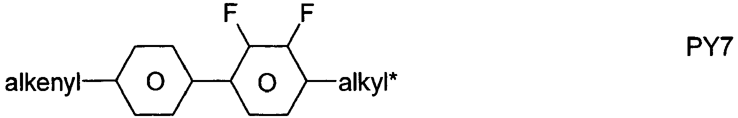 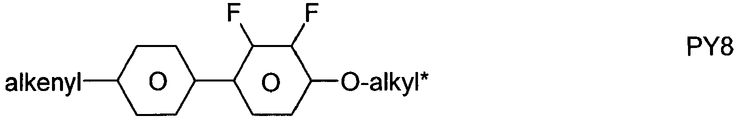 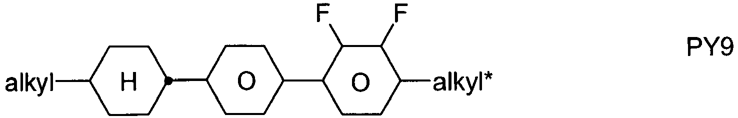 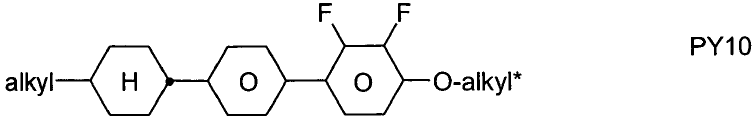 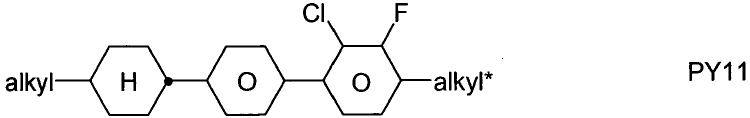 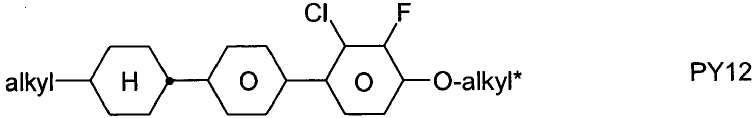 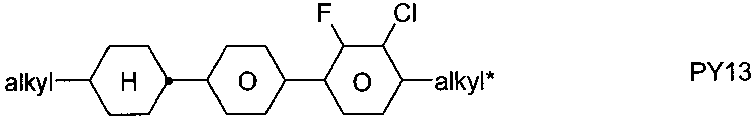 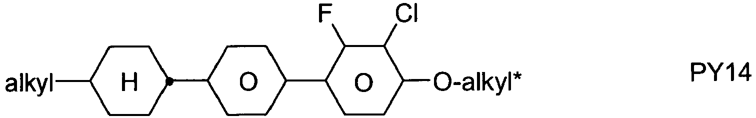 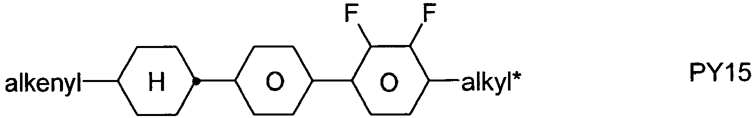 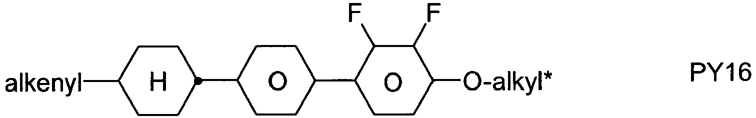 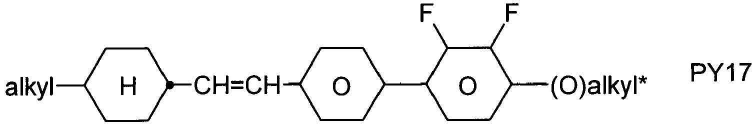 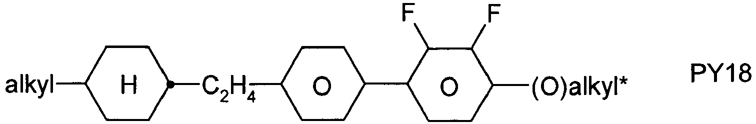 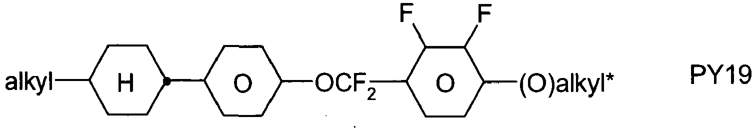 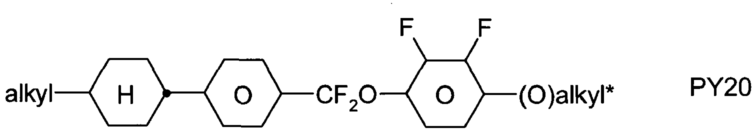 in which alkyl and alkyl* each, independently of one another, denote a straight-chain alkyl radical having 1-6 C atoms, and alkenyl denotes a straight-chain alkenyl radical having 2-6 C atoms, and (O) denotes an oxygen atom or a single bond. Alkenyl preferably denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
b) LC medium which additionally comprises one or more compounds of the following formula: 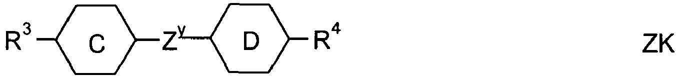 in which the individual radicals have the following meanings: 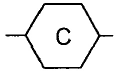 denotes 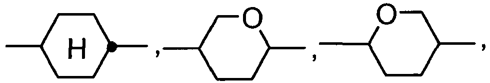 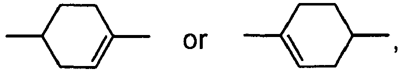 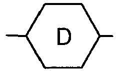 denotes 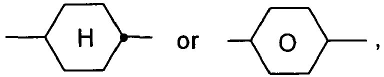
   - R³ and R⁴: each, independently of one another, denote alkyl having 1 to 12 C atoms, in which, in addition, one or two non-adjacent CH₂ groups may be replaced by -O-, -CH=CH-, -CO-, -O-CO- or -CO-O- in such a way that O atoms are not linked directly to one another,
   - Z^{y}: denotes -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-or a single bond, preferably a single bond.

   The compounds of the formula ZK are preferably selected from the group consisting of the following sub-formulae: 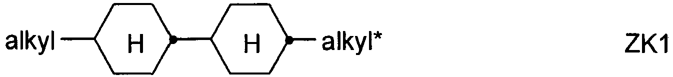 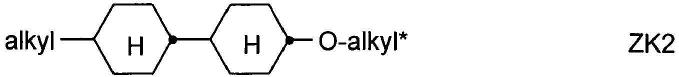 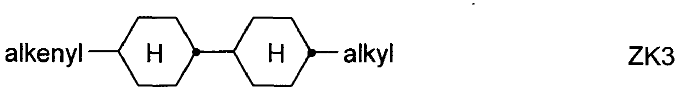 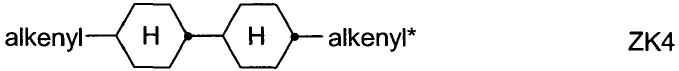 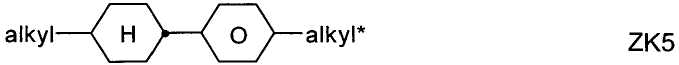 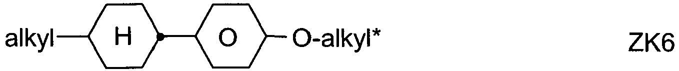 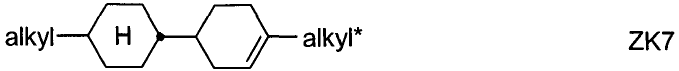 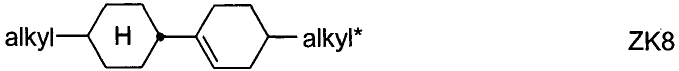 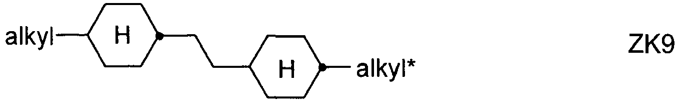 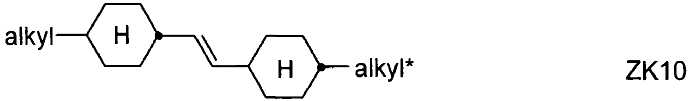 in which alkyl and alkyl* each, independently of one another, denote a straight-chain alkyl radical having 1-6 C atoms, and alkenyl denotes a straight-chain alkenyl radical having 2-6 C atoms. Alkenyl preferably denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   Especially preferred are compounds of formula ZK1.
   Particularly preferred compounds of formula ZK are selected from the following sub-formulae: 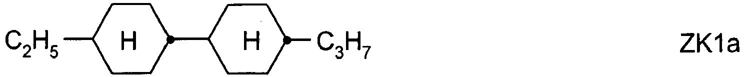 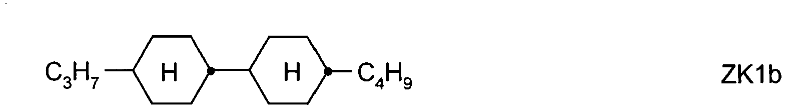 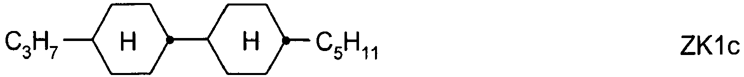 wherein the propyl, butyl and pentyl groups are straight-chain groups.
   Most preferred are compounds of formula ZK1a.
c) LC medium which additionally comprises one or more compounds of the following formula: 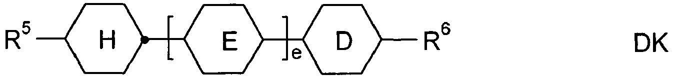 in which the individual radicals on each occurrence, identically or differently, have the following meanings:
   - R⁵ and R⁶: each, independently of one another, denote alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, preferably alkyl or alkoxy having 1 to 6 C atoms, 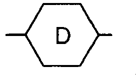 denotes 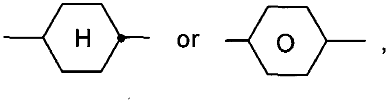 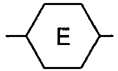 denotes 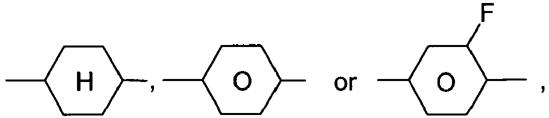 and
   - e: denotes 1 or 2.

   The compounds of the formula DK are preferably selected from the group consisting of the following sub-formulae: 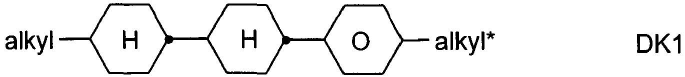 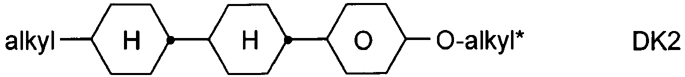 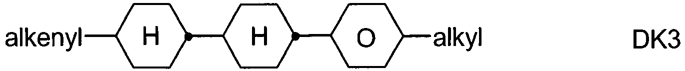 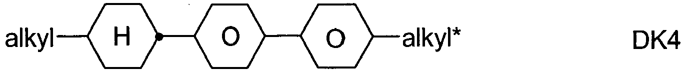 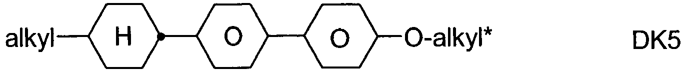 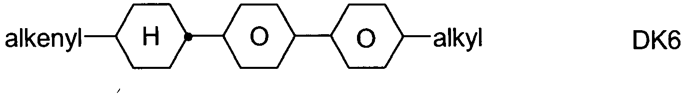 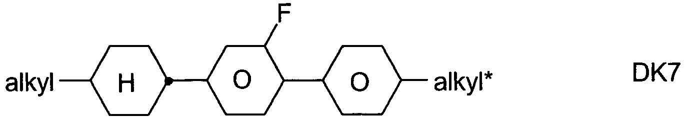 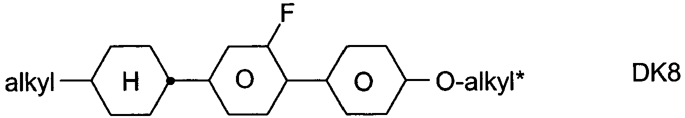 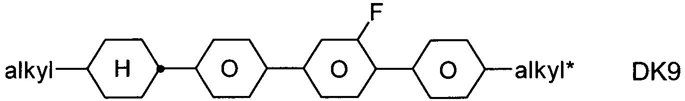 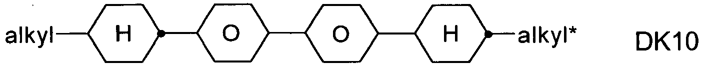 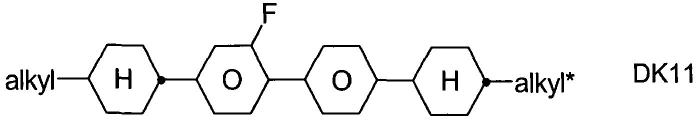 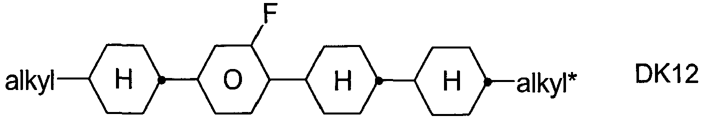 in which alkyl and alkyl* each, independently of one another, denote a straight-chain alkyl radical having 1-6 C atoms, and alkenyl denotes a straight-chain alkenyl radical having 2-6 C atoms. Alkenyl preferably denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
d) LC medium which additionally comprises one or more compounds of the following formula: 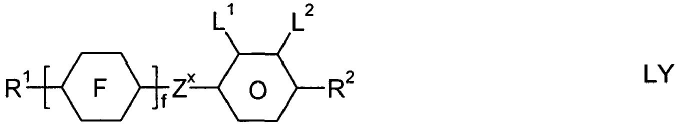 in which the individual radicals have the following meanings: 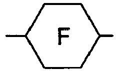 denotes 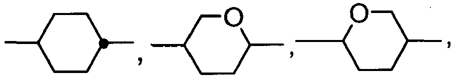 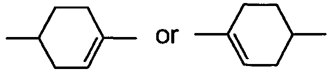 with at least one ring F being different from cyclohexylene,
   - f: denotes 1 or 2,
   - R¹ and R²: each, independently of one another, denote alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another,
   - Z^{x}: denotes -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O- or a single bond, preferably a single bond,
   - L¹ and L²: each, independently of one another, denote F, Cl, OCF₃, CF₃, CH₃, CH₂F, CHF₂.

   Preferably, both radicals L¹ and L² denote F or one of the radicals L¹ and L² denotes F and the other denotes CI.
   The compounds of the formula LY are preferably selected from the group consisting of the following sub-formulae: 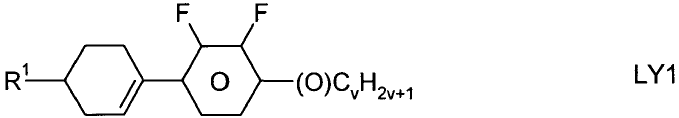 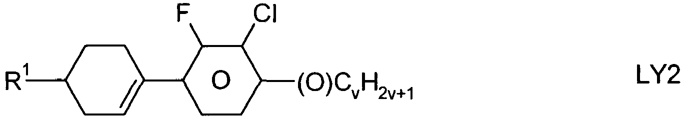 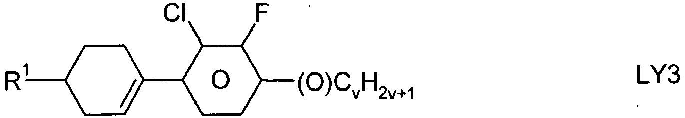 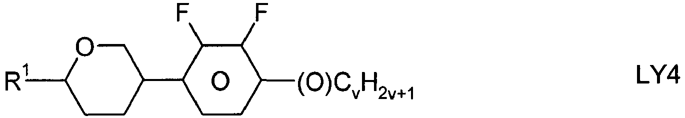 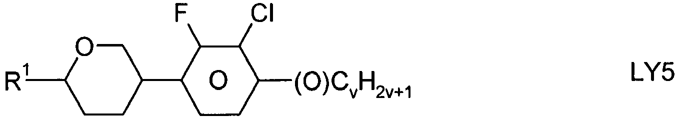 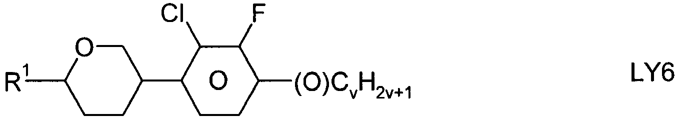 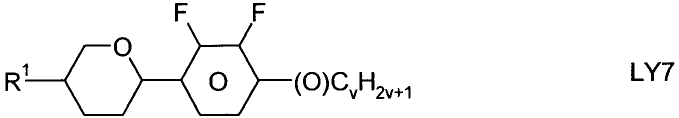 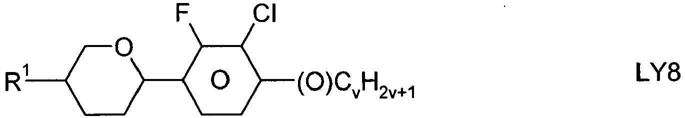 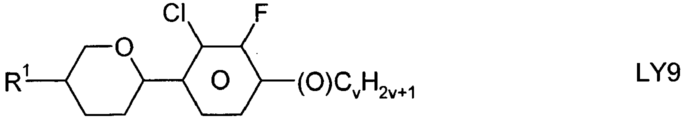 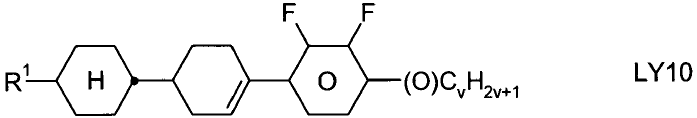 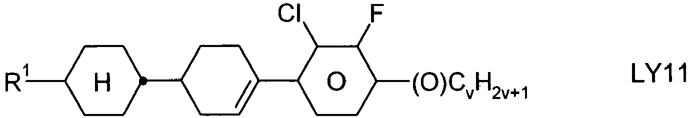 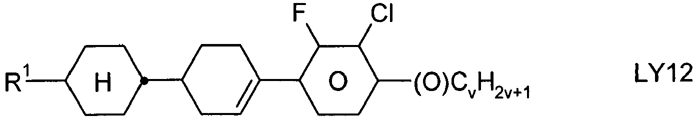 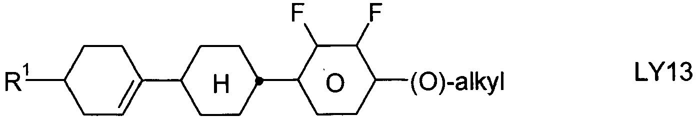 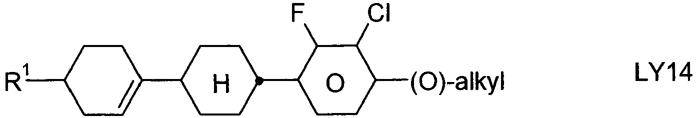 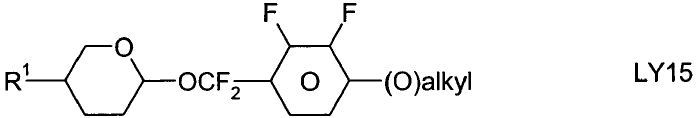 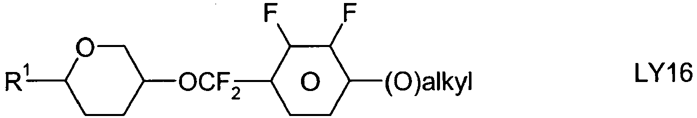 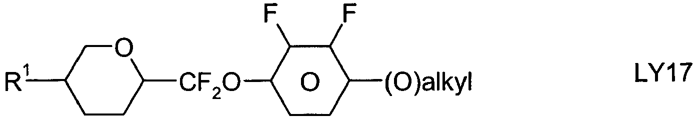 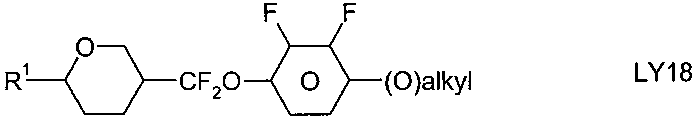 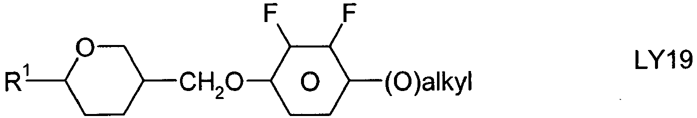 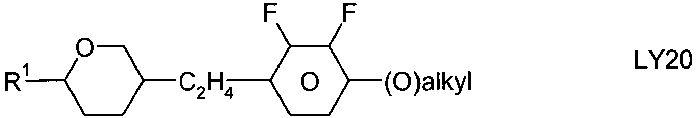 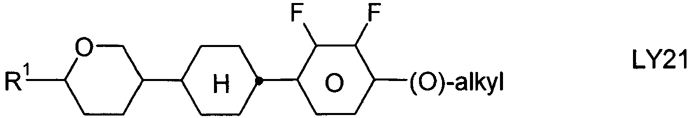 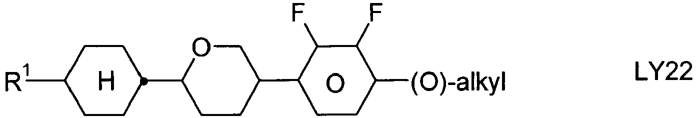 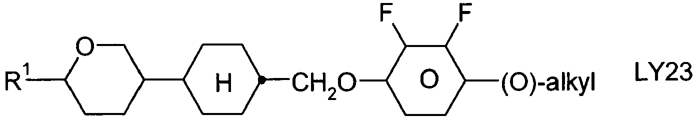 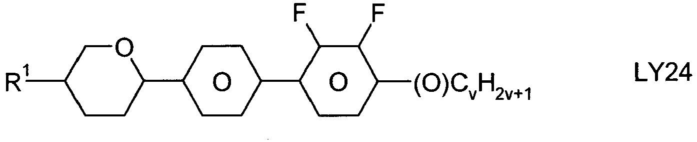 in which R¹ has the meaning indicated above, alkyl denotes a straight-chain alkyl radical having 1-6 C atoms, (O) denotes an oxygen atom or a single bond, and v denotes an integer from 1 to 6. R¹ preferably denotes straight-chain alkyl having 1 to 6 C atoms or straight-chain alkenyl having 2 to 6 C atoms, in particular CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
e) LC medium which additionally comprises one or more compounds selected from the group consisting of the following formulae: 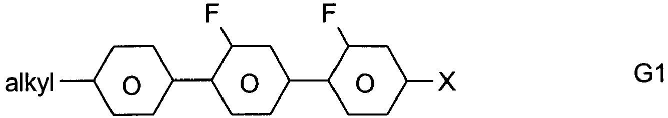 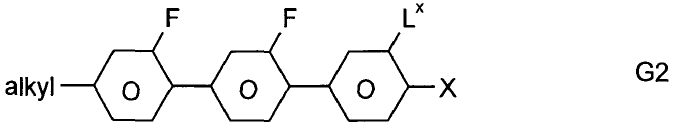 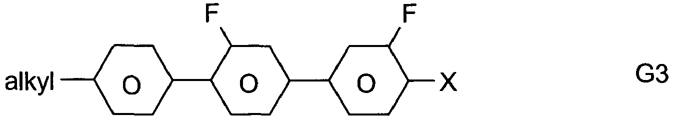 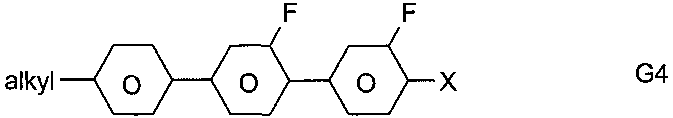 in which alkyl denotes C₁₋₆-alkyl, L^{x} denotes H or F, and X denotes F, Cl, OCF₃, OCHF₂ or OCH=CF₂. Particular preference is given to compounds of the formula G1 in which X denotes F.
f) LC medium which additionally comprises one or more compounds selected from the group consisting of the following formulae: 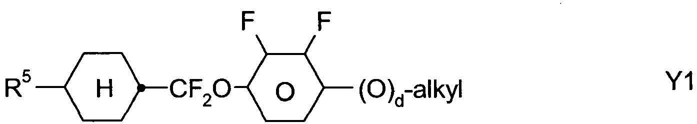 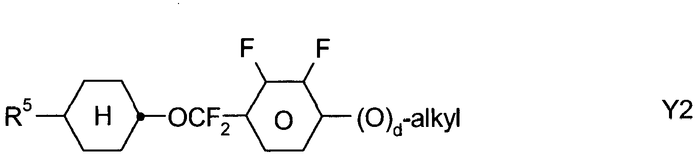 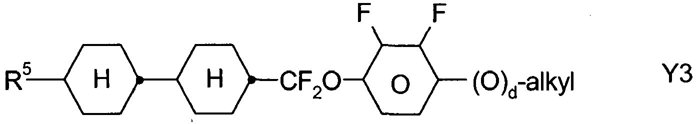 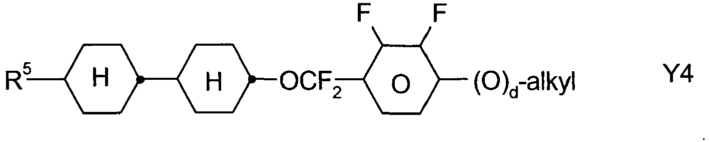 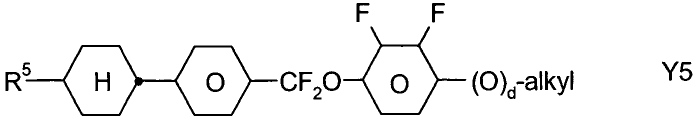 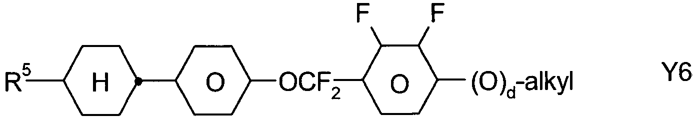 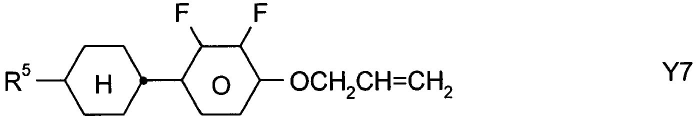 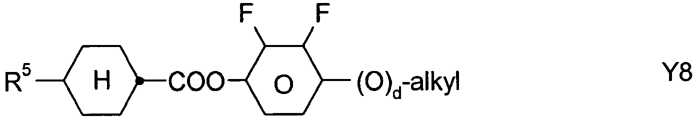 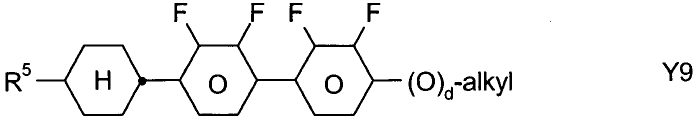 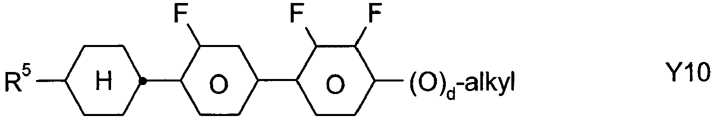 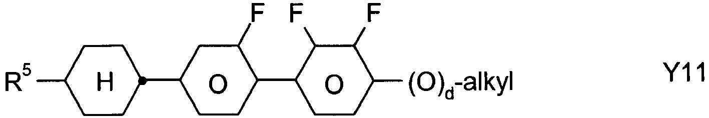 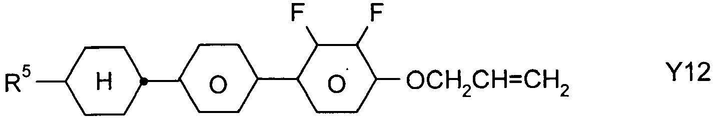 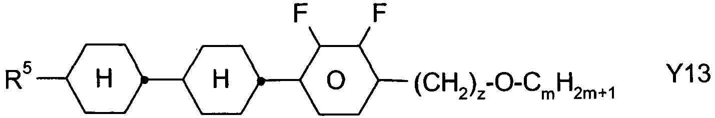 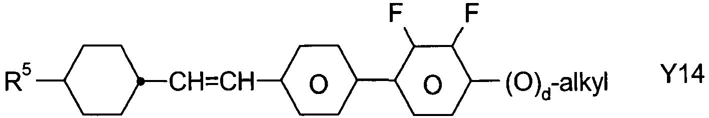 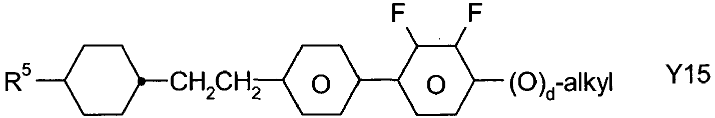 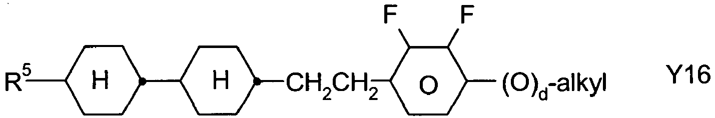 in which R⁵ has one of the meanings indicated above for R¹, alkyl denotes C₁₋₆-alkyl, d denotes 0 or 1, and z and m each, independently of one another, denote an integer from 1 to 6. R⁵ in these compounds is particularly preferably C₁₋₆-alkyl or -alkoxy or C₂₋₆-alkenyl, d is preferably 1. The LC medium according to the invention preferably comprises one or more compounds of the above-mentioned formulae in amounts of ≥ 5% by weight.
g) LC medium which additionally comprises one or more biphenyl compounds selected from the group consisting of the following formulae: 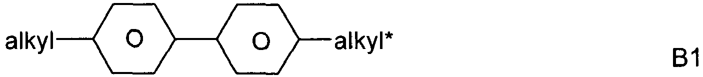 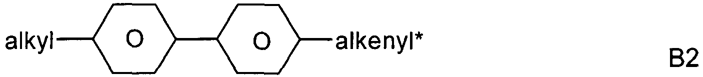 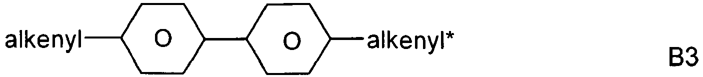 in which alkyl and alkyl* each, independently of one another, denote a straight-chain alkyl radical having 1-6 C atoms, and alkenyl and alkenyl* each, independently of one another, denote a straight-chain alkenyl radical having 2-6 C atoms. Alkenyl and alkenyl* preferably denote CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   The proportion of the biphenyls of the formulae B1 to B3 in the LC mixture is preferably at least 3% by weight, in particular ≥ 5% by weight.
   The compounds of the formula B2 are particularly preferred.
   The compounds of the formulae B1 to B3 are preferably selected from the group consisting of the following sub-formulae: 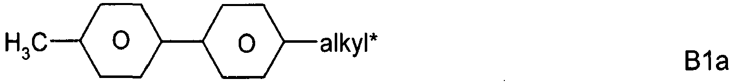 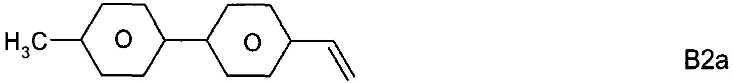 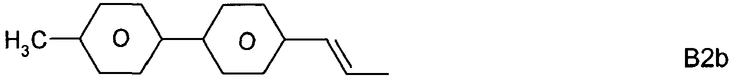 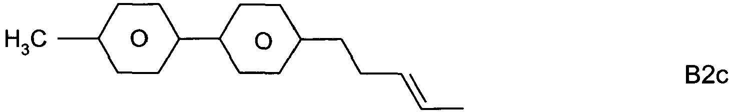 in which alkyl* denotes an alkyl radical having 1-6 C atoms. The medium according to the invention particularly preferably comprises one or more compounds of the formulae B1a and/or B2c.
h) LC medium which additionally comprises one or more terphenyl compounds of the following formula: 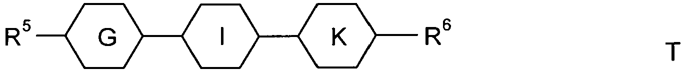 in which R⁵ and R⁶ each, independently of one another, have one of the meanings indicated above, and 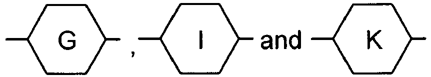 each, independently of one another, denote 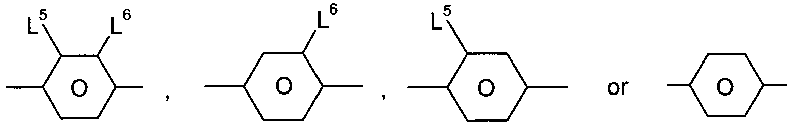 in which L⁵ denotes F or Cl, preferably F, and L⁶ denotes F, Cl, OCF₃, CF₃, CH₃, CH₂F or CHF₂, preferably F.
   The compounds of the formula T are preferably selected from the group consisting of the following sub-formulae: 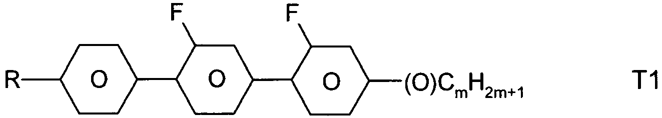 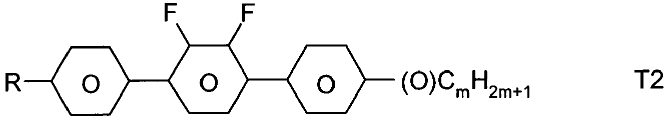 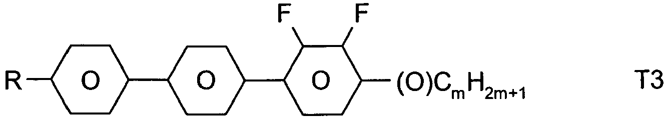 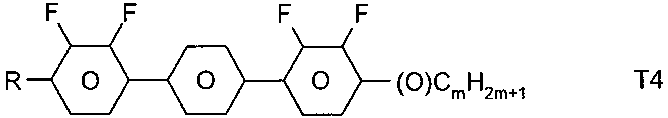 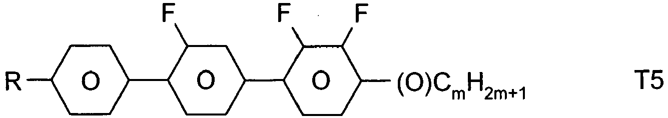 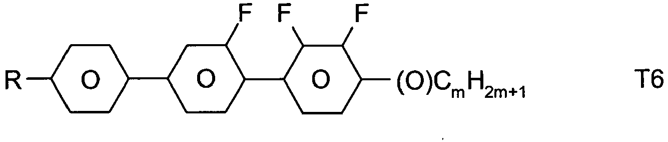 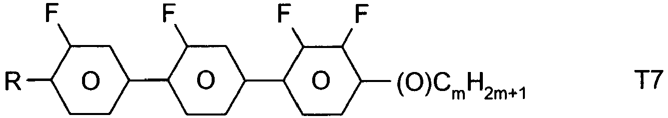 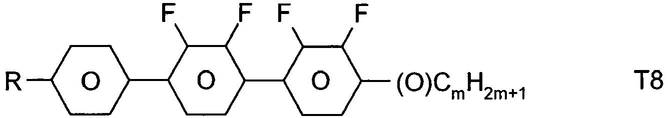 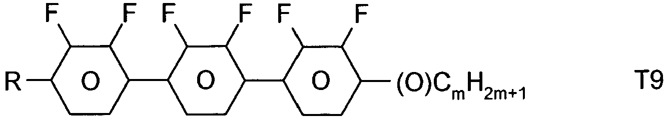 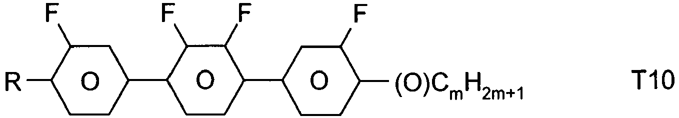 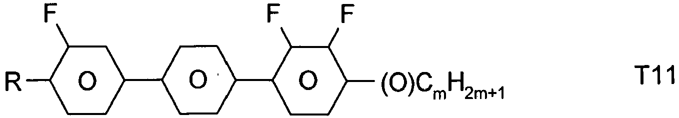 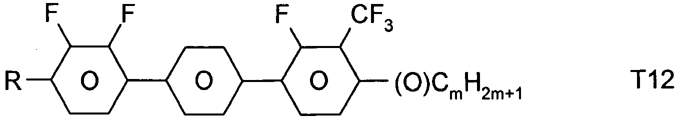 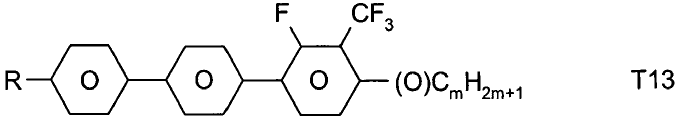 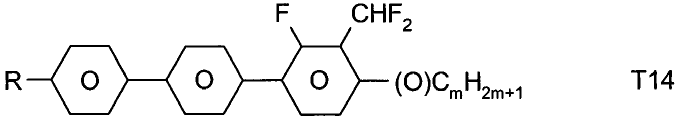 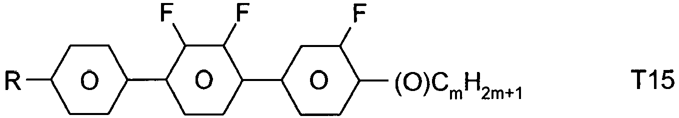 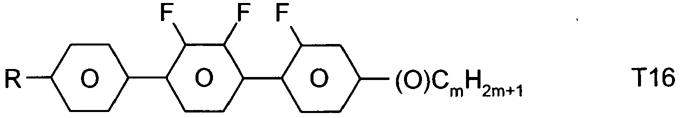 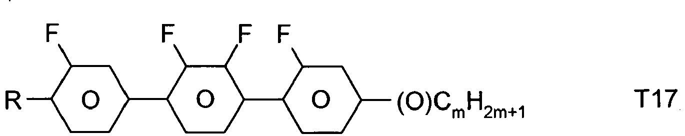 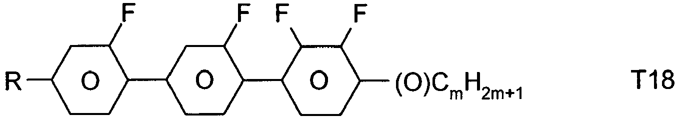 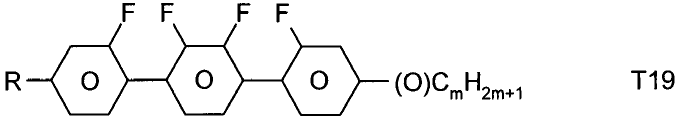 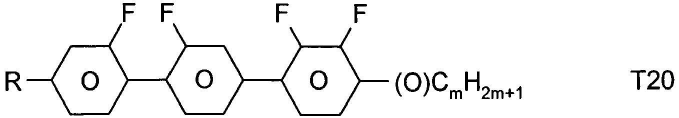 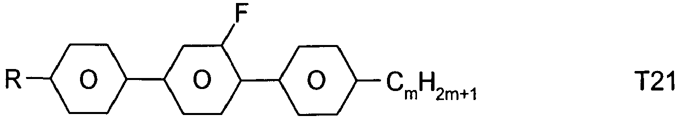 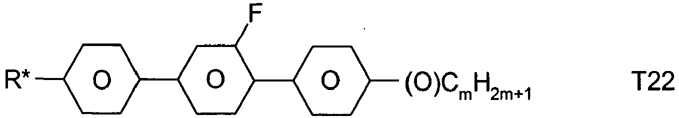 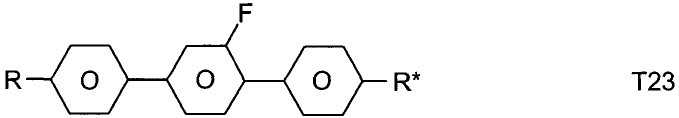 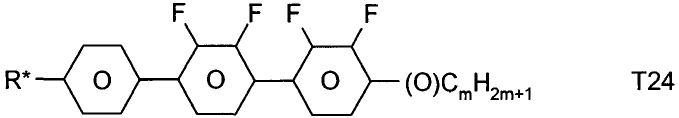 in which R denotes a straight-chain alkyl or alkoxy radical having 1-7 C atoms, R* denotes a straight-chain alkenyl radical having 2-7 C atoms, (O) denotes an oxygen atom or a single bond, and m denotes an integer from 1 to 6. R* preferably denotes CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   R preferably denotes methyl, ethyl, propyl, butyl, pentyl, hexyl, methoxy, ethoxy, propoxy, butoxy or pentoxy.
   The LC medium according to the invention preferably comprises the terphenyls of the formula T and the preferred sub-formulae thereof in an amount of 0.5-30% by weight, in particular 1-20% by weight.
   Particular preference is given to compounds of the formulae T1, T2, T3 and T21. In these compounds, R preferably denotes alkyl, furthermore alkoxy, each having 1-5 C atoms.
   The terphenyls are preferably employed in mixtures according to the invention if the Δn value of the mixture is to be ≥ 0.1. Preferred mixtures comprise 2-20% by weight of one or more terphenyl compounds of the formula T, preferably selected from the group of compounds T1 to T22.
i) LC medium which additionally comprises one or more compounds selected from the group consisting of the following formulae: 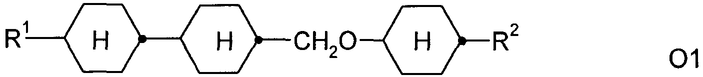 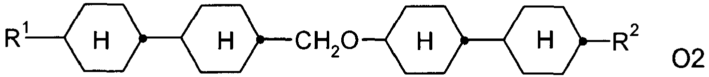 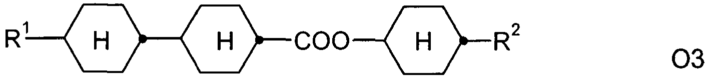 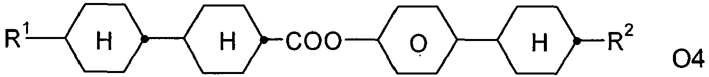 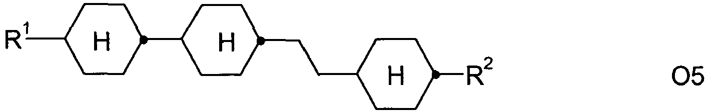 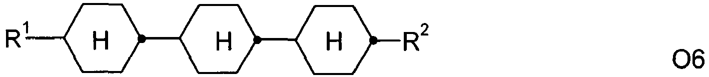 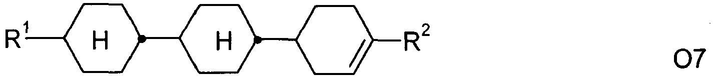 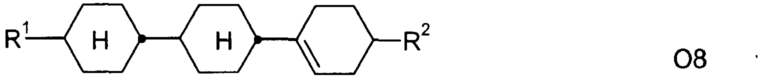 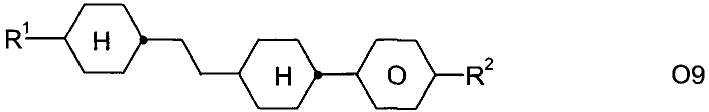 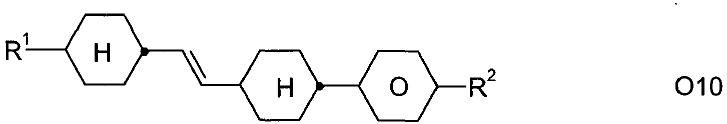 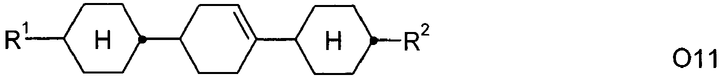 in which R¹ and R² have the meanings indicated above and preferably each, independently of one another, denote straight-chain alkyl having 1 to 6 C atoms or straight-chain alkenyl having 2 to 6 C atoms.
   Preferred media comprise one or more compounds selected from the formulae O1, O3 and O4.
k) LC medium which additionally comprises one or more compounds of the following formula: 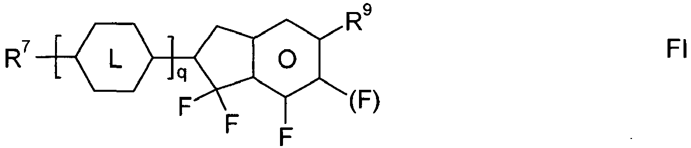 in which 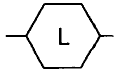 denotes 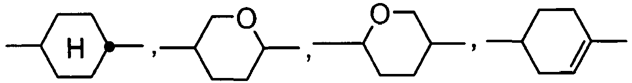 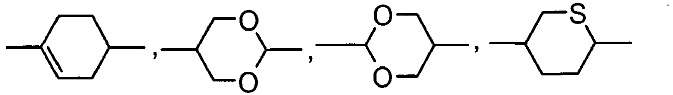 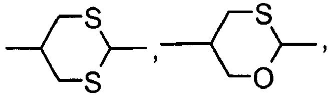 R⁹ denotes H, CH₃, C₂H₅ or n-C₃H₇, (F) denotes an optional fluorine substituent, and q denotes 1, 2 or 3, and R⁷ has one of the meanings indicated for R¹, preferably in amounts of > 3% by weight, in particular ≥ 5% by weight and very particularly preferably 5-30% by weight.
   Particularly preferred compounds of the formula FI are selected from the group consisting of the following sub-formulae: 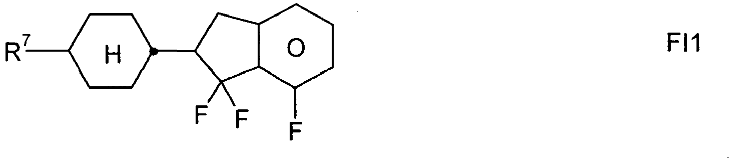 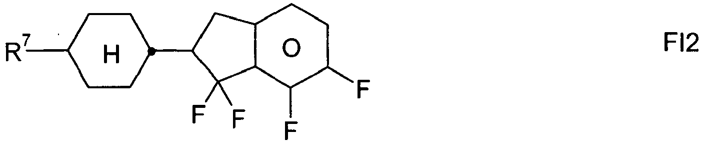 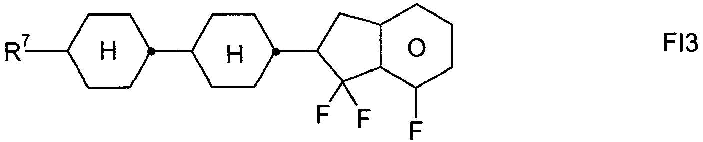 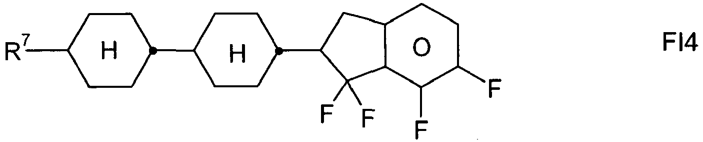 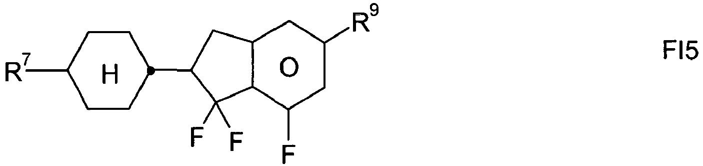 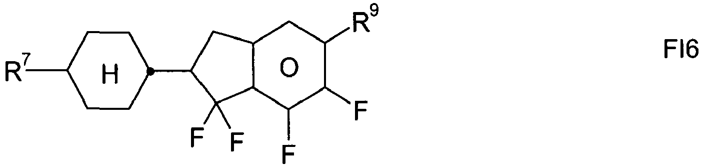 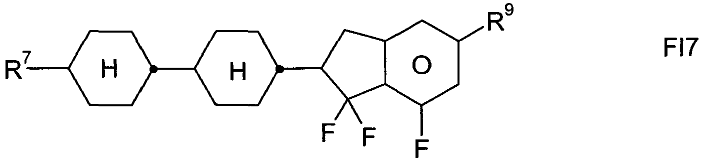 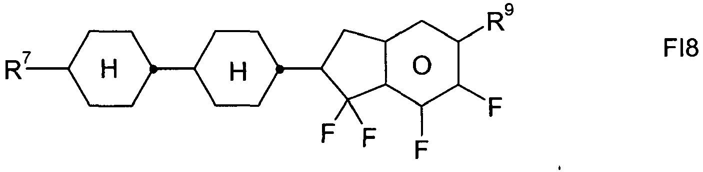 in which R⁷ preferably denotes straight-chain alkyl, and R⁹ denotes CH₃, C₂H₅ or n-C₃H₇. Particular preference is given to the compounds of the formulae FI1, FI2 and FI3.
I) LC medium which additionally comprises one or more compounds selected from the group consisting of the following formulae: 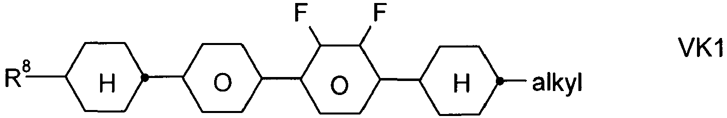 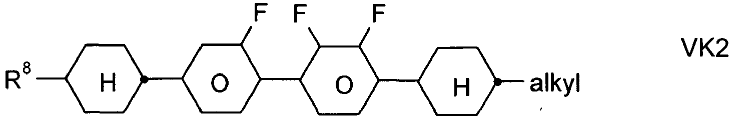 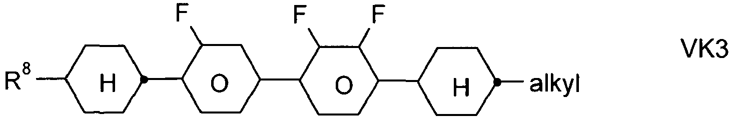 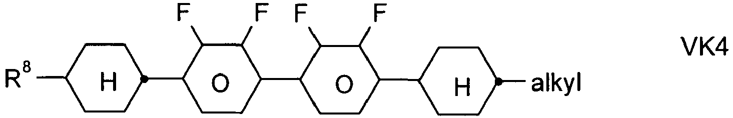 in which R⁸ has the meaning indicated for R¹, and alkyl denotes a straight-chain alkyl radical having 1-6 C atoms.
m) LC medium which additionally comprises one or more compounds which contain a tetrahydronaphthyl or naphthyl unit, such as, for example, the compounds selected from the group consisting of the following formulae: 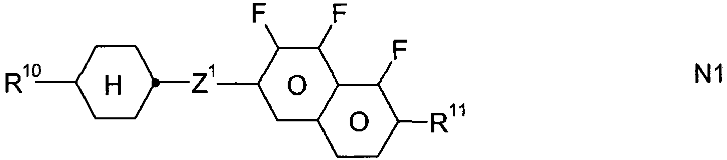 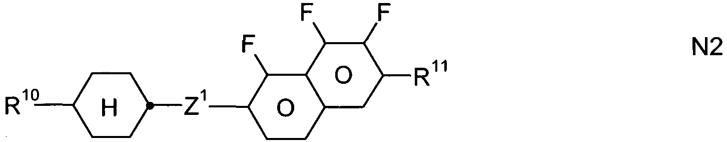 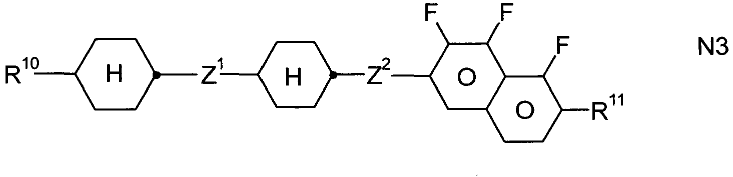 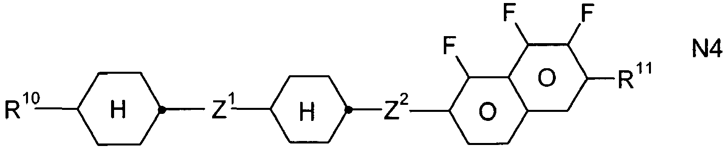 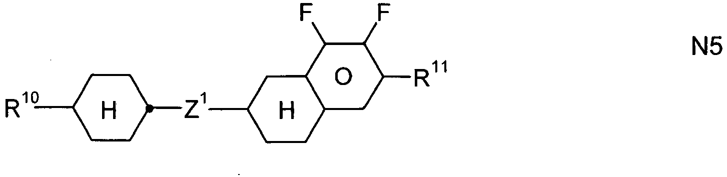 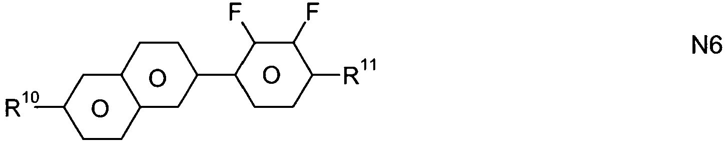 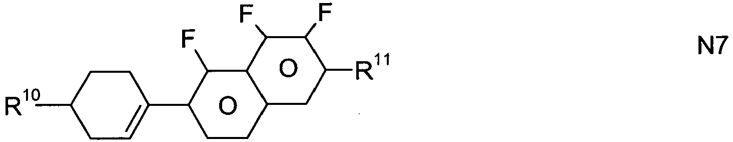 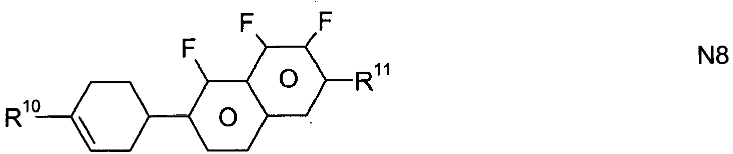 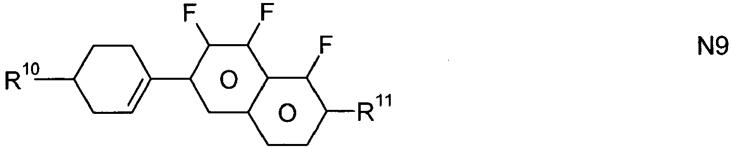 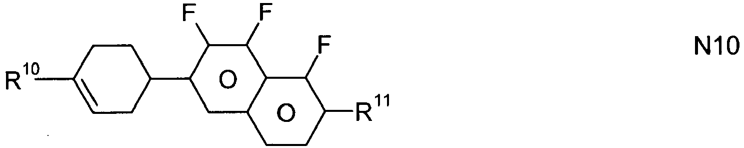 in which
   R¹⁰ and R¹¹ each, independently of one another, denote alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, preferably alkyl or alkoxy having 1 to 6 C atoms,
      and R¹⁰ and R¹¹ preferably denote straight-chain alkyl or alkoxy having 1 to 6 C atoms or straight-chain alkenyl having 2 to 6 C atoms, and
   Z¹ and Z² each, independently of one another, denote -C₂H₄-, -CH=CH-, -(CH₂)₄-, -(CH₂)₃O-, -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- or a single bond.
n) LC medium which additionally comprises one or more difluorodibenzo-chromans and/or chromans of the following formulae: 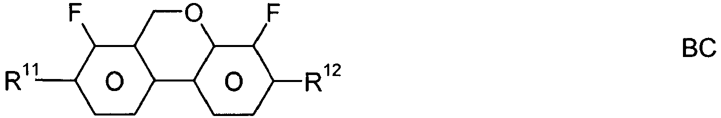 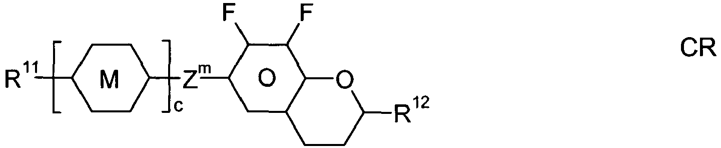 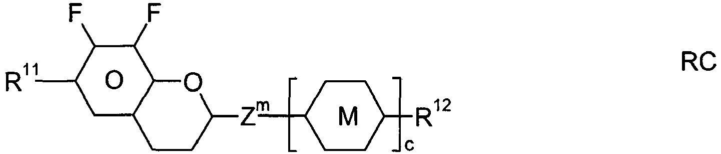 in which
   - R¹¹ and R¹²: each, independently of one another, have one of the meanings indicated above for R¹¹,
   - ring M: is trans-1,4-cyclohexylene or 1,4-phenylene,
   - Z^{m}: -C₂H₄-, -CH₂O-, -OCH₂-, -CO-O- or -O-CO-,
   - c: is 0, 1 or 2,
   preferably in amounts of 3 to 20% by weight, in particular in amounts of 3 to 15% by weight.
   Particularly preferred compounds of the formulae BC, CR and RC are selected from the group consisting of the following sub-formulae: 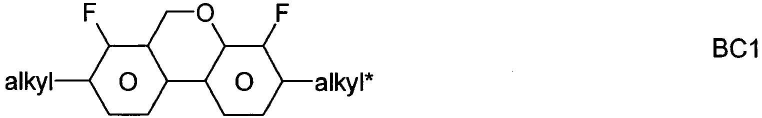 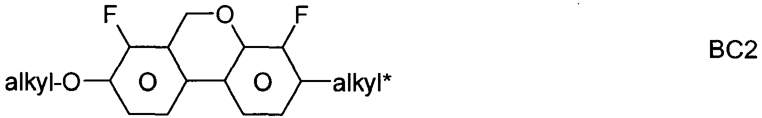 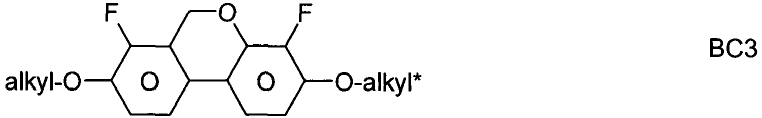 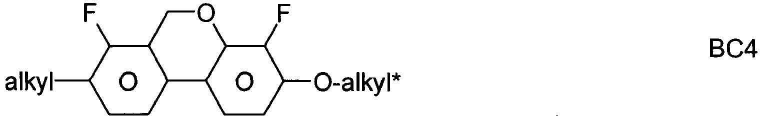 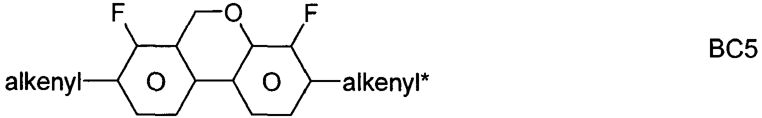 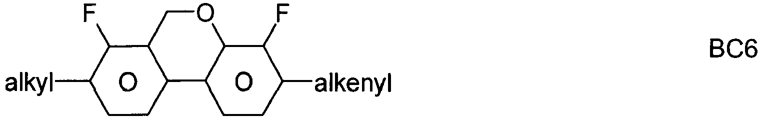 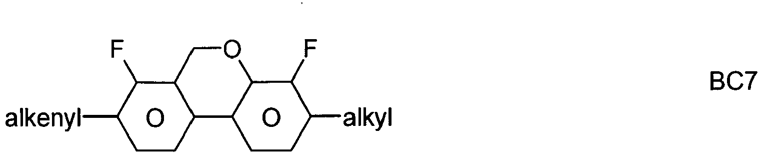 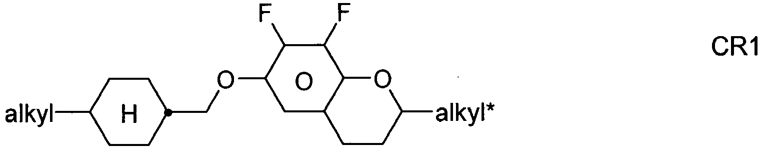 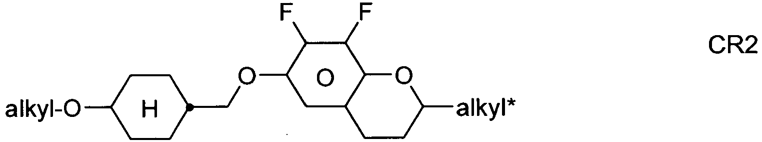 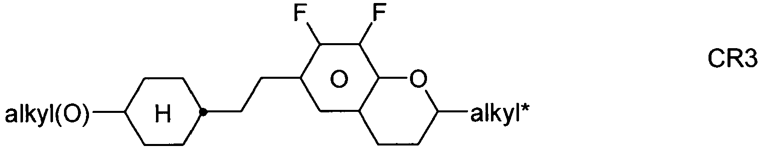 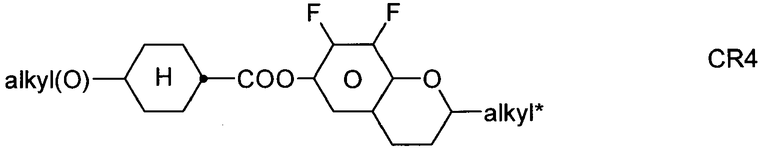 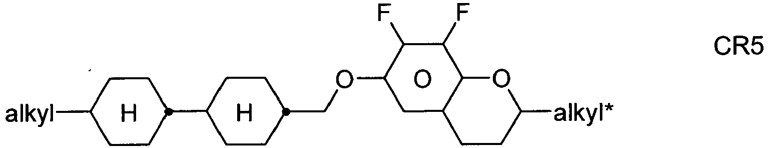 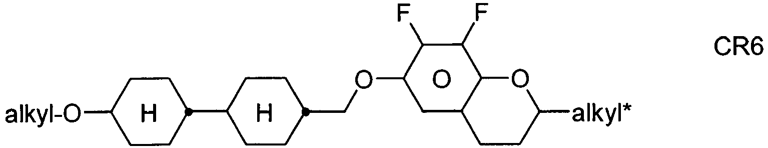 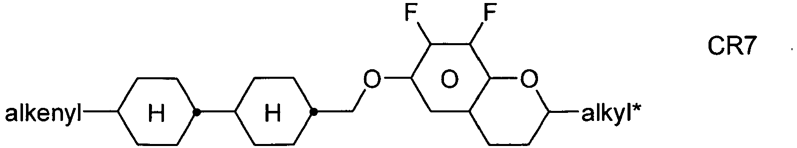 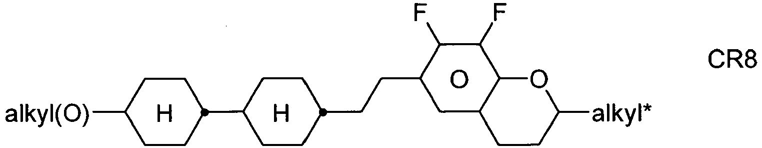 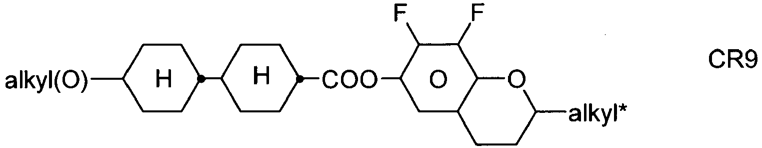 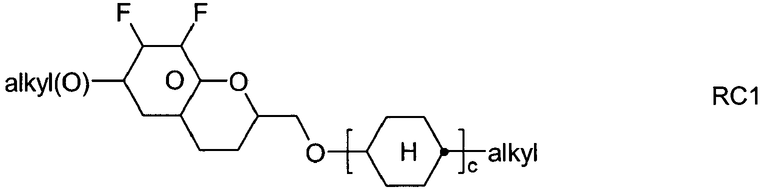 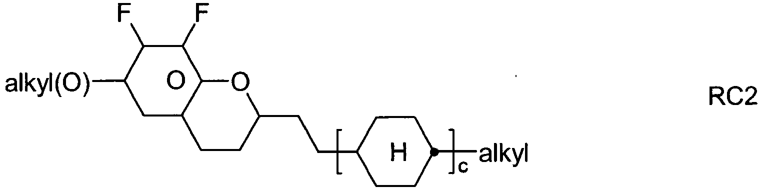 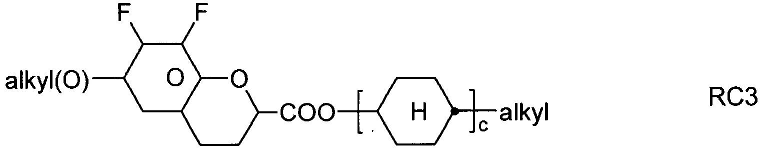 in which alkyl and alkyl* each, independently of one another, denote a straight-chain alkyl radical having 1-6 C atoms, (O) denotes an oxygen atom or a single bond, c is 1 or 2, and alkenyl and alkenyl* each, independently of one another, denote a straight-chain alkenyl radical having 2-6 C atoms. Alkenyl and alkenyl* preferably denote CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   Very particular preference is given to mixtures comprising one, two or three compounds of the formula BC-2.
o) LC medium which additionally comprises one or more fluorinated phenanthrenes and/or dibenzofurans of the following formulae: 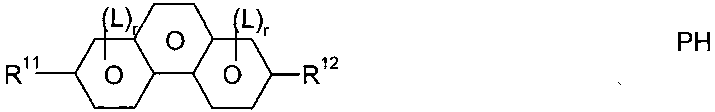 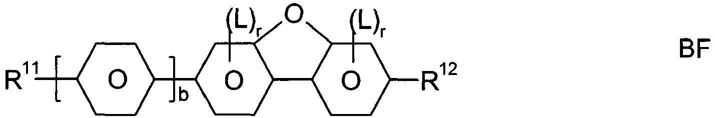 in which R¹¹ and R¹² each, independently of one another, have one of the meanings indicated above for R¹¹, b denotes 0 or 1, L denotes F, and r denotes 1, 2 or 3.
   Particularly preferred compounds of the formulae PH and BF are selected from the group consisting of the following sub-formulae: 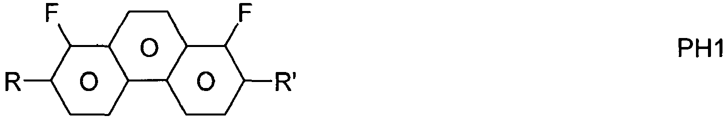 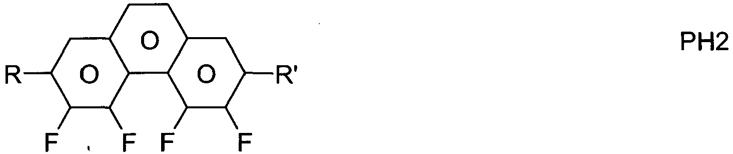 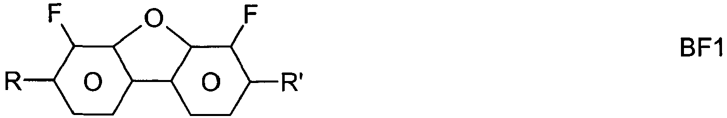 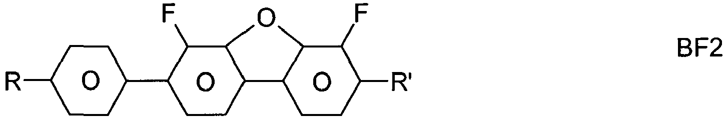 in which R and R' each, independently of one another, denote a straight-chain alkyl or alkoxy radical having 1-7 C atoms.
p) LC medium which additionally comprises one or more monocyclic compounds of the following formula 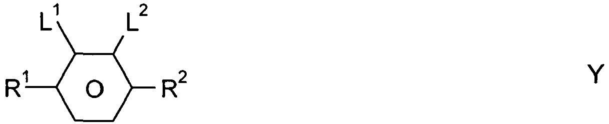 wherein
   - R¹ and R²: each, independently of one another, denote alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, preferably alkyl or alkoxy having 1 to 6 C atoms,
   - L¹ and L²: each, independently of one another, denote F, Cl, OCF₃, CF₃, CH₃, CH₂F, CHF₂.

   Preferably, both L¹ and L² denote F or one of L¹ and L² denotes F and the other denotes Cl,
   The compounds of the formula Y are preferably selected from the group consisting of the following sub-formulae: 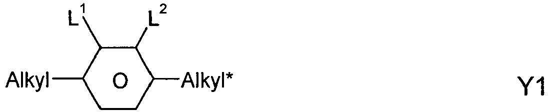 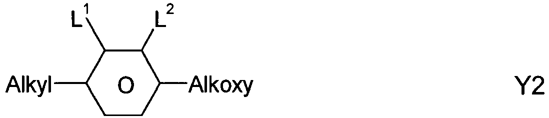 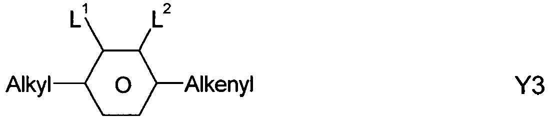 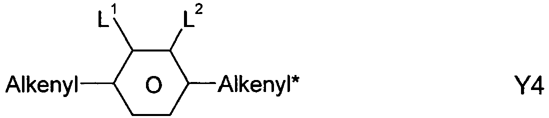 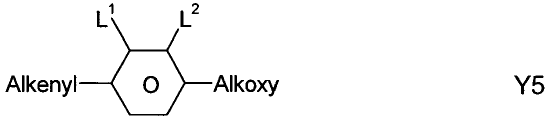 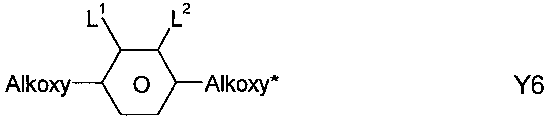 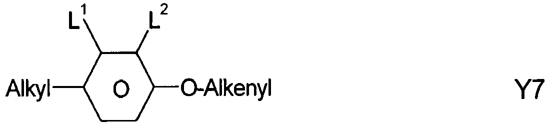 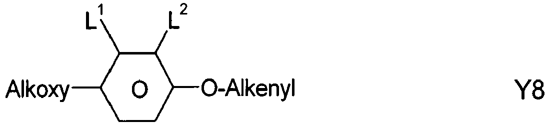 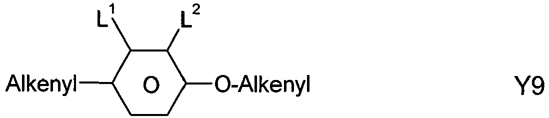 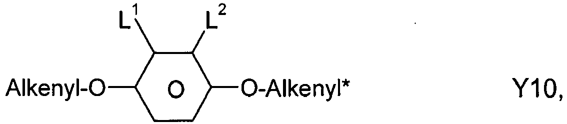 in which, Alkyl and Alkyl* each, independently of one another, denote a straight-chain alkyl radical having 1-6 C atoms, Alkoxy denotes a straight-chain alkoxy radical having 1-6 C atoms, Alkenyl and Alkenyl* each, independently of one another, denote a straight-chain alkenyl radical having 2-6 C atoms, and O denotes an oxygen atom or a single bond. Alkenyl and Alkenyl* preferably denote CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- or CH₃-CH=CH-(CH₂)₂-.
   Particularly preferred compounds of the formula Y are selected from the group consisting of the following sub-formulae: 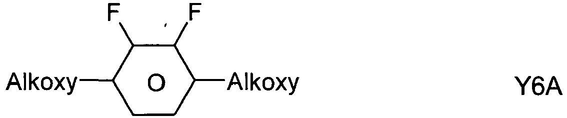 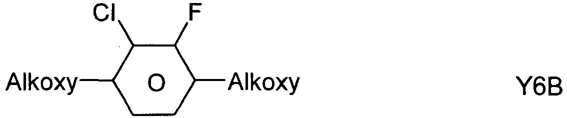 wherein Alkoxy preferably denotes straight-chain alkoxy with 3, 4, or 5 C atoms.
q) LC medium which, apart from the polymerisable compounds according to the invention, in particular of the formula I or sub-formulae thereof and the comonomers, comprises no compounds which contain a terminal vinyloxy group (-O-CH=CH₂).
r) LC medium which comprises 1 to 5, preferably 1, 2 or 3, polymerisable compounds, preferably selected from polymerisable compounds according to the invention, in particular of the formula I or sub-formulae thereof.
s) LC medium in which the proportion of polymerisable compounds, in particular of the formula I or sub-formulae thereof, in the mixture as a whole is 0.05 to 5%, preferably 0.1 to 1%.
t) LC medium which comprises 1 to 8, preferably 1 to 5, compounds of the formulae CY1, CY2, PY1 and/or PY2. The proportion of these compounds in the mixture as a whole is preferably 5 to 60%, particularly preferably 10 to 35%. The content of these individual compounds is preferably in each case 2 to 20%.
u) LC medium which comprises 1 to 8, preferably 1 to 5, compounds of the formulae CY9, CY10, PY9 and/or PY10. The proportion of these compounds in the mixture as a whole is preferably 5 to 60%, particularly preferably 10 to 35%. The content of these individual compounds is preferably in each case 2 to 20%.
v) LC medium which comprises 1 to 10, preferably 1 to 8, compounds of the formula ZK, in particular compounds of the formulae ZK1, ZK2 and/or ZK6. The proportion of these compounds in the mixture as a whole is preferably 3 to 25%, particularly preferably 5 to 45%. The content of these individual compounds is preferably in each case 2 to 20%.
w) LC medium in which the proportion of compounds of the formulae CY, PY and ZK in the mixture as a whole is greater than 70%, preferably greater than 80%.
x) LC medium in which the LC host mixture contains one or more compounds containing an alkenyl group, preferably selected from the group consisting of formula CY, PY and LY, wherein one or both of R¹ and R² denote straight-chain alkenyl having 2-6 C atoms, formula ZK and DK, wherein one or both of R³ and R⁴ or one or both of R⁵ and R⁶ denote straight-chain alkenyl having 2-6 C atoms, and formula B2 and B3, very preferably selected from formulae CY15, CY16, CY24, CY32, PY15, PY16, ZK3, ZK4, DK3, DK6, B2 and B3, most preferably selected from formulae ZK3, ZK4, B2 and B3. The concentration of these compounds in the LC host mixture is preferably from 2 to 70%, very preferably from 3 to 55%.
y) LC medium which contains one or more, preferably 1 to 5, compounds selected of formula PY1-PY8, very preferably of formula PY2. The proportion of these compounds in the mixture as a whole is preferably 1 to 30%, particularly preferably 2 to 20%. The content of these individual compounds is preferably in each case 1 to 20%.
z) LC medium which contains one or more, preferably 1, 2 or 3, compounds of formula T2. The content of these compounds in the mixture as a whole is preferably 1 to 20%.

In a second preferred embodiment the LC medium contains an LC host mixture based on compounds with positive dielectric anisotropy. Such LC media are especially suitable for use in PS-OCB-, PS-TN-, PS-Posi-VA-, PS-IPS- or PS-FFS-displays.

Particularly preferred is an LC medium of this second preferred embodiment, which contains one or more compounds selected from the group consisting of compounds of formula AA and BB 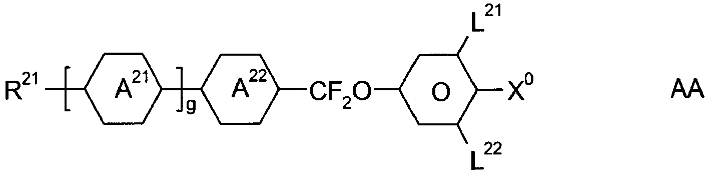 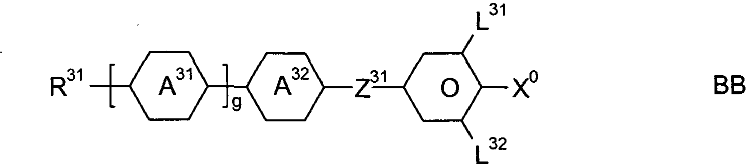 and optionally contains, in addition to the compounds of formula AA and/or BB, one or more compounds of formula CC 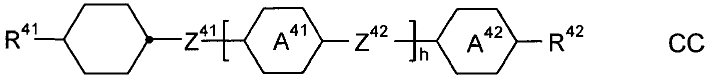 in which the individual radicals have the following meanings: 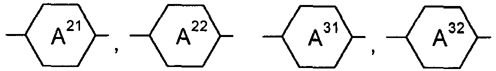 each, independently of one another, and on each occurrence, identically or differently 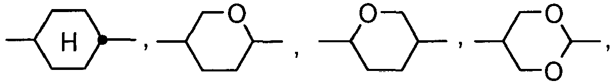 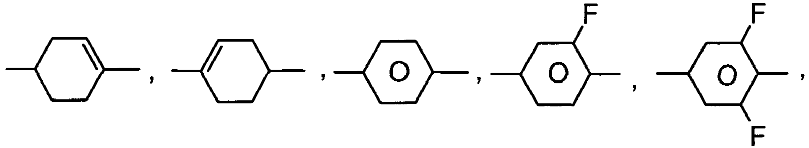 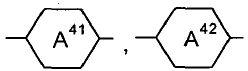 each, independently of one another, and on each occurrence, identically or differently 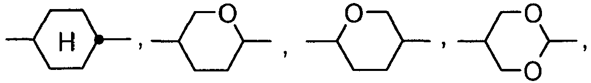 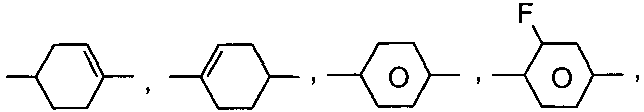
- R²¹, R³¹, R⁴¹, R⁴²: each, independently of one another, alkyl, alkoxy, oxaalkyl or alkoxyalkyl having 1 to 9 C atoms or alkenyl or alkenyloxy having 2 to 9 C atoms, all of which are optionally fluorinated,
- X⁰: F, Cl, halogenated alkyl or alkoxy having 1 to 6 C atoms or halogenated alkenyl or alkenyloxy having 2 to 6 C atoms,
- Z³¹: -CH₂CH₂-, -CF₂CF₂-, -COO-, *trans-*CH=CH-, *trans-*CF=CF-, -CH₂O- or a single bond, preferably -CH₂CH₂-,-COO-, *trans*-CH=CH- or a single bond, particularly preferably -COO-, *trans-*CH=CH- or a single bond,
- Z⁴¹, Z⁴²: -CH₂CH₂-, -COO-, *trans-*CH=CH-, *trans*-CF=CF-, -CH₂O-, -CF₂O-, -C≡C- or a single bond, preferably a single bond,
- L²¹, L²², L³¹, L³²: H or F,
- g: 0, 1, 2 or 3,
- h: 0, 1, 2 or 3.
X⁰ is preferably F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ or CH=CF₂, very preferably F or OCF₃

The compounds of formula AA are preferably selected from the group consisting of the following formulae: 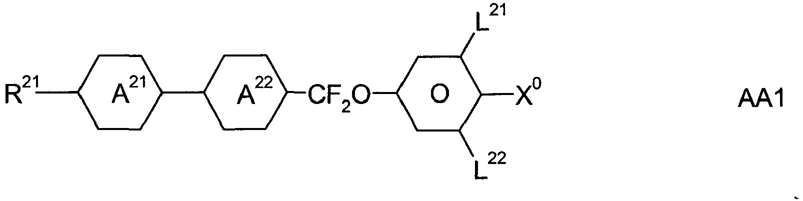 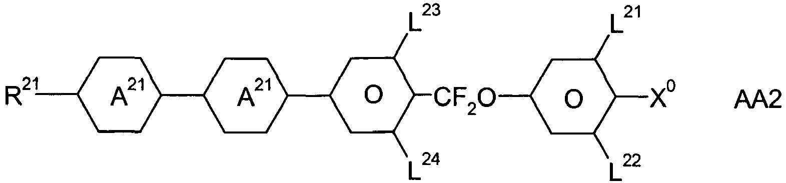 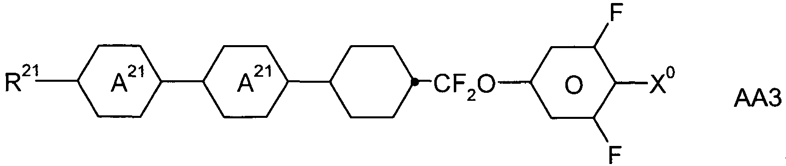 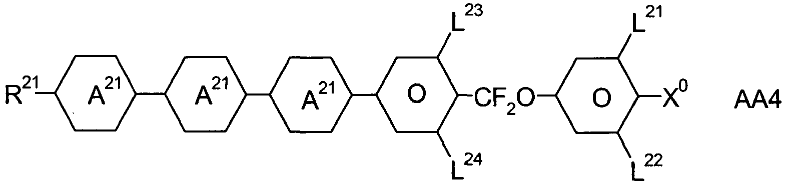 in which A²¹, R²¹, X⁰, L²¹ and L²² have the meanings given in formula AA, L²³ and L²⁴ each, independently of one another, are H or F, and X⁰ is preferably F. Particularly preferred are compounds of formulae AA1 and AA2.

Particularly preferred compounds of formula AA1 are selected from the group consisting of the following subformulae: 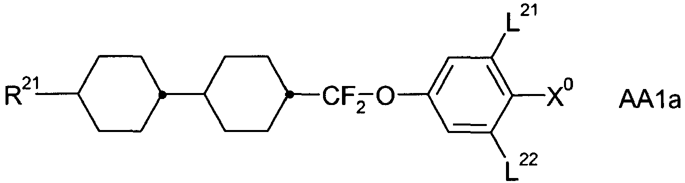 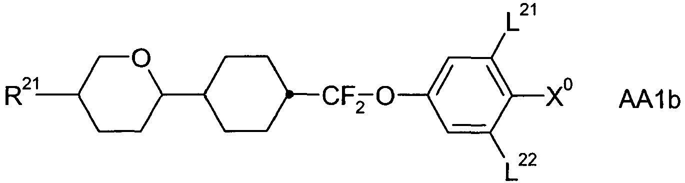 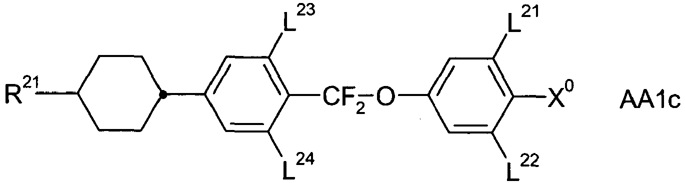 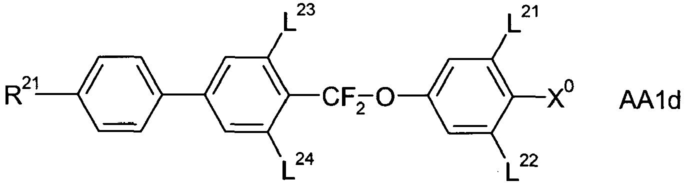 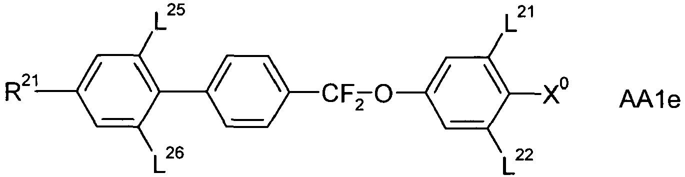 in which R²¹, X⁰, L²¹ and L²² have the meaning given in formula AA1, L²³, L²⁴, L²⁵ and L²⁶ are each, independently of one another, H or F, and X⁰ is preferably F.

Very particularly preferred compounds of formula AA1 are selected from the group consisting of the following subformulae:

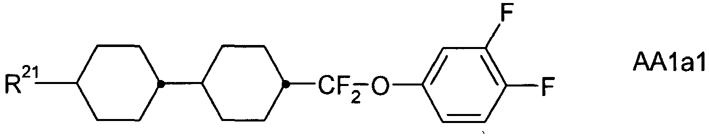

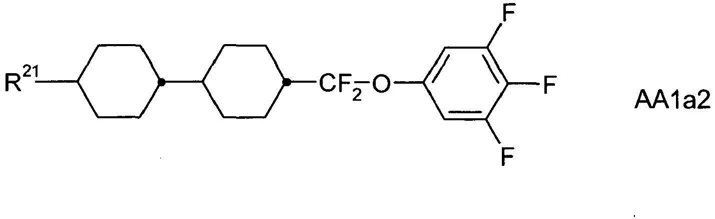

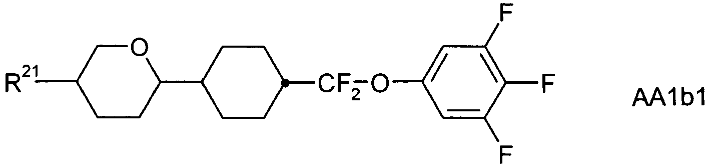

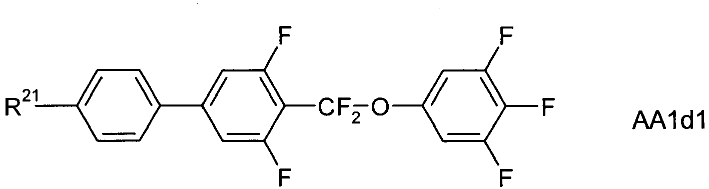

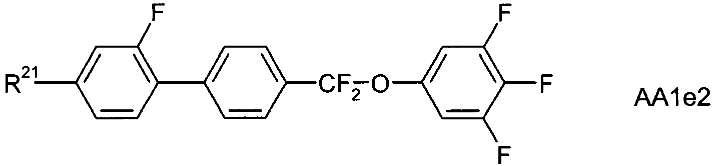

In which R²¹ is as defined in formula AA1.

Very preferred compounds of formula AA2 are selected from the group consisting of the following subformulae: 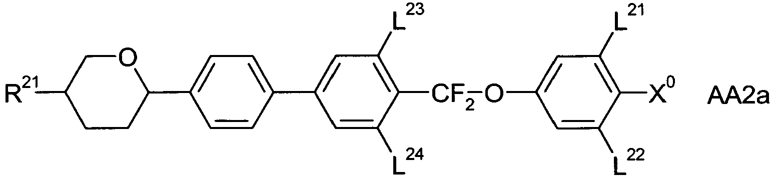 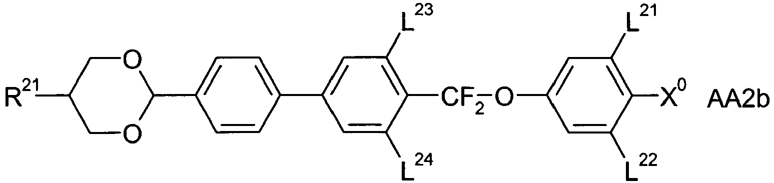 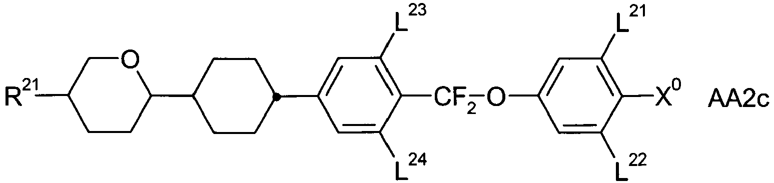 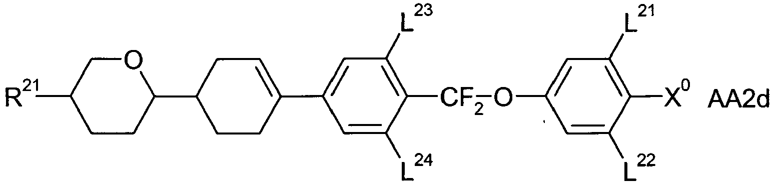 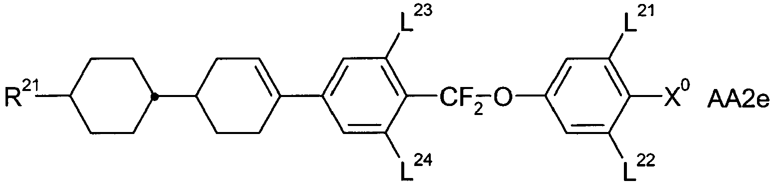 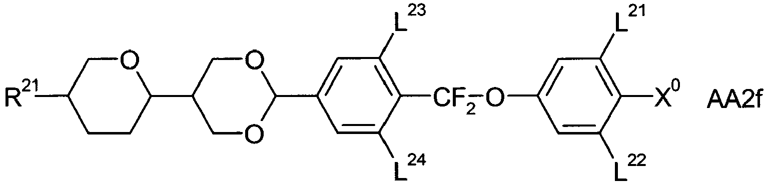 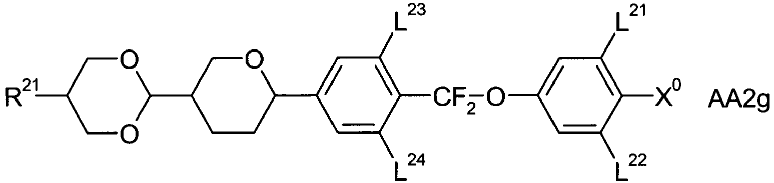 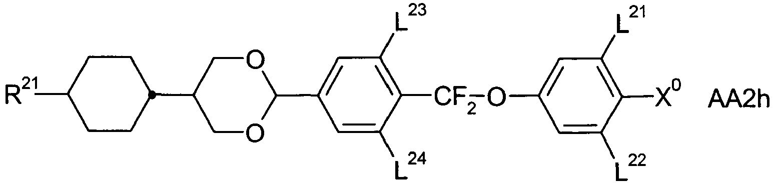 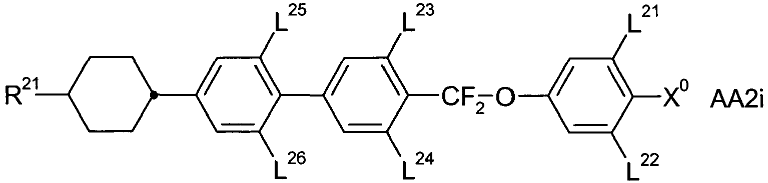 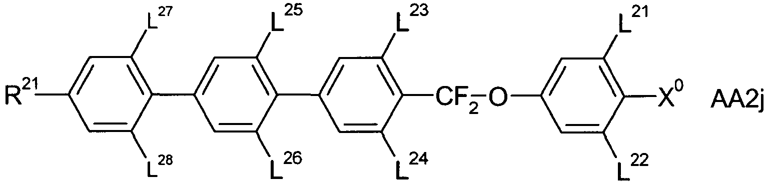 in which R²¹, X⁰, L²¹ and L²² have the meaning given in formula AA2, L²³, L²⁴, L²⁵ and L²⁶ each, independently of one another, are H or F, and X⁰ is preferably F.

Very particularly preferred compounds of formula AA2 are selected from the group consisting of the following subformulae: 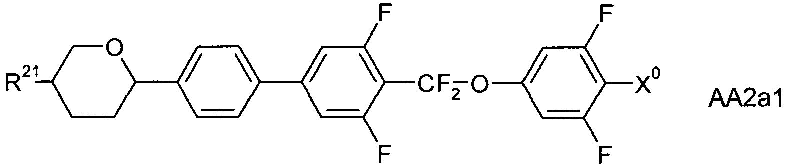 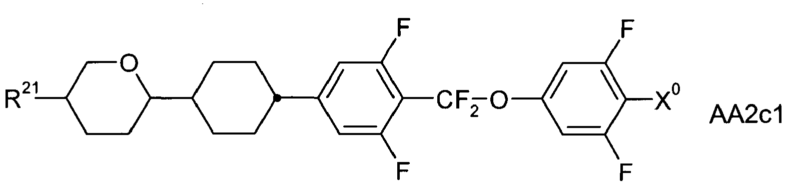 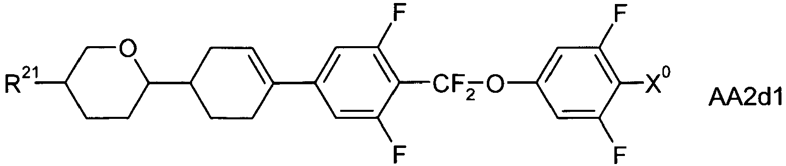 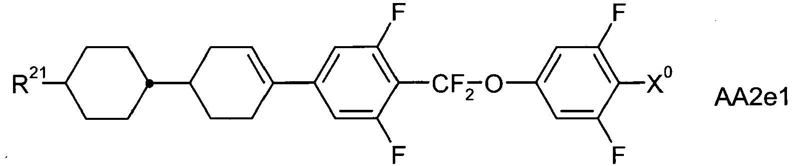 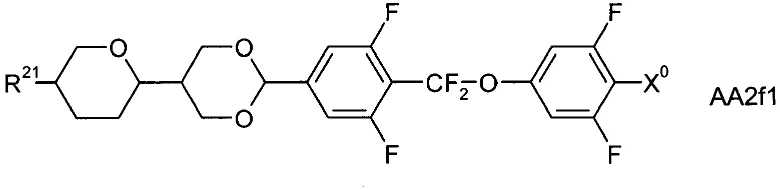 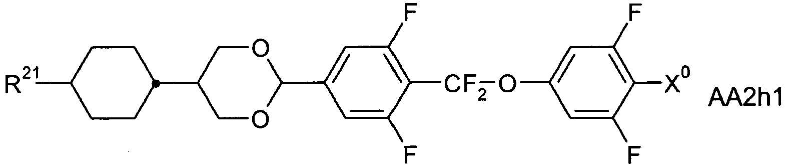 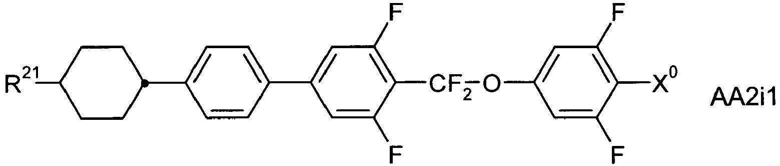 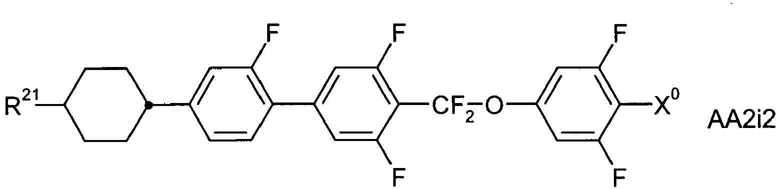 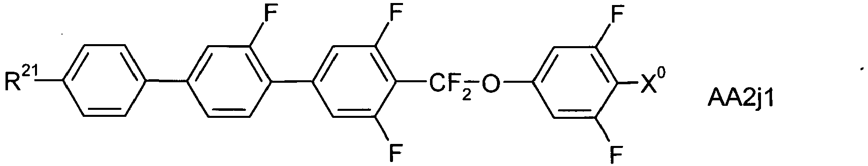 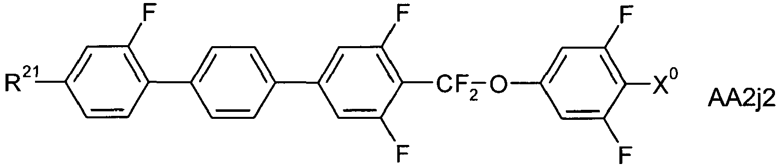 in which R²¹ and X⁰ are as defined in formula AA2.

Particularly preferred compounds of formula AA3 are selected from the group consisting of the following subformulae: 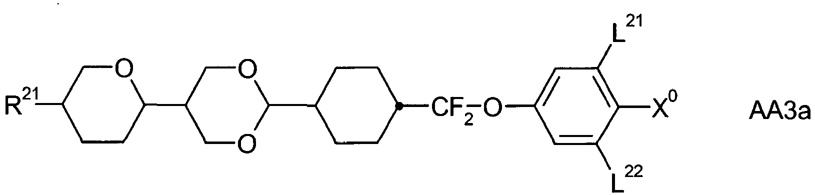 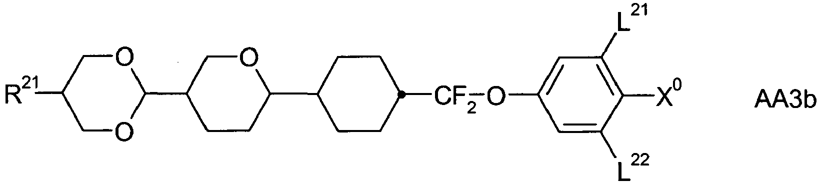 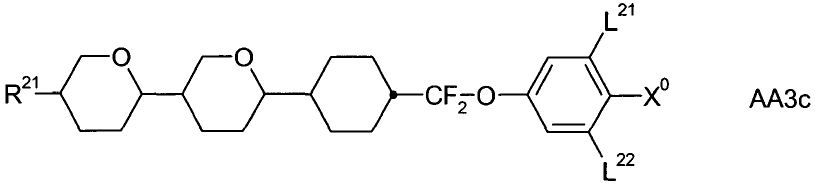 in which R²¹, X⁰, L²¹ and L²² have the meaning given in formula AA3, and X⁰ is preferably F.

Particularly preferred compounds of formula AA4 are selected from the group consisting of the following subformulae: 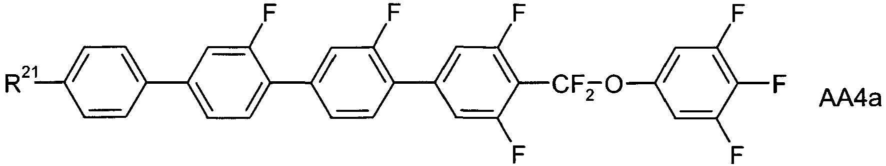 in which R²¹ is as defined in formula AA4.

The compounds of formula BB are preferably selected from the group consisting of the following formulae: 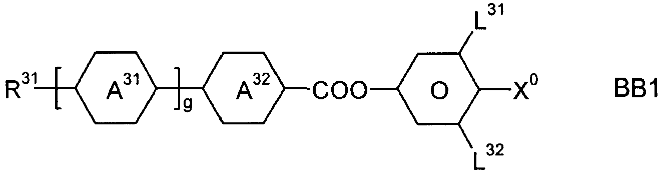 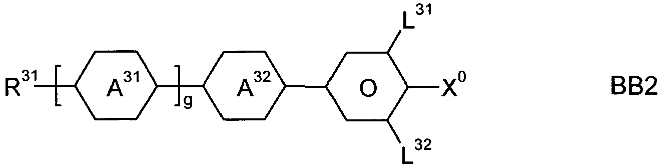 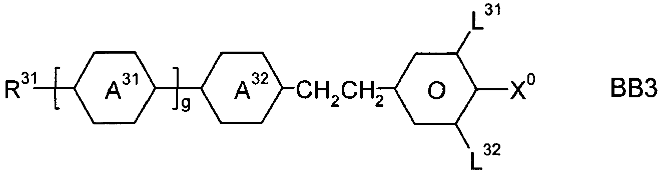 in which g, A³¹, A³², R³¹, X⁰, L³¹ and L³² have the meanings given in formula BB, and X⁰ is preferably F. Particularly preferred are compounds of formulae BB1 and BB2.

Particularly preferred compounds of formula BB1 are selected from the group consisting of the following subformulae: 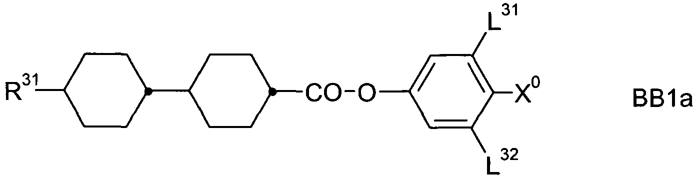 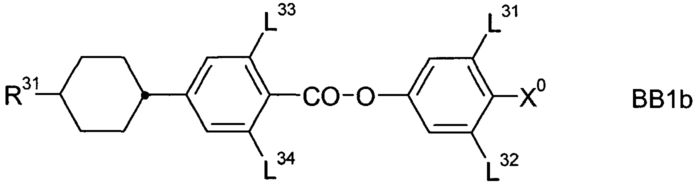 in which R³¹, X⁰, L³¹ and L³² have the meaning given in formula BB1, and X⁰ is preferably F.

Very particularly preferred compounds of formula BB1a are selected from the group consisting of the following subformulae: 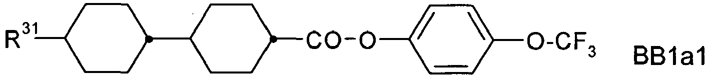 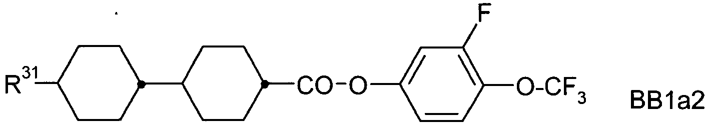 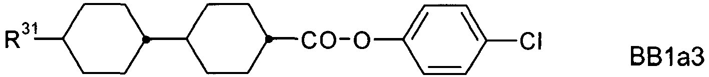 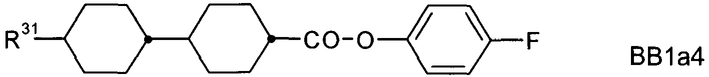 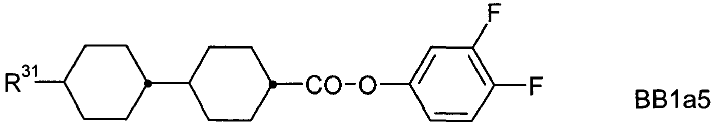 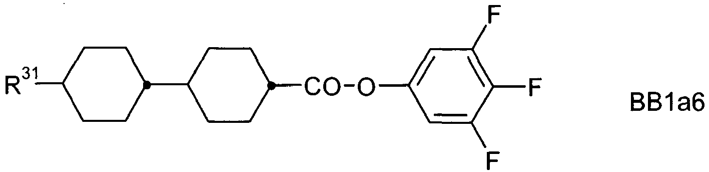 in which R³¹ is as defined in formula BB1.

Very particularly preferred compounds of formula BB1b are selected from the group consisting of the following subformulae: 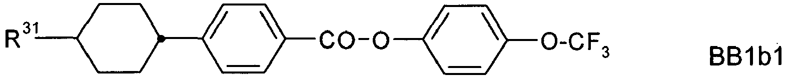 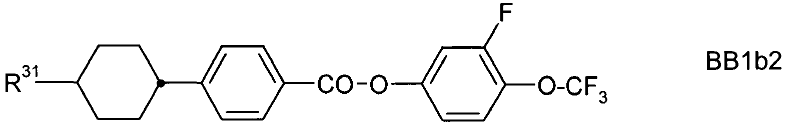 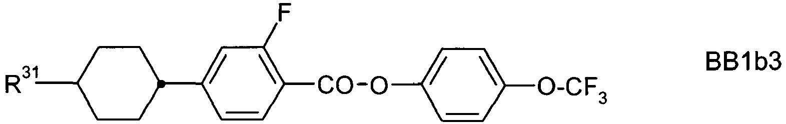 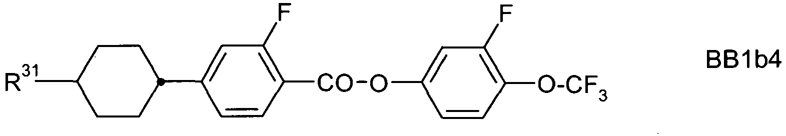 in which R³¹ is as defined in formula BB1.

Particularly preferred compounds of formula BB2 are selected from the group consisting of the following subformulae: 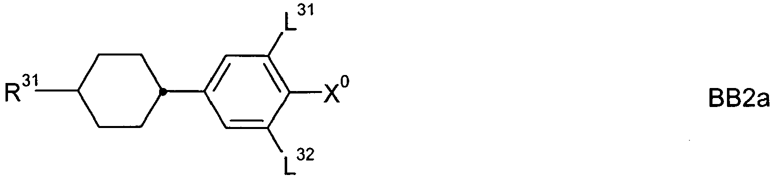 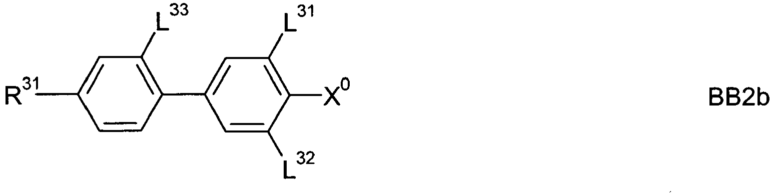 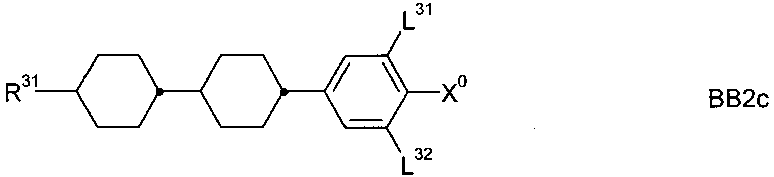 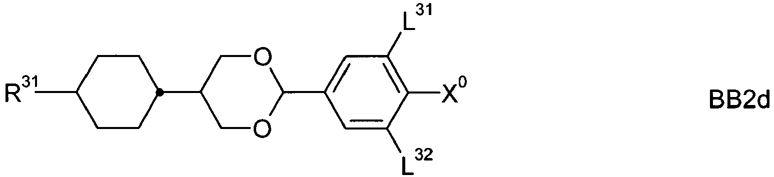 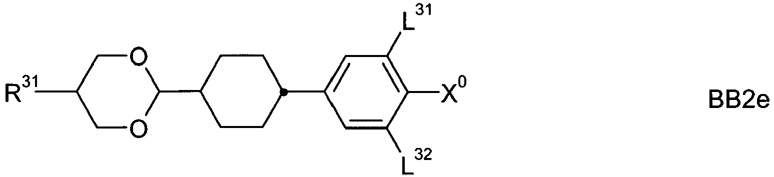 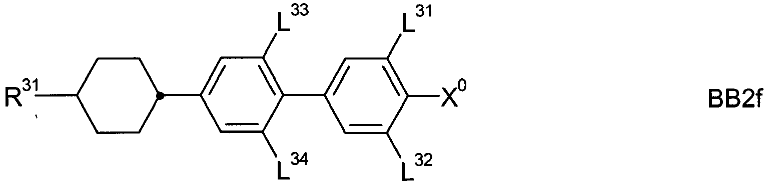 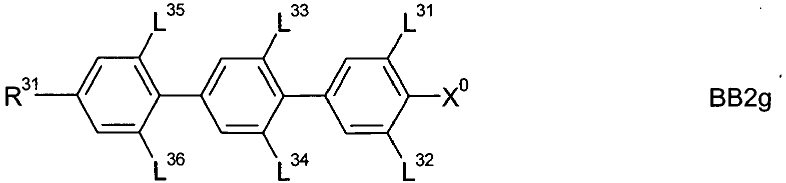 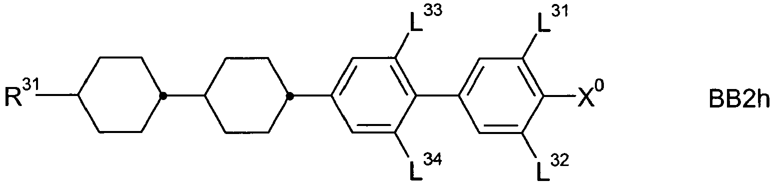 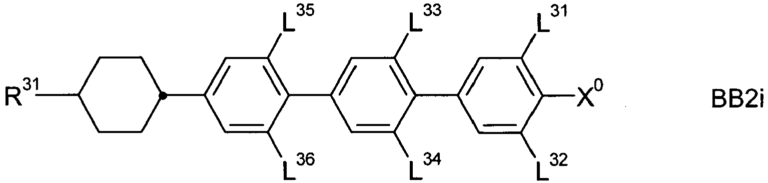 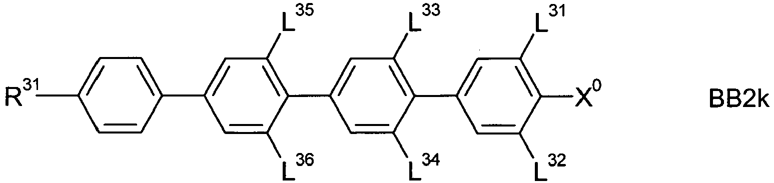 in which R³¹, X⁰, L³¹ and L³² have the meaning given in formula BB2, L³³, L³⁴, L³⁵ and L³⁶ are each, independently of one another, H or F, and X⁰ is preferably F.

Very particularly preferred compounds of formula BB2 are selected from the group consisting of the following subformulae: 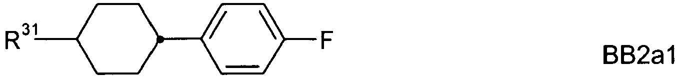 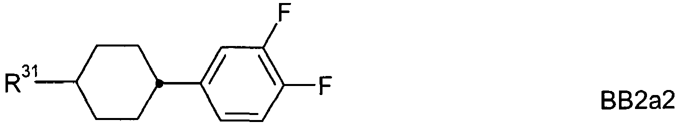 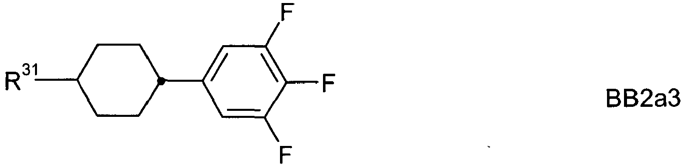 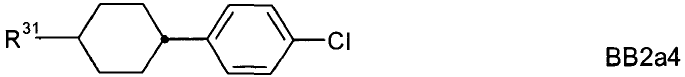 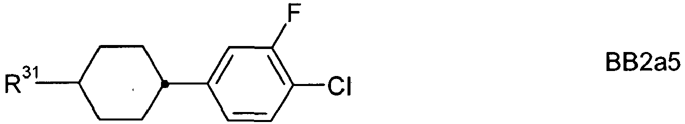 in which R³¹ is as defined in formula BB2.

Very particularly preferred compounds of formula BB2b are selected from the group consisting of the following subformulae 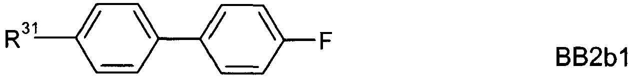 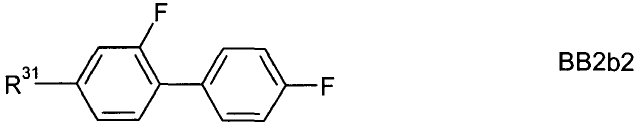 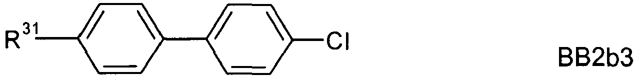 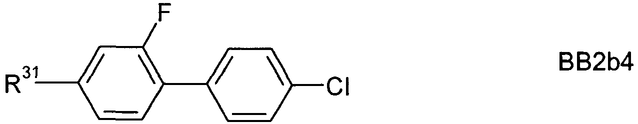 in which R³¹ is as defined in formula BB2.

Very particularly preferred compounds of formula BB2c are selected from the group consisting of the following subformulae: 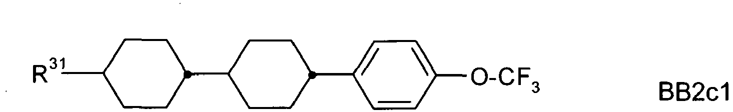 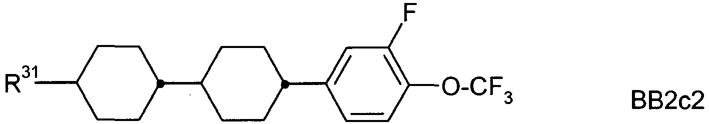 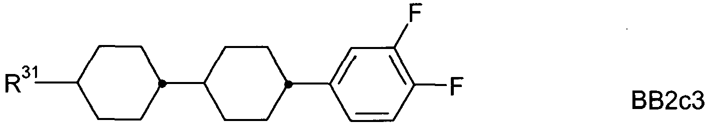 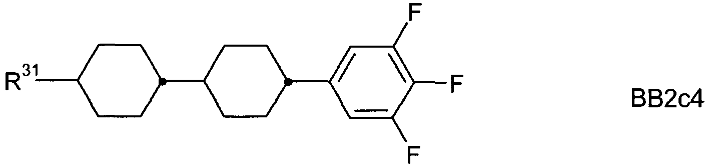 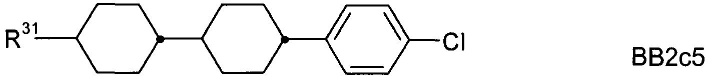 in which R³¹ is as defined in formula BB2.

Very particularly preferred compounds of formula BB2d and BB2e are selected from the group consisting of the following subformulae: 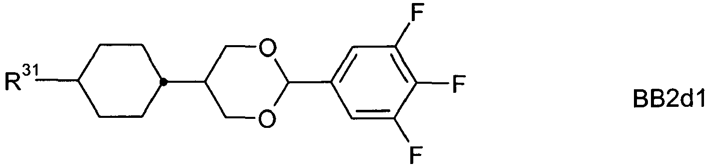 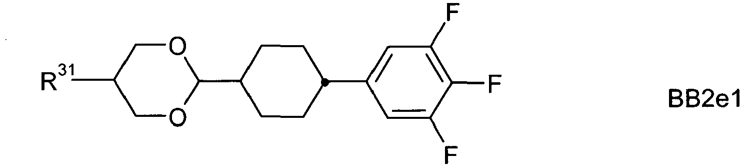 in which R³¹ is as defined in formula BB2.

Very particularly preferred compounds of formula BB2f are selected from the group consisting of the following subformulae: 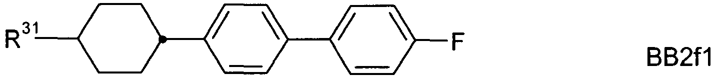 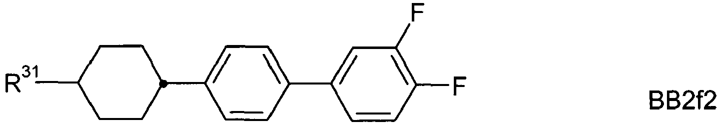 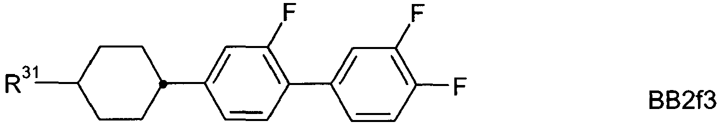 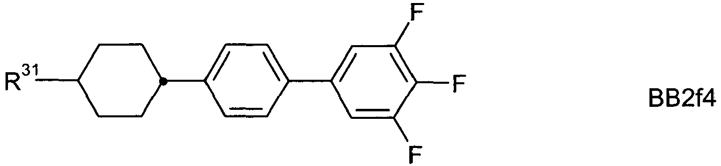 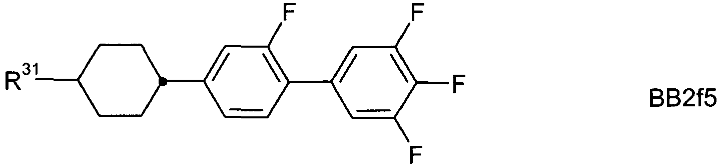 in which R³¹ is as defined in formula BB2.

Very particularly preferred compounds of formula BB2g are selected from the group consisting of the following subformulae: 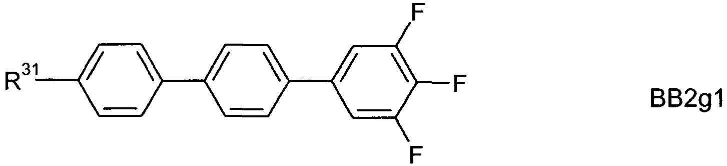 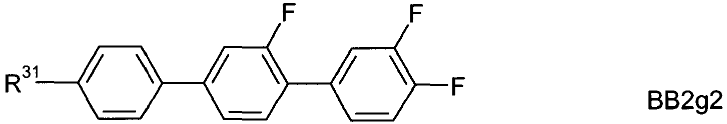 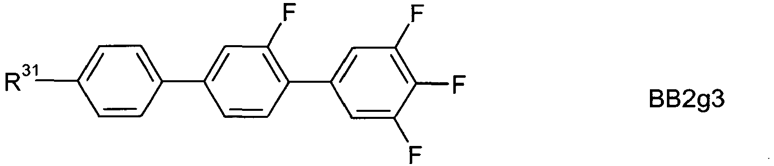 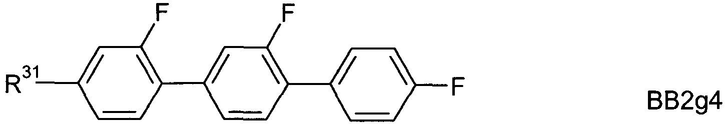 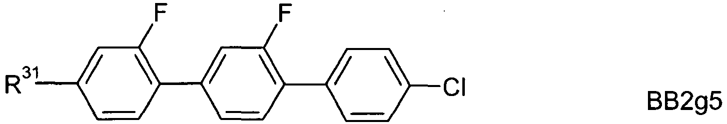 in which R³¹ is as defined in formula BB2.

Very particularly preferred compounds of formula BB2h are selected from the group consisting of the following subformulae: 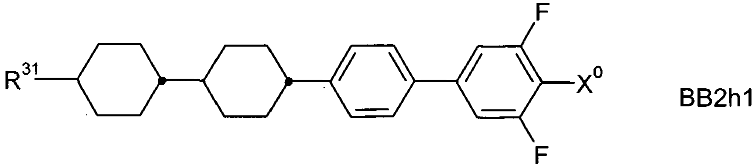 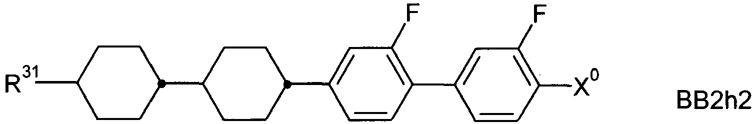 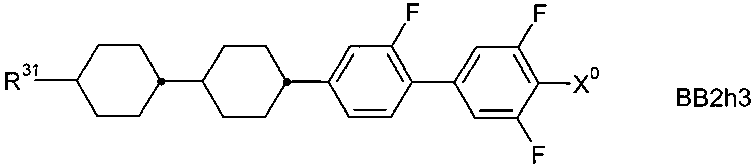 in which R³¹ and X⁰ are as defined in formula BB2.

Very particularly preferred compounds of formula BB2i are selected from the group consisting of the following subformulae: 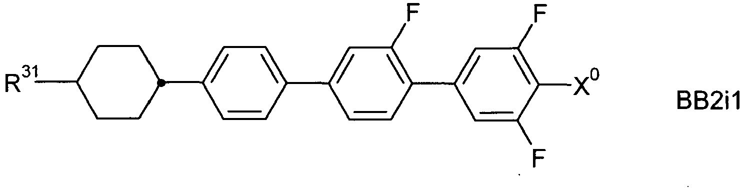 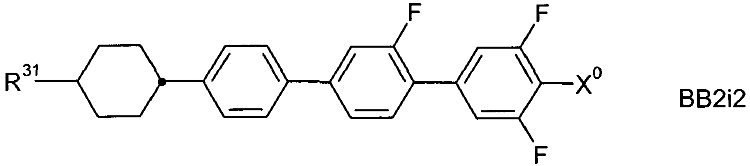 in which R³¹ and X⁰ are as defined in formula BB2.

Very particularly preferred compounds of formula BB2k are selected from the group consisting of the following subformulae: 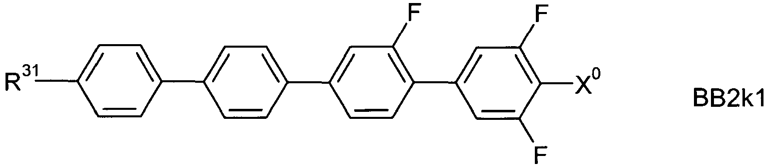 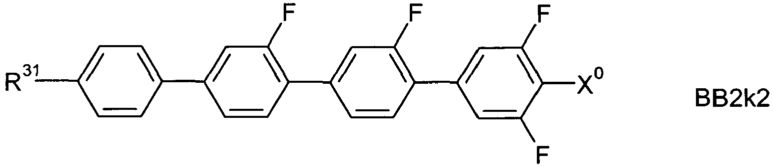 in which R³¹ and X⁰ are as defined in formula BB2.

Alternatively to, or in addition to, the compounds of formula BB1 and/or BB2 the LC media may also comprise one or more compounds of formula BB3 as defined above.

Particularly preferred compounds of formula BB3 are selected from the group consisting of the following subformulae: 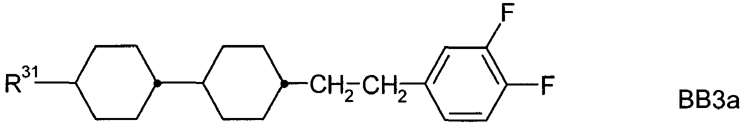 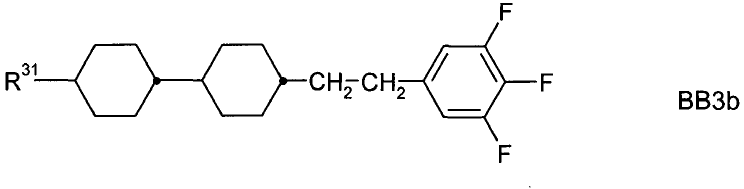 in which R³¹ is as defined in formula BB3.

Preferably the LC media according to this second preferred embodiment comprise, in addition to the compounds of formula AA and/or BB, one or more dielectrically neutral compounds having a dielectric anisotropy in the range from -1.5 to +3, preferably selected from the group of compounds of formula CC as defined above.

Particularly preferred compounds of formula CC are selected from the group consisting of the following subformulae:

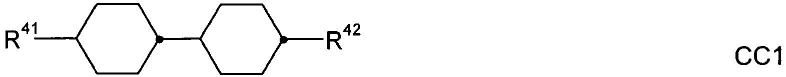

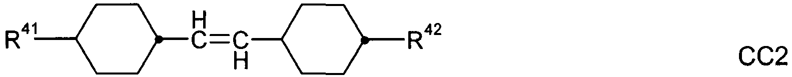

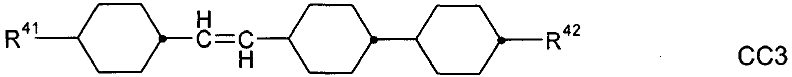

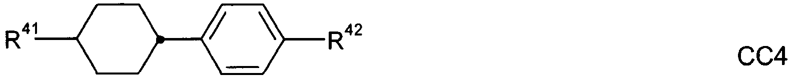

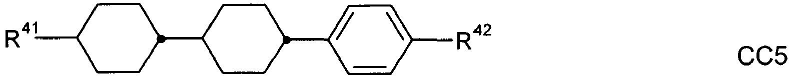

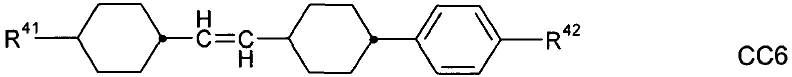

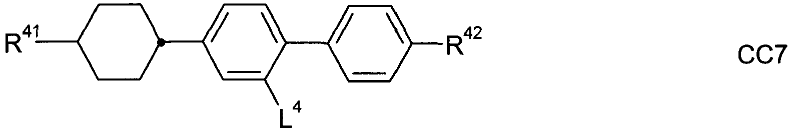

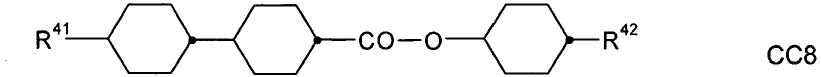

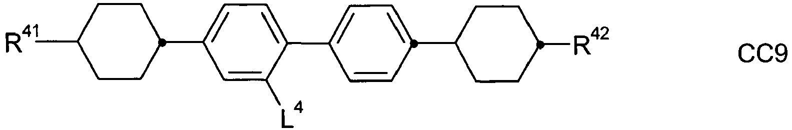

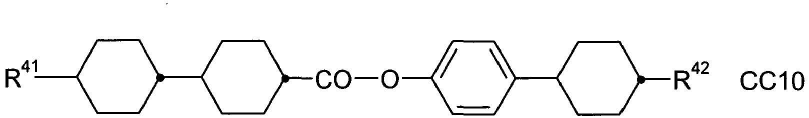

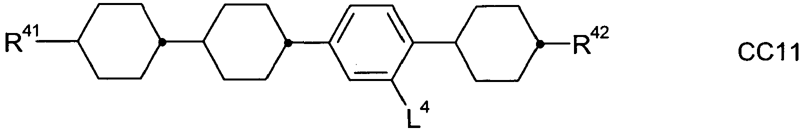

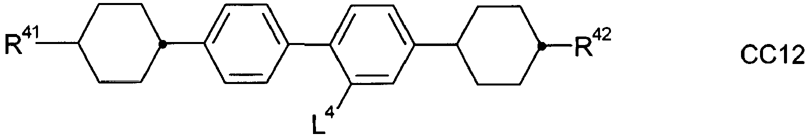

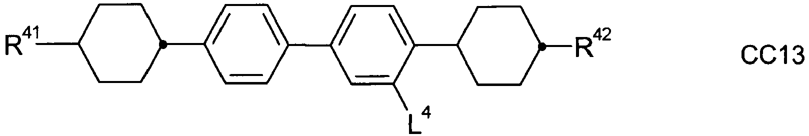

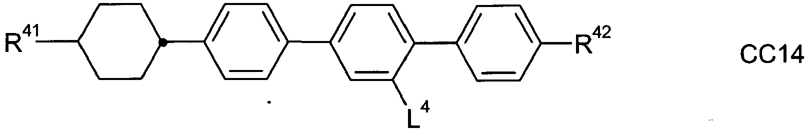

In which R⁴¹ and R⁴² have the meanings given in formula CC, and preferably denote each, independently of one another, alkyl, alkoxy, fluorinated alkyl or fluorinated alkoxy with 1 to 7 C atoms, or alkenyl, alkenyloxy, alkoxyalkyl or fluorinated alkenyl with 2 to 7 C atoms, and L⁴ is H or F.

Preferably the LC media according to this second preferred embodiment comprise, in addition or alternatively to the dielectrically neutral compounds of formula CC, one or more dielectrically neutral compounds having a dielectric anisotropy in the range from -1.5 to +3, selected from the group of compounds of formula DD.

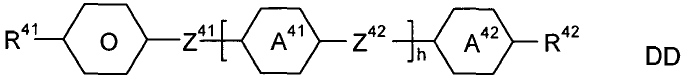

In which A⁴¹, A⁴², Z⁴¹, Z⁴², R⁴¹, R⁴² and h have the meanings given in formula CC.

Particularly preferred compounds of formula DD are selected from the group consisting of the following subformulae: 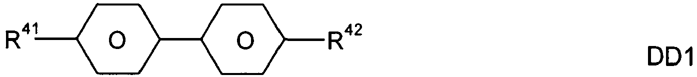 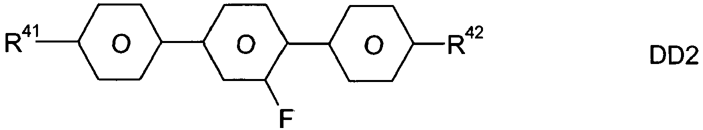 in which R⁴¹ and R⁴² have the meanings given in formula DD and R⁴¹ preferably denotes alkyl bedeutet, and in formula DD1 R⁴² preferably denotes alkenyl, particularly preferably -(CH₂)₂-CH=CH-CH₃, and in formula DD2 R⁴² preferably denotes alkyl, -(CH₂)₂-CH=CH₂ or -(CH₂)₂-CH=CH-CH₃.

The compounds of formula AA and BB are preferably used in the LC medium according to the invention in a concentration from 2% to 60%, more preferably from 3% to 35%, and very particularly preferably from 4% to 30% in the mixture as a whole.

The compounds of formula CC and DD are preferably used in the LC medium according to the invention in a concentration from 2% to 70%, more preferably from 5% to 65%, even more preferably from 10% to 60%, and very particularly preferably from10%, preferably 15%, to 55% in the mixture as a whole.

The combination of compounds of the preferred embodiments mentioned above with the polymerised compounds described above causes low threshold voltages, low rotational viscosities and very good low-temperature stabilities in the LC media according to the invention at the same time as constantly high clearing points and high HR values, and allows the rapid establishment of a particularly low pretilt angle in PSA displays. In particular, the LC media exhibit significantly shortened response times, in particular also the grey-shade response times, in PSA displays compared with the media from the prior art.

The LC media and LC host mixtures of the present invention preferably have a nematic phase range of at least 80 K, particularly preferably at least 100 K, and a rotational viscosity ≤ 250 mPa·s, preferably ≤ 200 mPa·s, at 20°C.

In the VA-type displays according to the invention, the molecules in the layer of the LC medium in the switched-off state are aligned perpendicular to the electrode surfaces (homeotropically) or have a a tilted homeotropic alignment. On application of an electrical voltage to the electrodes, a realignment of the LC molecules takes place with the longitudinal molecular axes parallel to the electrode surfaces.

LC media according to the invention based on compounds with negative dielectric anisotropy according to the first preferred embodiment, in particular for use in displays of the PS-VA and PS-UB-FFS type, have a negative dielectric anisotropy Δε, preferably from -0.5 to -10, in particular from -2.5 to -7.5, at 20°C and 1 kHz.

The birefringence Δn in LC media according to the invention for use in displays of the PS-VA and PS-UB-FFS type is preferably below 0.16, particularly preferably from 0.06 to 0.14, very particularly preferably from 0.07 to 0,12.

In the OCB-type displays according to the invention, the molecules in the layer of the LC medium have a "bend" alignment. On application of an electrical voltage, a realignment of the LC molecules takes place with the longitudinal molecular axes perpendicular to the electrode surfaces.

LC media according to the invention for use in displays of the PS-OCB, PS-TN, PS-IPS, PS-posi-VA and PS-FFS type are preferably those based on compounds with positive dielectric anisotropy according to the second preferred embodiment, and preferably have a positive dielectric anisotropy Δε from +4 to +17 at 20°C and 1 kHz.

The birefringence Δn in LC media according to the invention for use in displays of the PS-OCB type is preferably from 0.14 to 0.22, particularly preferably from 0.16 to 0.22.

The birefringence Δn in LC media according to the invention for use in displays of the PS-TN-, PS-posi-VA-, PS-IPS- oder PS-FFS-type is preferably from 0.07 to 0.15, particularly preferably from 0.08 to 0.13.

LC media according to the invention, based on compounds with positive dielectric anisotropy according to the second preferred embodiment, for use in displays of the PS-TN-, PS-posi-VA-, PS-IPS- oder PS-FFS-type, preferably have a positive dielectric anisotropy Δε from +2 to +30, particularly preferably from +3 to +20, at 20°C and 1 kHz.

The LC media according to the invention may also comprise further additives which are known to the person skilled in the art and are described in the literature, such as, for example, polymerisation initiators, inhibitors, stabilisers, surface-active substances or chiral dopants. These may be polymerisable or non-polymerisable. Polymerisable additives are accordingly ascribed to the polymerisable component or component A). Non-polymerisable additives are accordingly ascribed to the non-polymerisable component or component B).

In a preferred embodiment the LC media contain one or more chiral dopants, preferably in a concentration from 0.01 to 1%, very preferably from 0.05 to 0.5%. The chiral dopants are preferably selected from the group consisting of compounds from Table B below, very preferably from the group consisting of R- or S-1011, R- or S-2011, R- or S-3011, R- or S-4011, and R- or S-5011.

In another preferred embodiment the LC media contain a racemate of one or more chiral dopants, which are preferably selected from the chiral dopants mentioned in the previous paragraph.

Furthermore, it is possible to add to the LC media, for example, 0 to 15% by weight of pleochroic dyes, furthermore nanoparticles, conductive salts, preferably ethyldimethyldodecylammonium 4-hexoxybenzoate, tetrabutylammonium tetraphenylborate or complex salts of crown ethers (cf., for example, Haller et al., Mol. Cryst. Liq. Cryst. 24, 249-258 (1973)), for improving the conductivity, or substances for modifying the dielectric anisotropy, the viscosity and/or the alignment of the nematic phases. Substances of this type are described, for example, in DE-A 22 09 127, 22 40 864, 23 21 632, 23 38 281, 24 50 088, 26 37 430 and 28 53 728.

The individual components of the preferred embodiments a)-z) of the LC media according to the invention are either known or methods for the preparation thereof can readily be derived from the prior art by the person skilled in the relevant art, since they are based on standard methods described in the literature. Corresponding compounds of the formula CY are described, for example, in EP-A-0 364 538. Corresponding compounds of the formula ZK are described, for example, in DE-A-26 36 684 and DE-A-33 21 373.

The LC media which can be used in accordance with the invention are prepared in a manner conventional per se, for example by mixing one or more of the above-mentioned compounds with one or more polymerisable compounds as defined above, and optionally with further liquid-crystalline compounds and/or additives. In general, the desired amount of the components used in lesser amount is dissolved in the components making up the principal constituent, advantageously at elevated temperature. It is also possible to mix solutions of the components in an organic solvent, for example in acetone, chloroform or methanol, and to remove the solvent again, for example by distillation, after thorough mixing. The invention furthermore relates to the process for the preparation of the LC media according to the invention.

It goes without saying to the person skilled in the art that the LC media according to the invention may also comprise compounds in which, for example, H, N, O, Cl, F have been replaced by the corresponding isotopes like deuterium etc.

The following examples explain the present invention without restricting it. However, they show the person skilled in the art preferred mixture concepts with compounds preferably to be employed and the respective concentrations thereof and combinations thereof with one another. In addition, the examples illustrate which properties and property combinations are accessible.

The following abbreviations are used:
(n, m, z: in each case, independently of one another, 1, 2, 3, 4, 5 or 6)

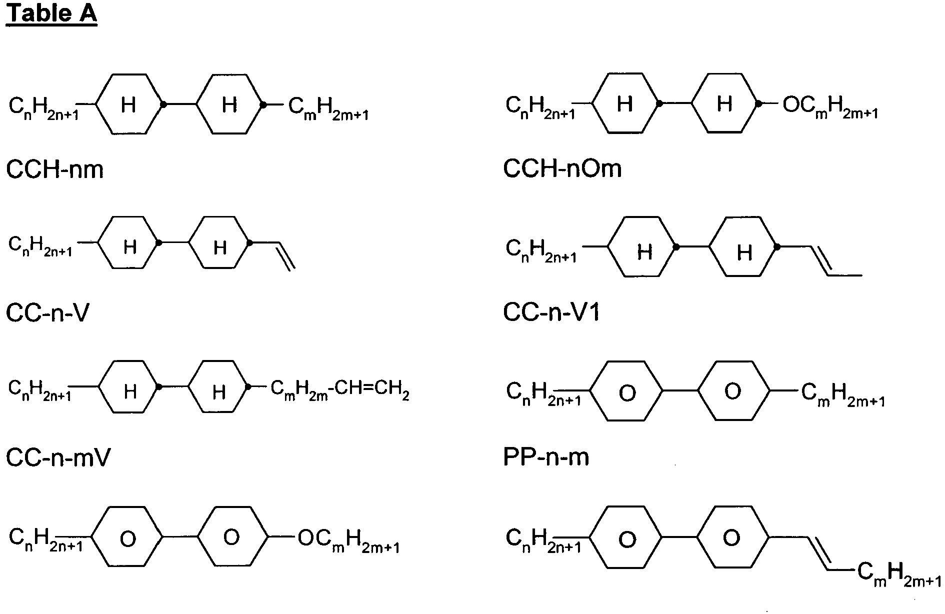

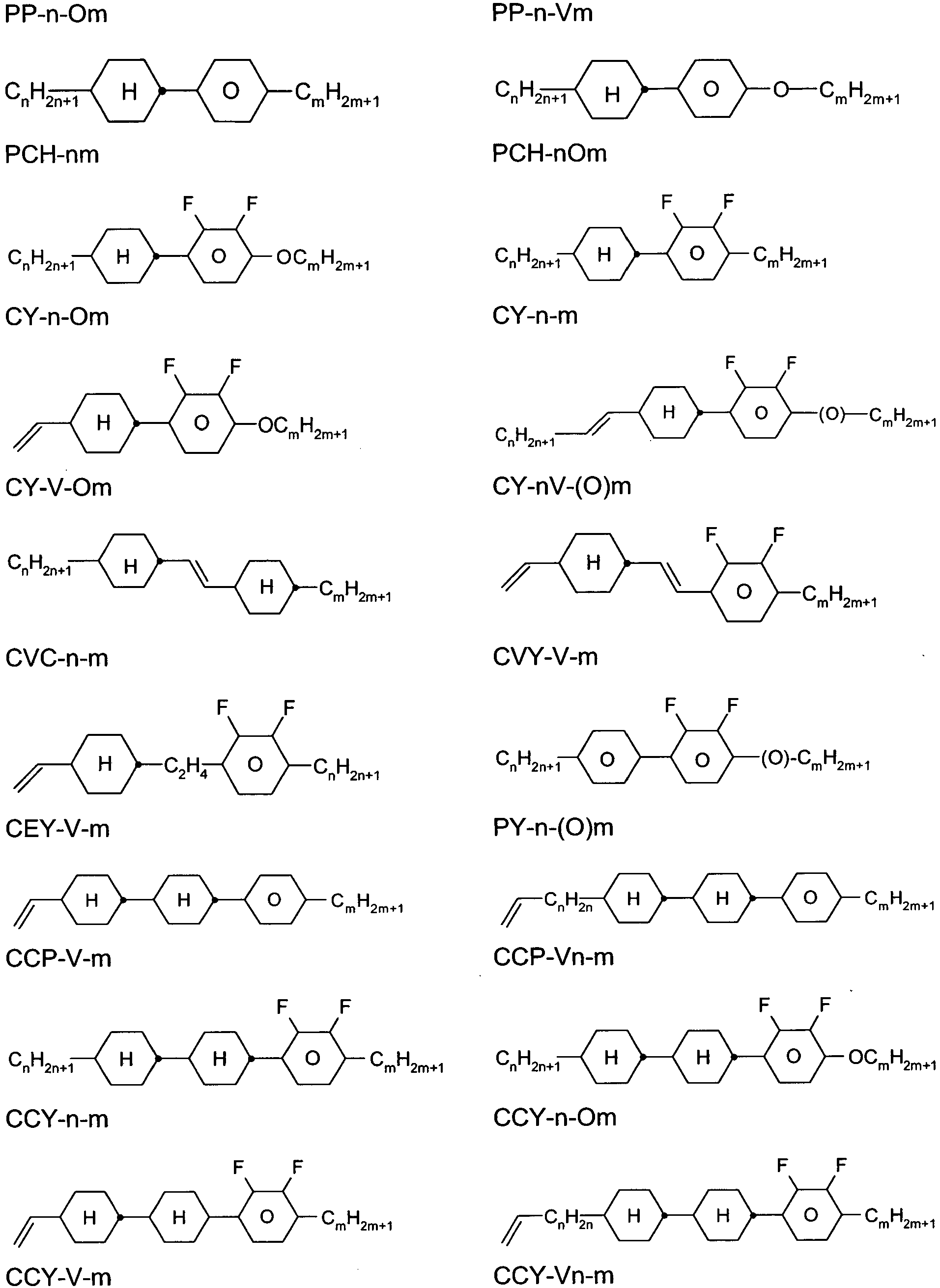

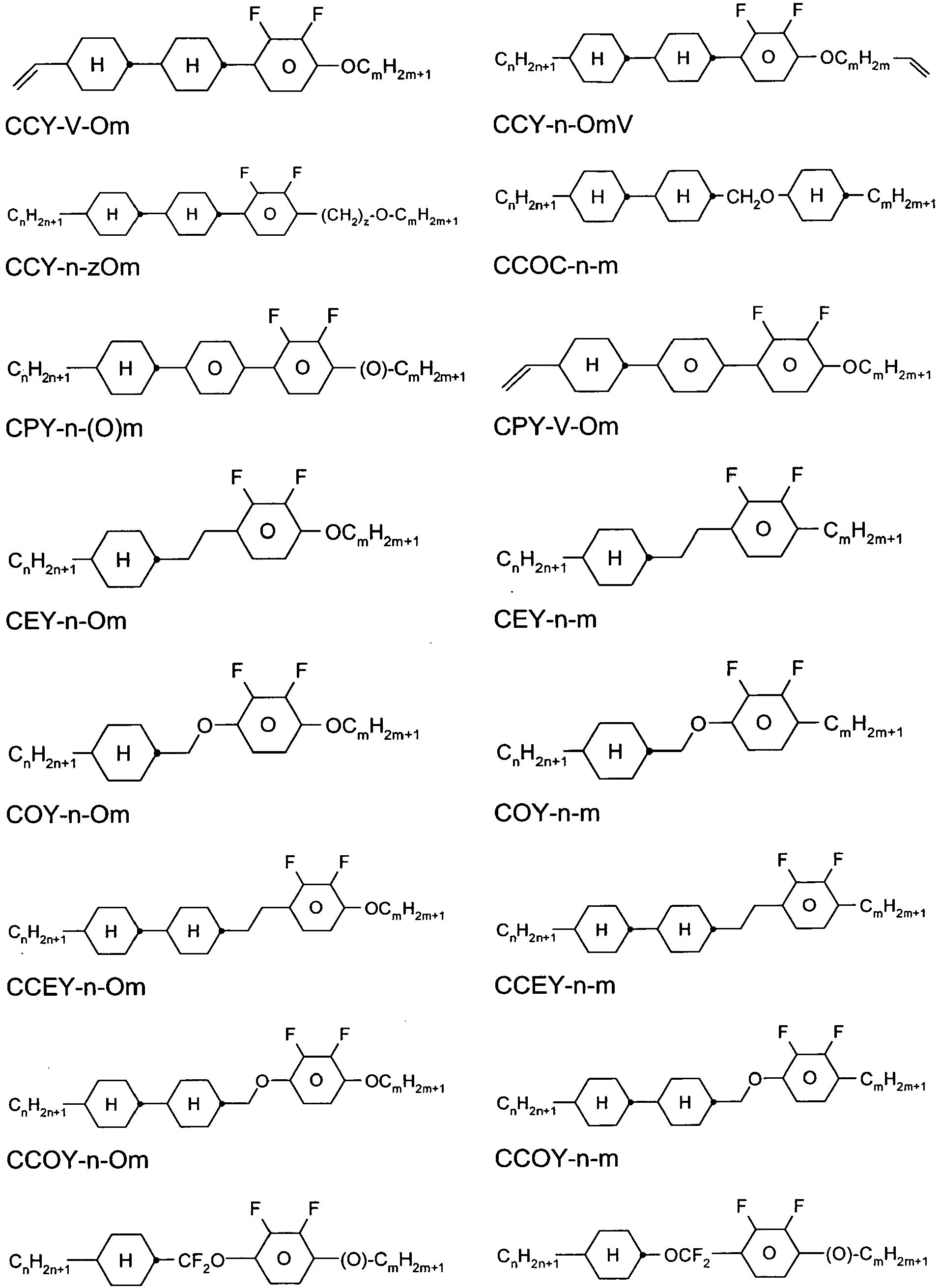

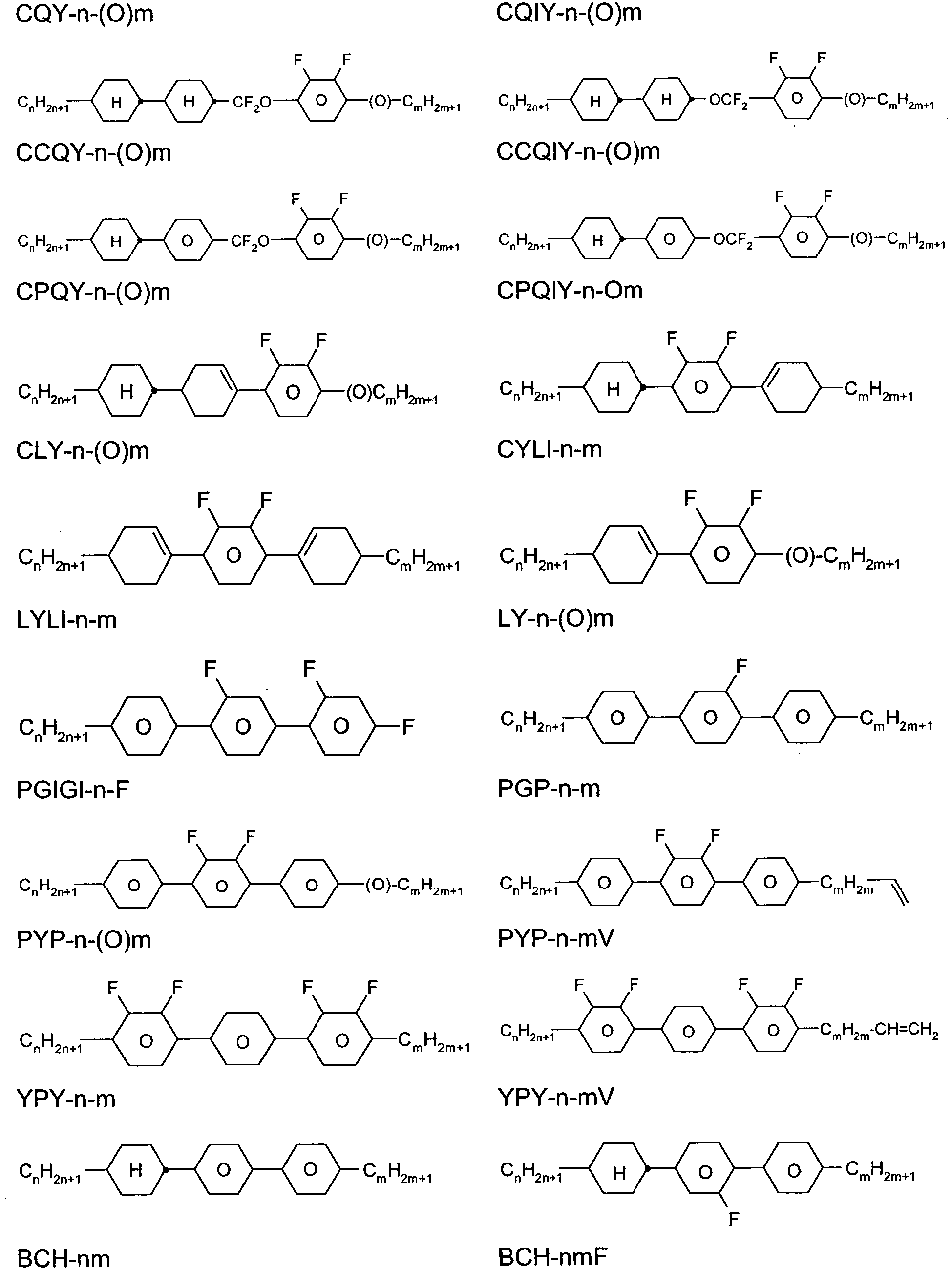

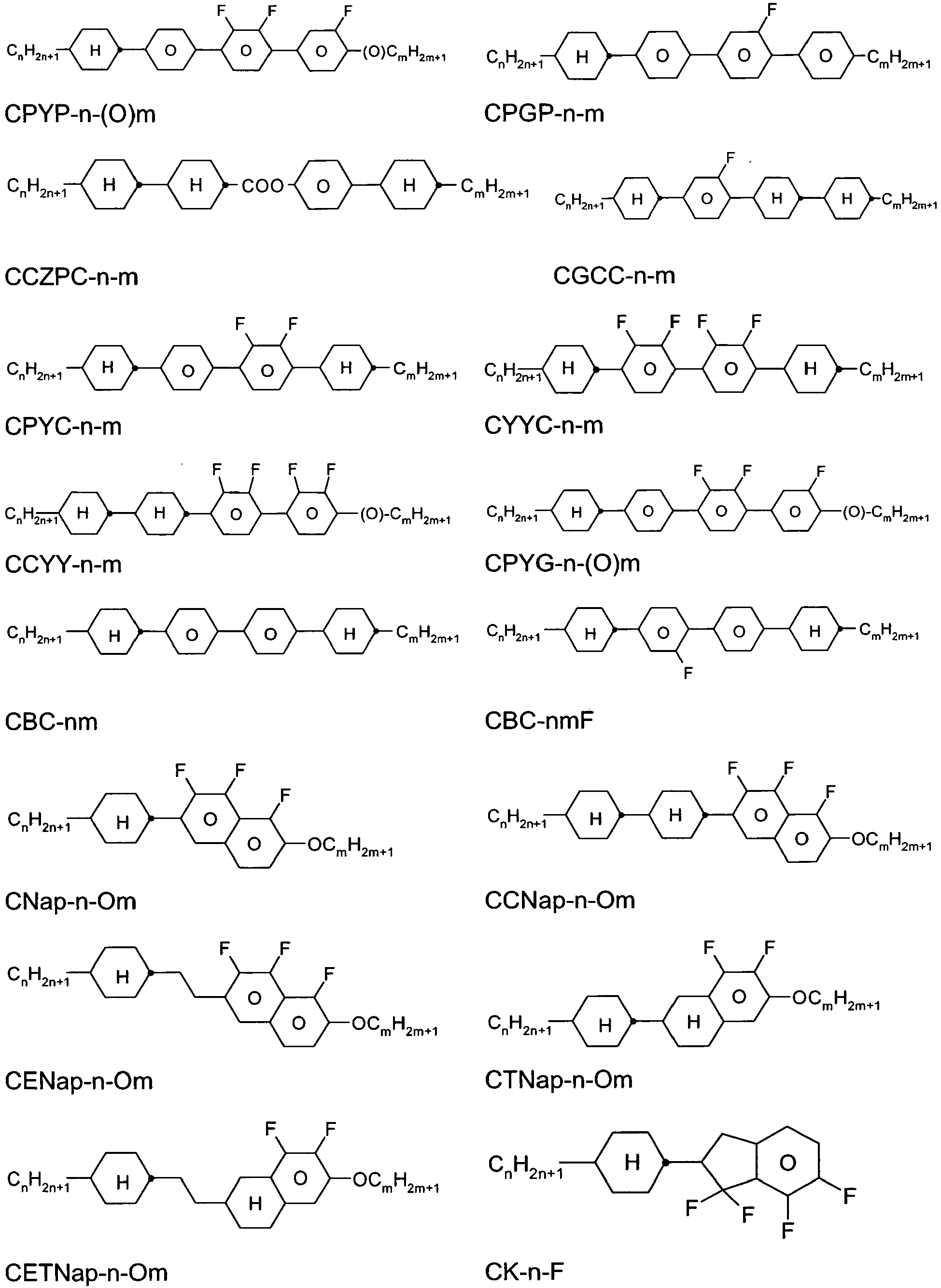

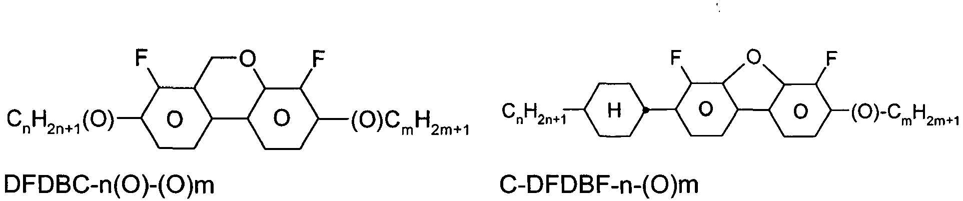

In a preferred embodiment of the present invention, the LC media according to the invention comprise one or more compounds selected from the group consisting of compounds from Table A.

**Table B**

| Table B shows possible chiral dopants which can be added to the LC media according to the invention. |
|---|
| 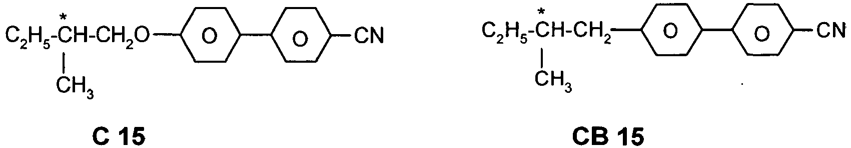 |
| 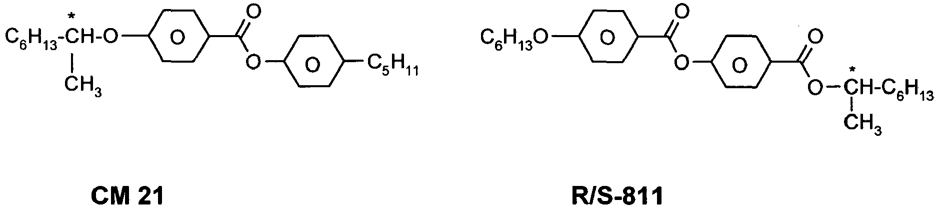 |
| 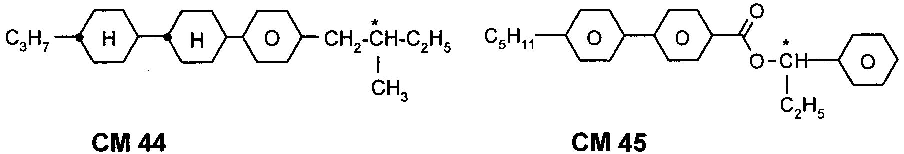 |
| 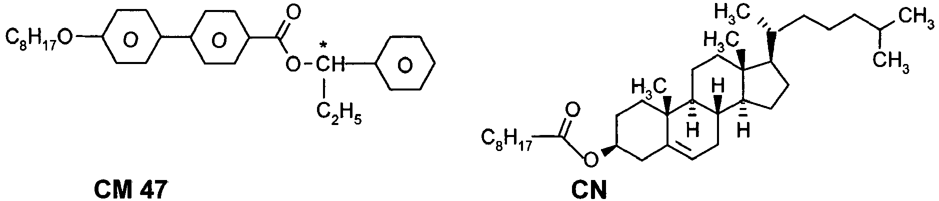 |
| 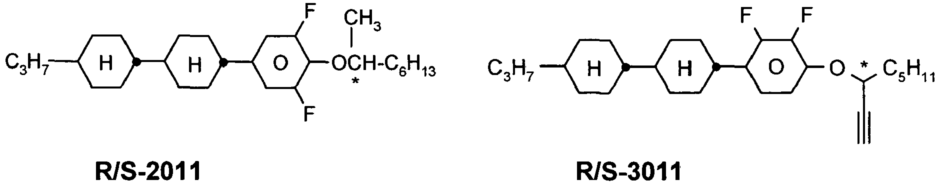 |
| 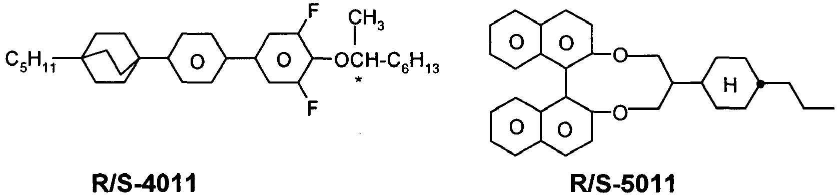 |
| 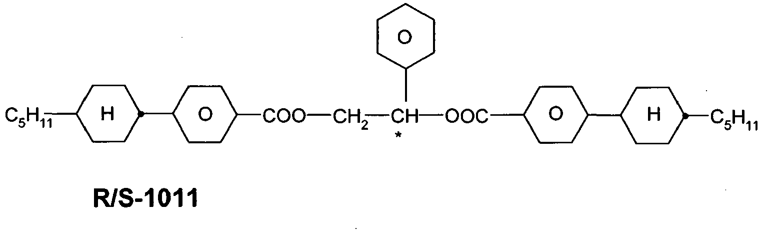 |

The LC media preferably comprise 0 to 10% by weight, in particular 0.01 to 5% by weight, particularly preferably 0.1 to 3% by weight, of dopants. The LC media preferably comprise one or more dopants selected from the group consisting of compounds from Table B.

**Table C**

| Table C shows possible stabilisers which can be added to the LC media according to the invention. |
|---|
| (n here denotes an integer from 1 to 12, preferably 1, 2, 3, 4, 5, 6, 7 or 8, terminal methyl groups are not shown). |
| 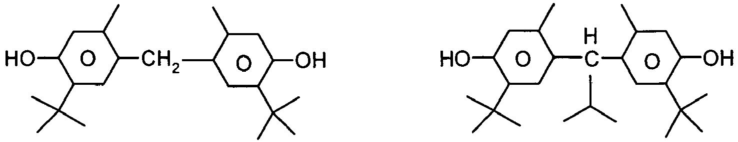 |
| 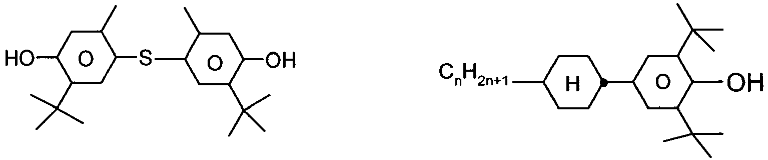 |
| 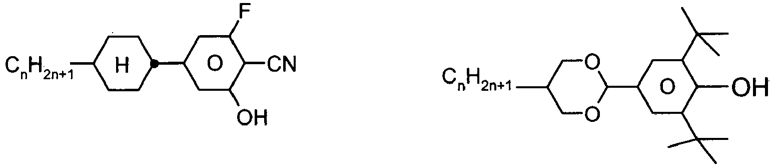 |
| 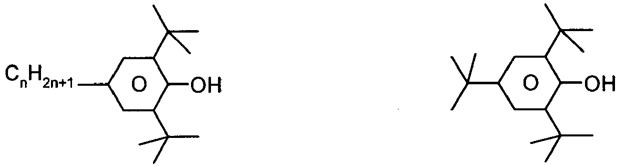 |
| 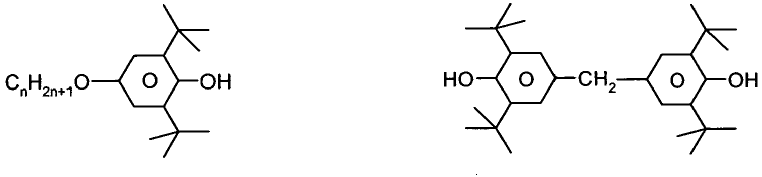 |
| 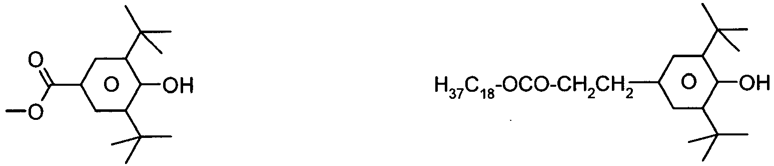 |
| 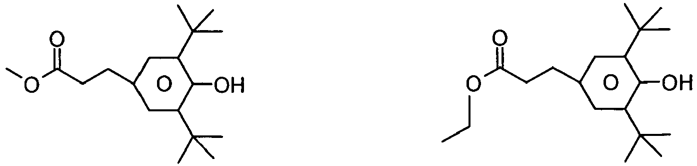 |
| 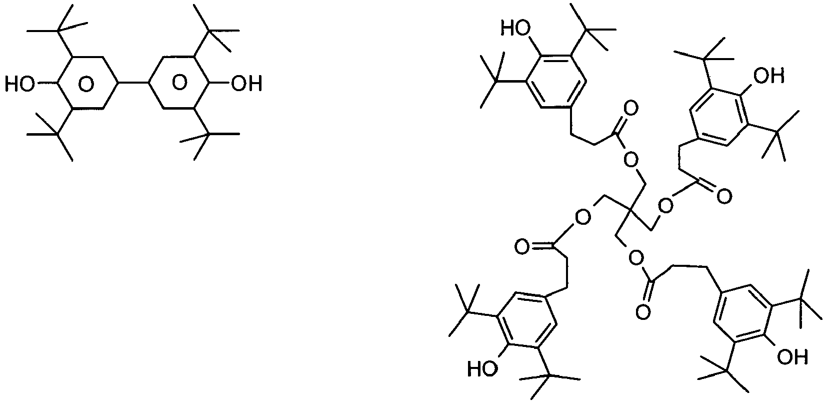 |
| 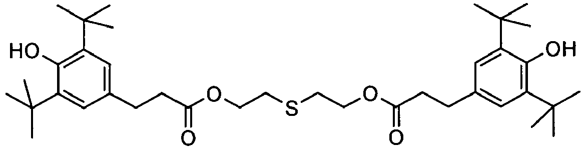 |
| 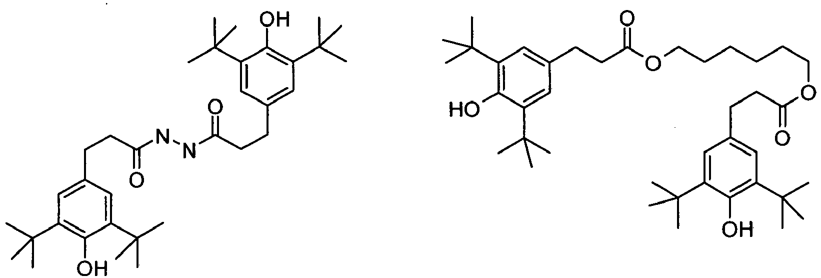 |
| 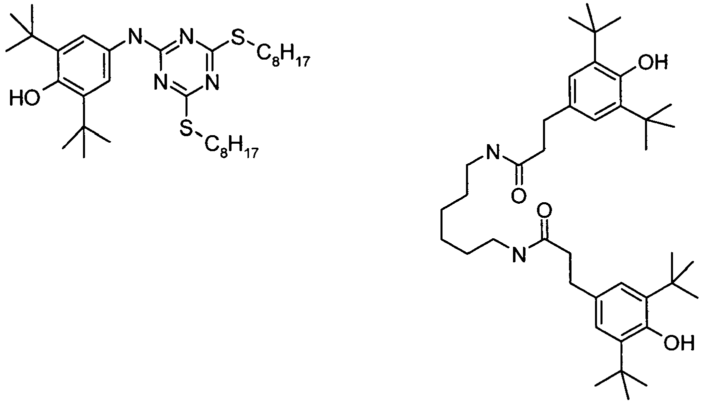 |
| 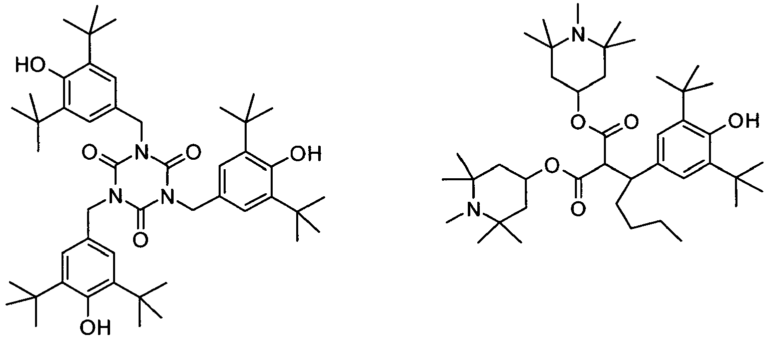 |
| 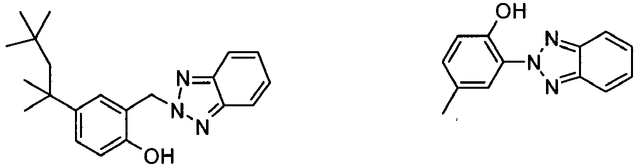 |
| 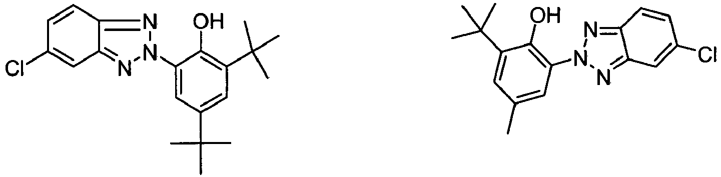 |
| 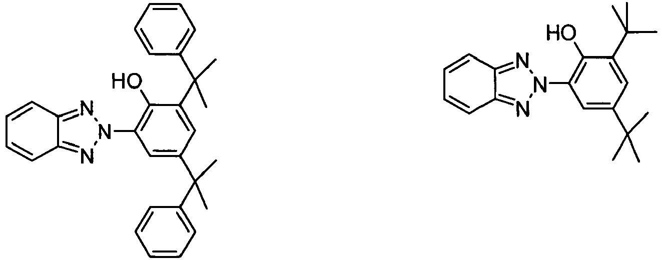 |
| 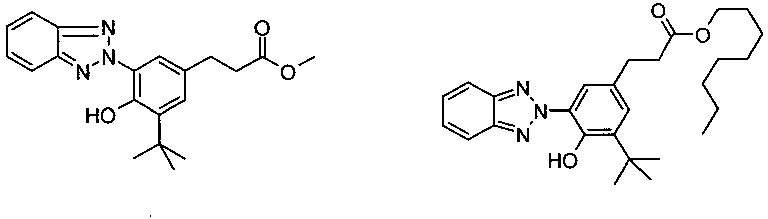 |
| 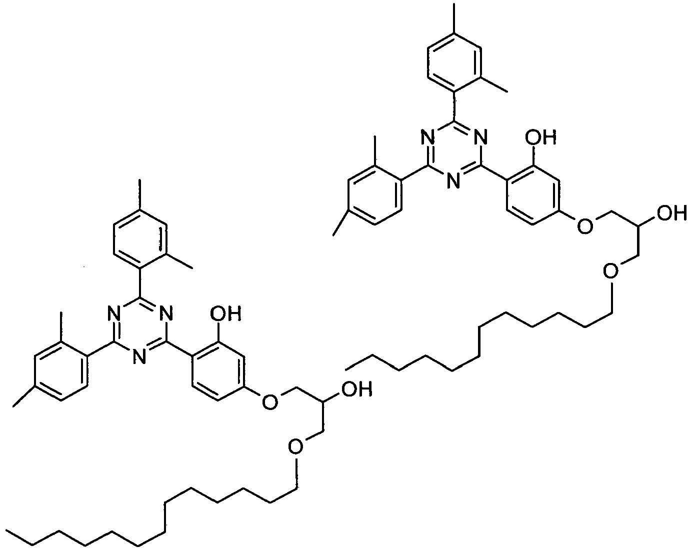 |
| 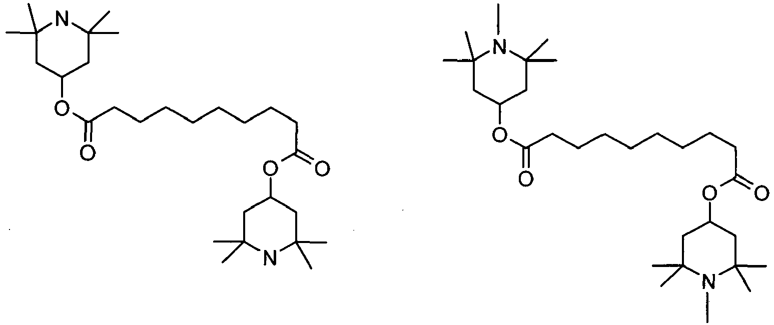 |

The LC media preferably comprise 0 to 10% by weight, in particular 1 ppm to 5% by weight, particularly preferably 1 ppm to 1% by weight, of stabilisers. The LC media preferably comprise one or more stabilisers selected from the group consisting of compounds from Table C.

**Table D**

| Table D shows illustrative compounds which can be used in the LC media in accordance with the present invention, preferably as reactive mesogenic compounds. |
|---|
| 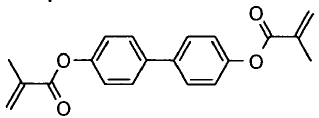 |
| 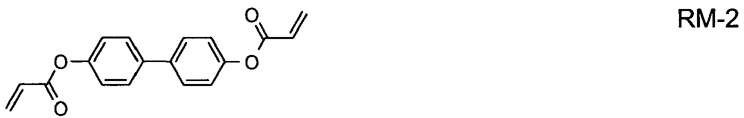 |
| 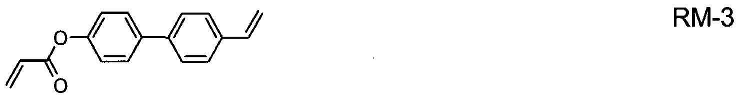 |
| 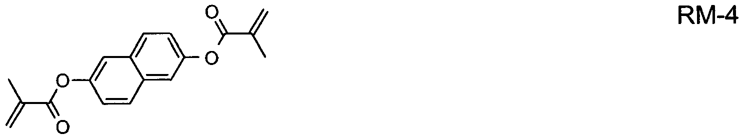 |
| 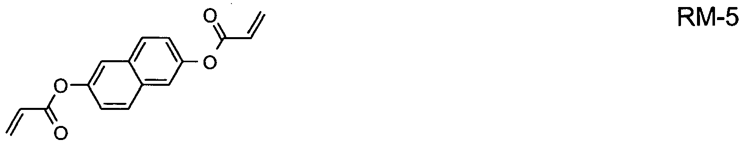 |
| 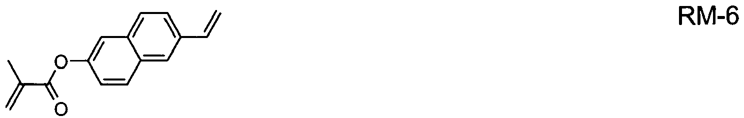 |
| 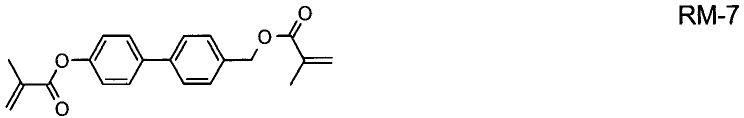 |
| 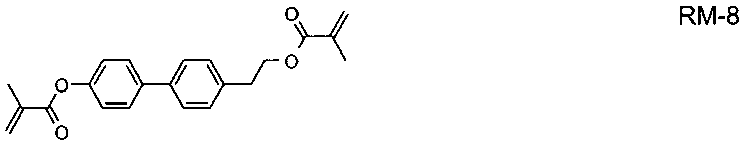 |
| 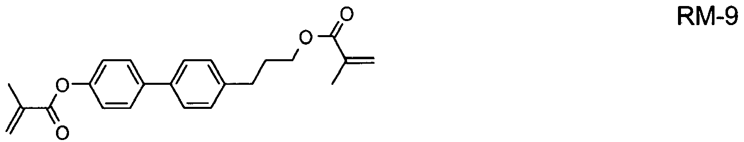 |
| 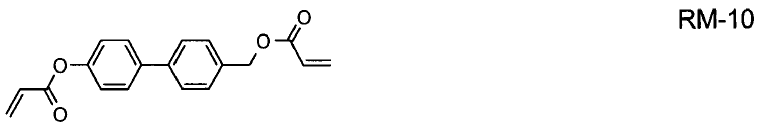 |
| 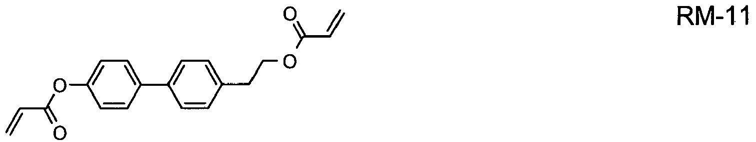 |
| 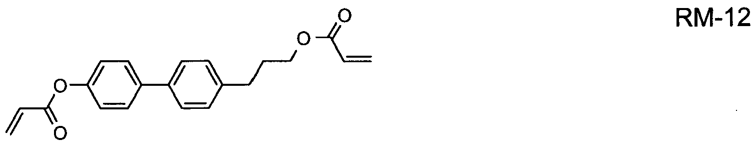 |
| 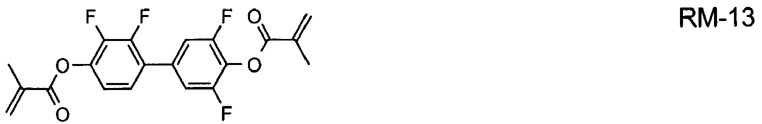 |
| 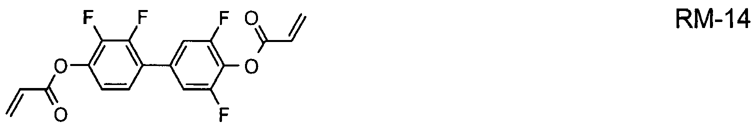 |
| 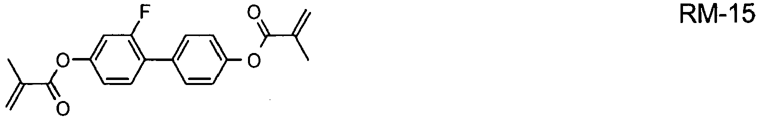 |
| 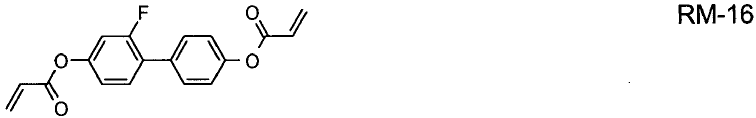 |
| 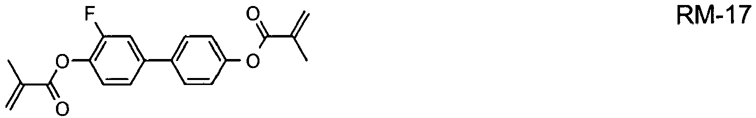 |
| 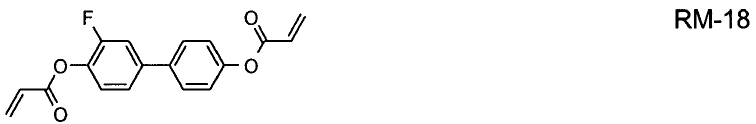 |
| 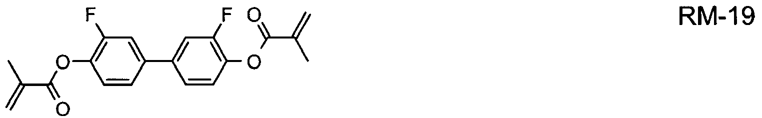 |
| 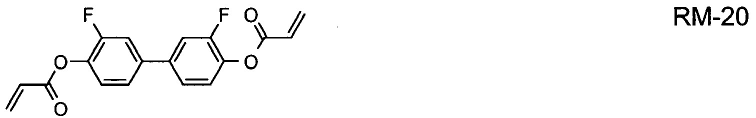 |
| 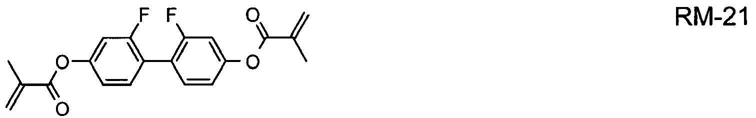 |
| 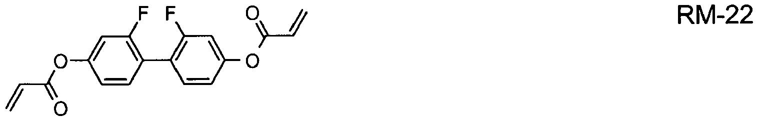 |
| 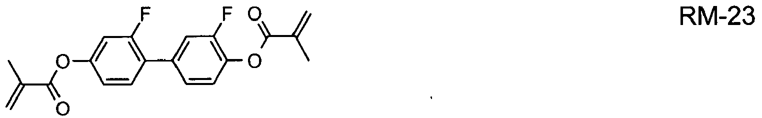 |
| 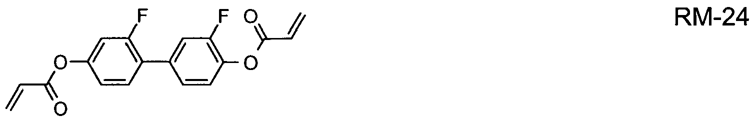 |
| 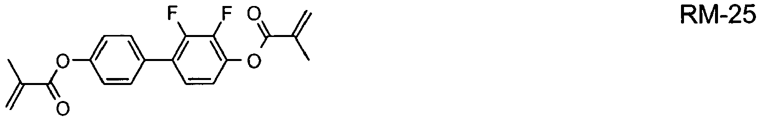 |
| 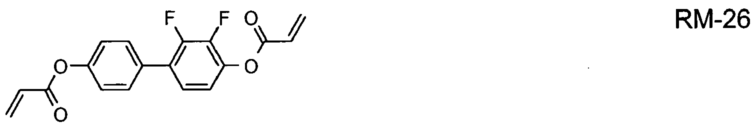 |
| 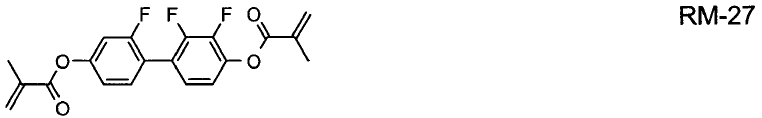 |
| 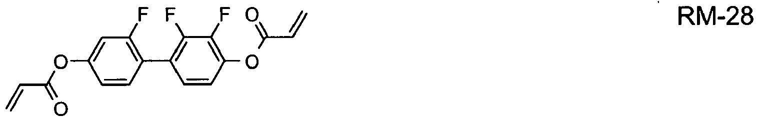 |
| 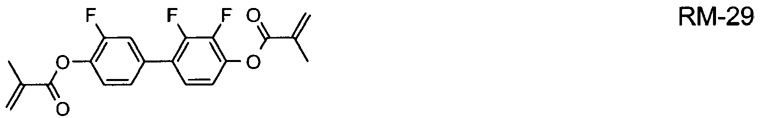 |
| 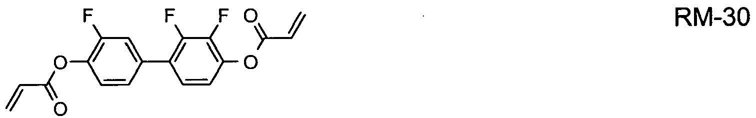 |
| 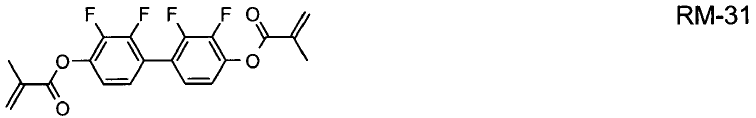 |
| 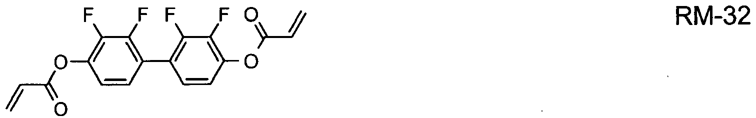 |
| 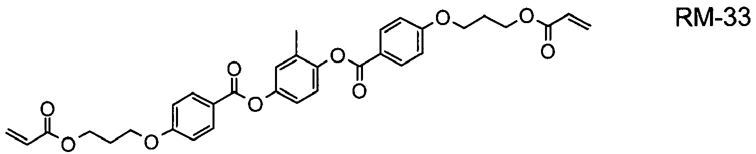 |
| 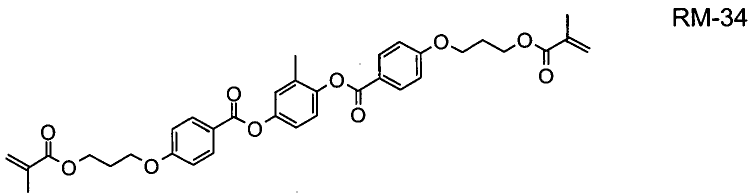 |
| 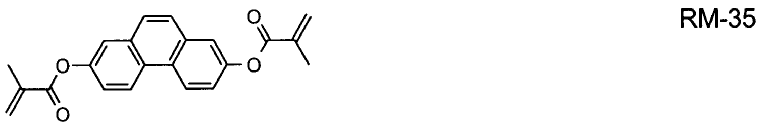 |
| 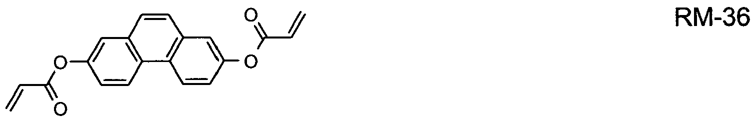 |
| 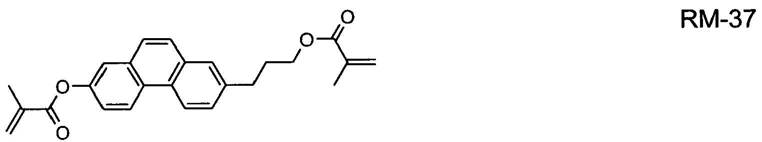 |
| 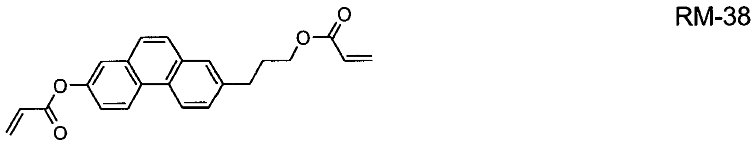 |
| 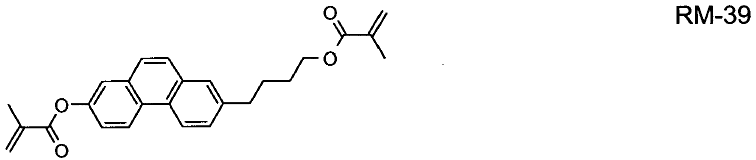 |
| 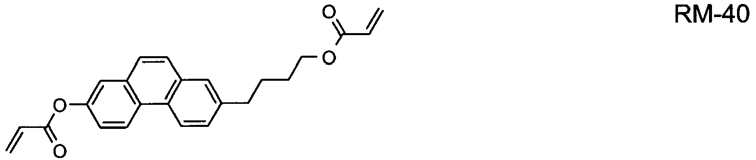 |
| 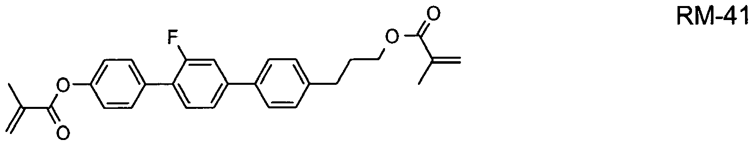 |
| 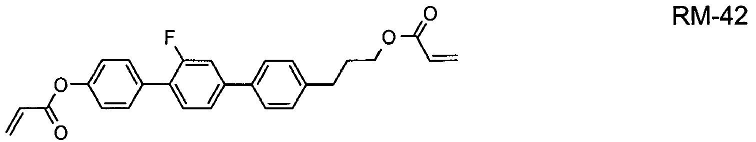 |
| 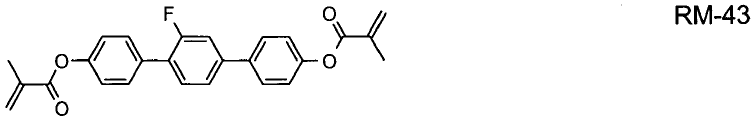 |
| 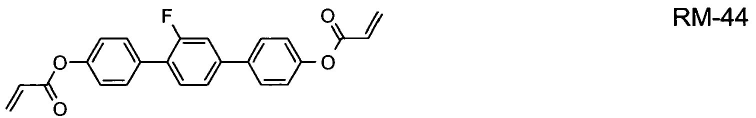 |
| 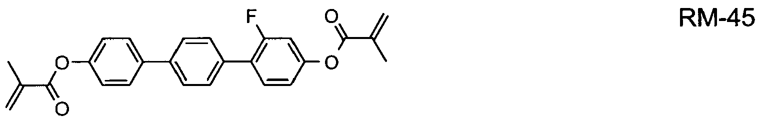 |
| 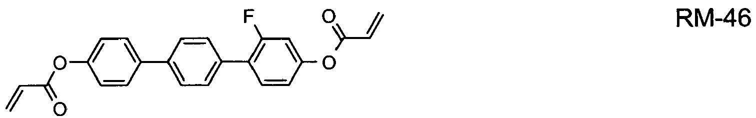 |
| 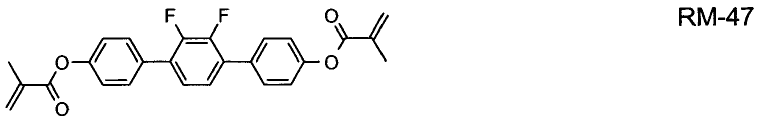 |
| 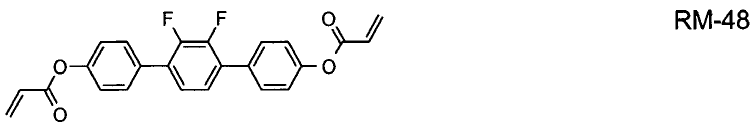 |
| 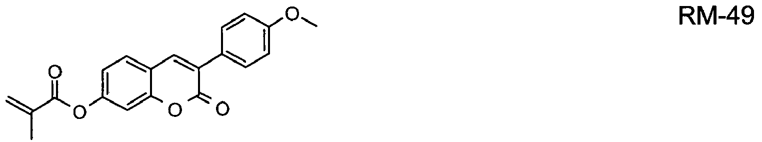 |
| 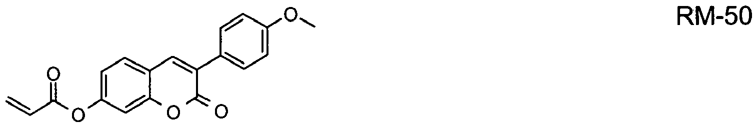 |
| 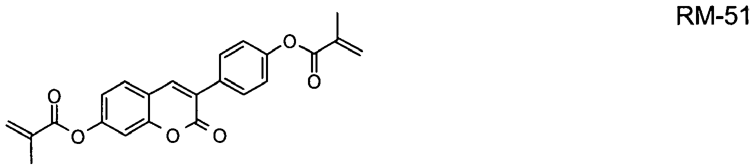 |
| 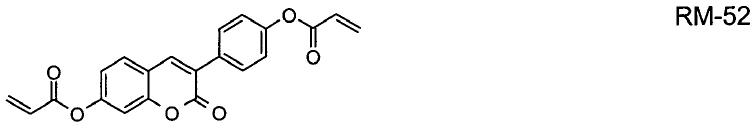 |
| 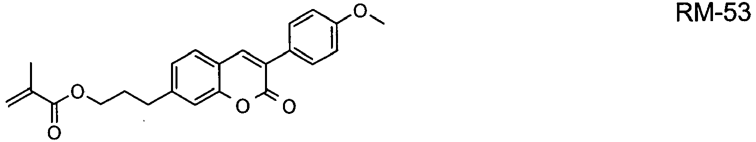 |
| 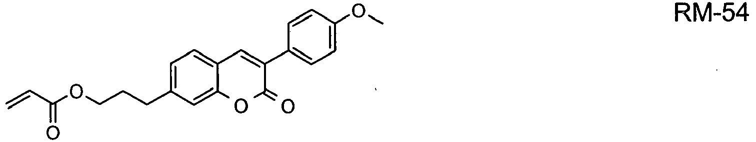 |
| 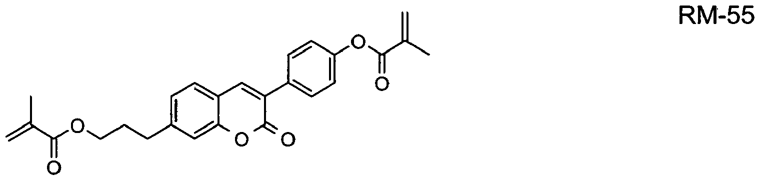 |
| 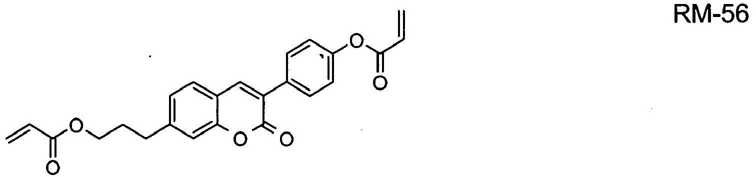 |
| 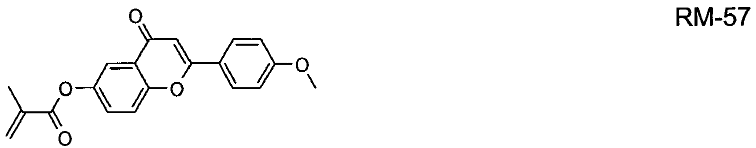 |
| 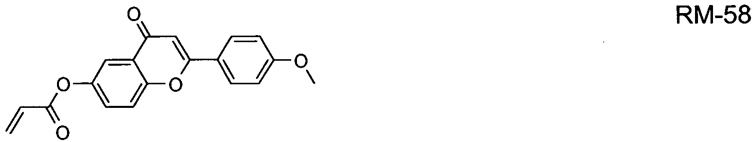 |
| 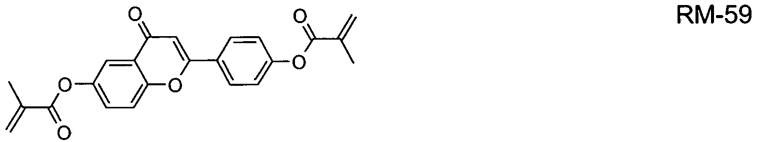 |
| 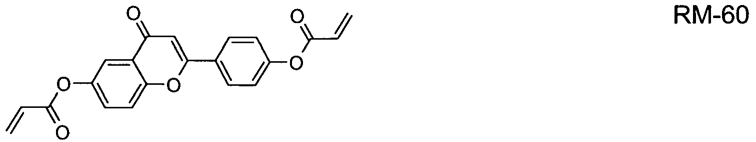 |
| 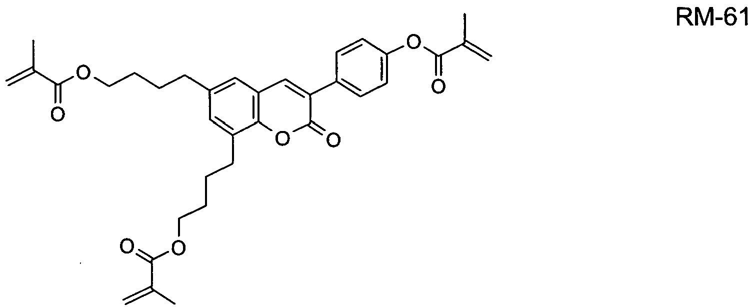 |
| 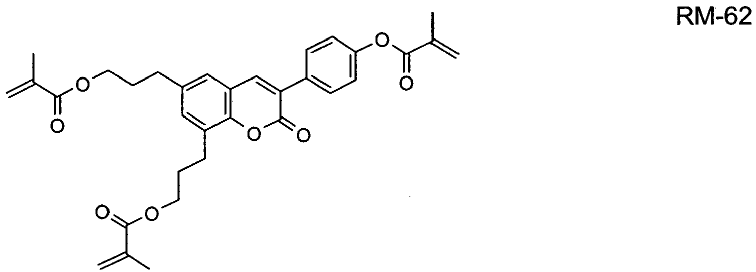 |
| 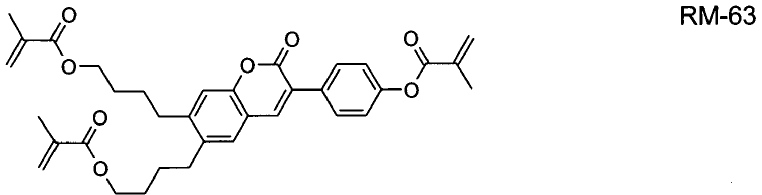 |
| 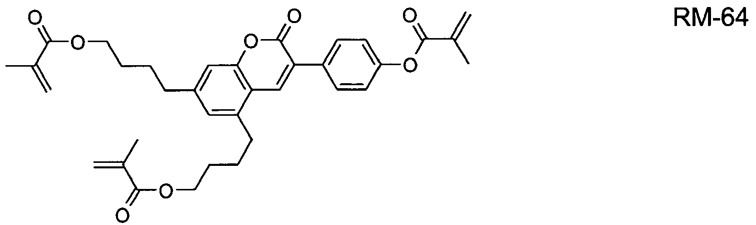 |
| 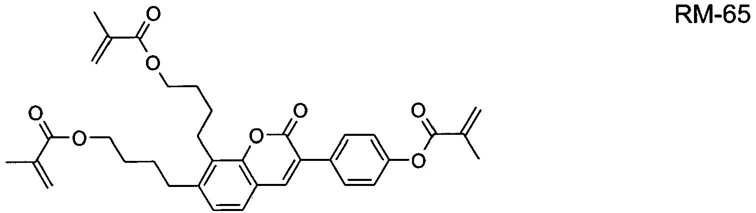 |
| 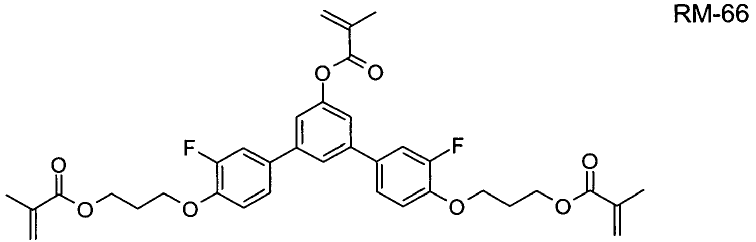 |
| 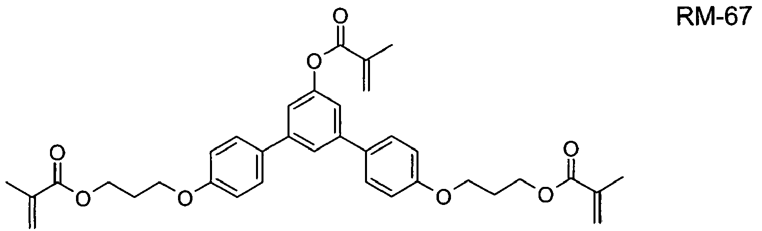 |
| 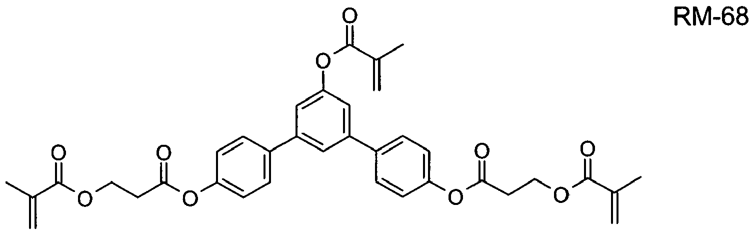 |
| 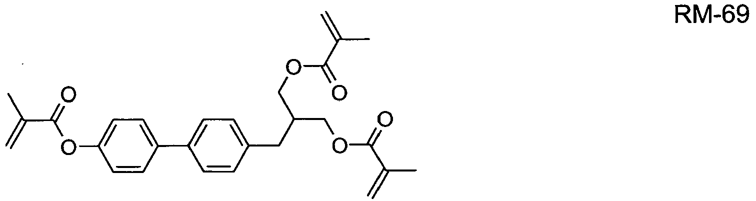 |
| 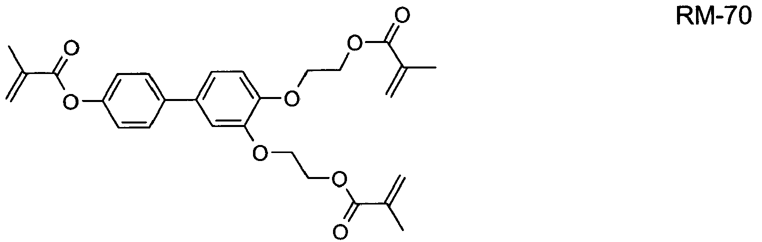 |
| 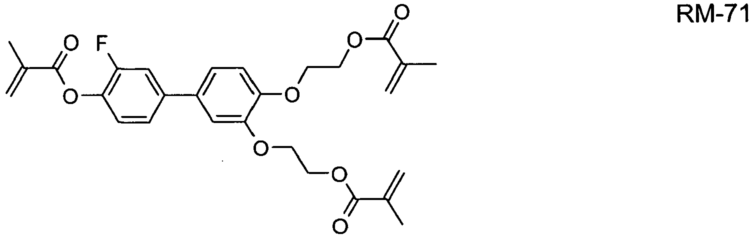 |

In a preferred embodiment of the present invention, the mesogenic media comprise one or more compounds selected from the group of the compounds from Table D.

In addition, the following abbreviations and symbols are used:
- V₀: threshold voltage, capacitive [V] at 20°C,
- nₑ: extraordinary refractive index at 20°C and 589 nm,
- nₒ: ordinary refractive index at 20°C and 589 nm,
- Δn: optical anisotropy at 20°C and 589 nm,
- ε_{⊥}: dielectric permittivity perpendicular to the director at 20°C and 1 kHz,
- ε_{∥}: dielectric permittivity parallel to the director at 20°C and 1 kHz,
- Δε: dielectric anisotropy at 20°C and 1 kHz,
- cl.p., T(N,I): clearing point [°C],
- γ₁: rotational viscosity at 20°C [mPa·s],
- K₁: elastic constant, "splay" deformation at 20°C [pN],
- K₂: elastic constant, "twist" deformation at 20°C [pN],
- K₃: elastic constant, "bend" deformation at 20°C [pN].

Unless explicitly noted otherwise, all concentrations in the present application are quoted in per cent by weight and relate to the corresponding mixture as a whole, comprising all solid or liquid-crystalline components, without solvents.

Unless explicitly noted otherwise, all temperature values indicated in the present application, such as, for example, for the melting point T(C,N), the transition from the smectic (S) to the nematic (N) phase T(S,N) and the clearing point T(N,I), are quoted in degrees Celsius (°C). M.p. denotes melting point, cl.p. = clearing point. Furthermore, C = crystalline state, N = nematic phase, S = smectic phase and I = isotropic phase. The data between these symbols represent the transition temperatures.

All physical properties are and have been determined in accordance with "Merck Liquid Crystals, Physical Properties of Liquid Crystals", Status Nov. 1997, Merck KGaA, Germany, and apply for a temperature of 20°C, and Δn is determined at 589 nm and Δε at 1 kHz, unless explicitly indicated otherwise in each case.

The term "threshold voltage" for the present invention relates to the capacitive threshold (V₀), also known as the Freedericks threshold, unless explicitly indicated otherwise. In the examples, the optical threshold may also, as generally usual, be quoted for 10% relative contrast (V₁₀).

Unless stated otherwise, the process of polymerising the polymerisable compounds in the PSA displays as described above and below is carried out at a temperature where the LC medium exhibits a liquid crystal phase, preferably a nematic phase, and most preferably is carried out at room temperature.

Unless stated otherwise, methods of preparing test cells and measuring their electrooptical and other properties are carried out by the methods as described hereinafter or in analogy thereto.

The display used for measurement of the capacitive threshold voltage consists of two plane-parallel glass outer plates at a separation of 25 µm, each of which has on the inside an electrode layer and an unrubbed polyimide alignment layer on top, which effect a homeotropic edge alignment of the liquid-crystal molecules.

The display or test cell used for measurement of the tilt angles consists of two plane-parallel glass outer plates at a separation of 4 µm, each of which has on the inside an electrode layer and a polyimide alignment layer on top, where the two polyimide layers are rubbed antiparallel to one another and effect a homeotropic edge alignment of the liquid-crystal molecules.

The polymerisable compounds are polymerised in the display or test cell by irradiation with UVA light of defined intensity for a prespecified time, with a voltage simultaneously being applied to the display (usually 10 V to 30 V alternating current, 1 kHz). In the examples, unless indicated otherwise, a metal halide lamp and an intensity of 100 mW/cm² is used for polymerisation. The intensity is measured using a standard UVA meter (Hoenle UV-meter high end with UVA sensor).

The tilt angle is determined by crystal rotation experiment (Autronic-Melchers TBA-105). A low value (i.e. a large deviation from the 90° angle) corresponds to a large tilt here.

The VHR value is measured as follows: 0.3% of a polymerisable monomeric compound is added to the LC host mixture, and the resultant mixture is introduced into VA-VHR test cells which comprise an unrubbed VA-polyimide alignment layer. The LC-layer thickness d is approx. 6 µm, unless stated othewise. The VHR value is determined after 5 min at 100°C before and after UV exposure at 1 V, 60 Hz, 64 µs pulse (measuring instrument: Autronic-Melchers VHRM-105).

### Example 1

Polymerisable mesogenic compound (RM) (1) is prepared as follows.

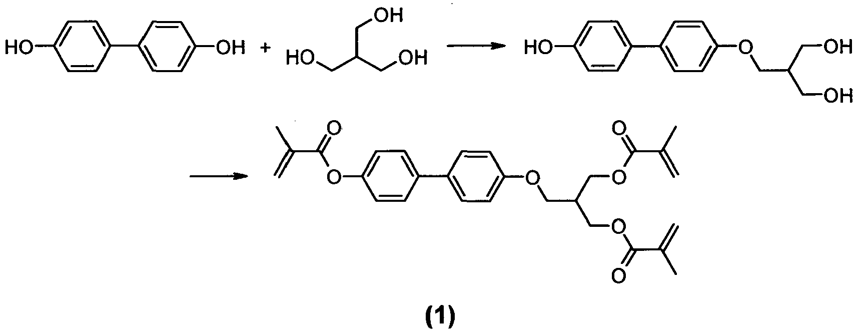

### 1.1 2-(4'-Hydroxy-biphenyl-4-yloxymethyl)-propane-1,3-diol

To a solution of biphenyl-4,4'-diol (7.02 g, 37.7 mmol), 2-hydroxylpropane-1,3-diol (4.00 g, 37.7 mmol) and triphenylphosphine (11.22 g, 42.8 mmol) in THF (100 ml) is added diisopropylazodicarboxylate (8.72 ml, 44.4 mmol) dropwise at room temperature. The resulted suspension is stirred at room temperature for 2 hs. The solvent is then removed in vacuo. The oily residue is purified by silica gel chromatography with dichloromethane/methyl-t-butylether as eluent. The obtained crude product is recrystallized with acetonitrile to provide 2-(4'-hydroxy-biphenyl-4-yloxymethyl)-propane-1,3-diol as white solid (1.0 g).

### 1.2 2-Methyl-acrylic acid 4'-[3-(2-methyl-acryloyloxy)-2-(2-methyl-acryloy loxymethyl)-propoxy]-biphenyl-4-yl ester

Methacrylic acid (1.79 g, 20.8 mmol) and 4-(dimethylamino)pyridine (0.045 g, 0.36 mmol) is added to a suspension of 2-(4'-hydroxy-biphenyl-4-yloxymethyl)-propane-1,3-diol (1.0 g, 3.6 mmol) in dichloromethane (40 ml). The reaction mixture is treated dropwise at 0 °C with a solution of *N*-(3-dimethylaminopropyl)-*N'*-ethylcarbodiimide (3.23 g, 20.8 mmol) in dichloromethane (10 ml) and stirred for 20 h at room temperature. After removing solvent in vacuo, the oily residue is purified by silica gel chromatography with dichloromethane. The obtained product is recrystallized from acetonitrile to afford white crystals of 2-methyl-acrylic acid 4'-[3-(2-methyl-acryloyloxy)-2-(2-methyl-acryloyloxymethyl)-propoxy]-biphenyl-4-yl ester (0.38 g, m.p. 104 °C).
¹H-NMR (DMSO-d₆, 500 MHz): δ (ppm) = 7.66 (d, *J* = 8.5 Hz, 2 H, Ar-H), 7.61 (d, *J* = 8.5 Hz, 2 H, Ar-H), 7.22 (d, *J* = 8.5 Hz, 2 H, Ar-H), 7.05 (d, *J* = 8.5 Hz, 2 H, Ar-H), 6.330 (m, 1 H, H_{olefin}), 6.07 (m, 2 H, H_{olefin}), 5.91 (m, 1 H, H_{olefin}), 5.69 (m, 2 H, H_{olefin}), 4.31 (d, *J* = 6 Hz, 4 H, OCH₂), 4.17 (d, *J* = 6 Hz, 2 H, OCH₂), 2.63 (m, 1 H, OCH₂*CH*), 2.02 (br. s, 3 H, CH₃), 1.89 (m, 6H, 2 x CH₃).

### Comparative Example 2

Polymerisable mesogenic compound (RM) (**2**) is prepared as follows.

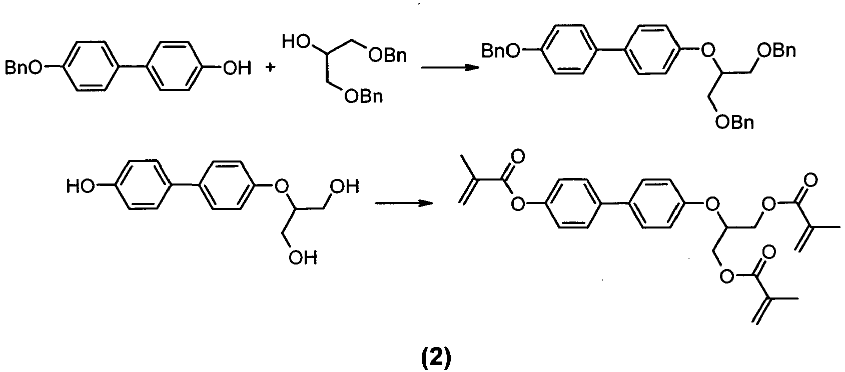

### 2.1 4'-(2-Benzyloxy-1-benzyloxymethyl-ethoxy)-biphenyl-4-ol

To a solution of 4'-benzoyloxy-biphenyl-4-ol (5.00 g, 18.1 mmol), 1,3-bis-benzyloxy-propan-2-ol (4.93 g, 18.1 mmol), and triphenylphosphine (5.39 g, 20.5 mmol) in THF (50 ml) is added diisopropylazodicarboxylate (4.19 ml, 21.3 mmol) dropwise at room temperature. The resulted suspension is stirred at room temperature overnight. The solvent is then removed in vacuo. The oily residue is purified by silica gel chromatography with toluene as eluent. The obtained crude product is recrystallized with heptanes/ethylacetate solvent mixture to provide 4'-(2-benzyloxy-1-benzyloxymethyl-ethoxy)-biphenyl-4-ol as white solid (5.2 g).

### 2.2 2-(4'-hydroxy-biphenyl-4-yloxy)-propane-1,3-diol

A solution of 4'-(2-benzyloxy-1-benzyloxymethyl-ethoxy)-biphenyl-4-ol (5.2 g, 9.8 mmol) in tetrahydrofuran (50 ml) is treated with palladium (5%) on activated charcoal (1.0 g) and submitted to hydrogenation for 22 hs. The catalyst is then filtered off, and the remaining solution is concentrated in vacuo. The residue is recrystallized from acetonitrile to give white crystals of 2-(4'-hydroxy-biphenyl-4-yloxy)-propane-1,3-diol (2.0 g).

### 2.3 2-Methyl-acrylic acid 4'-[2-(2-methyl-acryloyloxy)-1-(2-methyl-acryloy loxymethyl)-ethoxy]-biphenyl-4-yl ester

Methacrylic acid (3.78 g, 43.8 mmol) and 4-(dimethylamino)pyridine (0.094 g, 0.77 mmol) is added to a suspension of 2-(4'-hydroxy-biphenyl-4-yloxy)-propane-1,3-diol (2.0 g, 7.7 mmol) in dichloromethane (60 ml). The reaction mixture is treated dropwise at 0 °C with a solution of *N*-(3-dimethylaminopropyl)-*N'*-ethylcarbodiimide (6.80 g, 43.8 mmol) in dichloromethane (20 ml) and stirred for 20 h at room temperature. After removing solvent in vacuo, the oily residue is purified by silica gel chromatography with heptanes/ethylacetate as eluent. The obtained product is recrystallized from ethanol to afford white crystals of 2-methyl-acrylic acid 4'-[3-(2-methyl-acryloyloxy)-2-(2-methyl-acryloyloxymethyl)-propoxy]-biphenyl-4-yl ester (0.60 g, m.p. 50 °C).
¹H-NMR (DMSO-d₆, 500 MHz): δ (ppm) = 7.66 (d, *J* = 8.5 Hz, 2 H, Ar-H), 7.61 (d, *J* = 8.5 Hz, 2 H, Ar-H), 7.23 (d, *J* = 8.5 Hz, 2 H, Ar-H), 7.13 (d, *J* = 8.5 Hz, 2 H, Ar-H), 6.30 (m, 1 H, H_{olefin}), 6.00 (m, 2 H, H_{olefin}), 5.91 (m, 1 H, H_{olefin}), 5.68 (m, 2 H, H_{olefin}), 5.00 (m, 1 H, OCH), 4.42 (m, 4 H, 2 x OCH₂), 2.02 (br. s, 3 H, CH₃), 1.86 (m, 6H, 2 x CH₃).

### Example 3

Polymerisable mesogenic compound (RM) (3) is prepared as follows.

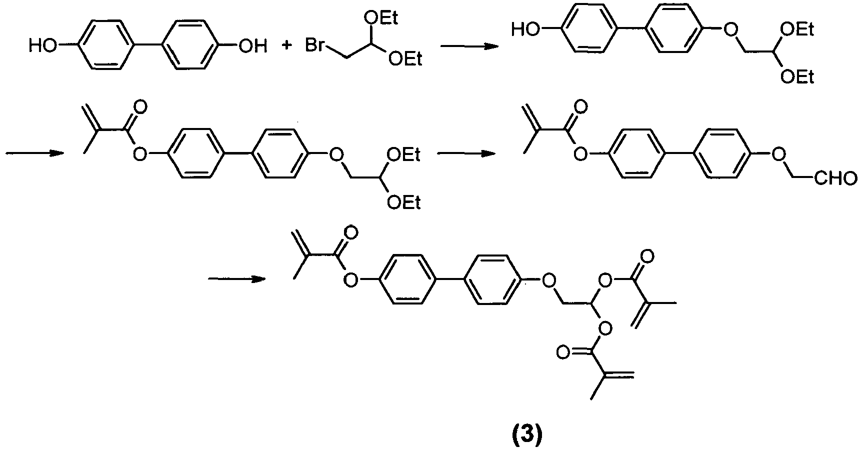

### 3.1 4'-(2,2-Diethoxy-ethoxy)-biphenyl-4-ol

Sodium hydride (60% suspension in paraffin oil, 5.08 g, 127.0 mmol) is added in several portions to a solution of biphenyl-4,4'-diol (21.5 g, 115.5 mmol) in dry DMF (270 ml) at 10°C. The resulted suspension is stirred at room temperature for one hour, and then heated to 60°C. The solution of 2-bromo-1,1-diethoxy-ethane (26.2 g, 132.8 mmol) in 30 ml dry DMF is added dropwise at 60°C. The reaction mixture is then heated to 95°C and stirred for 4 hours. After cooling to room temperature, the reaction mixture is added into 600 ml water, and extracted with 2 x 150 ml ethylacetate. The organic phase is combined. After removing solvent in vacuo, the crude product is purified by silica gel chromatography with toluene/acetate 4:1 as eluent to provide 4'-(2,2-diethoxy-ethoxy)-biphenyl-4-ol as white solid (13.5 g).

### 3.2 2-Methyl-acrylic acid 4'-(2,2-diethoxy-ethoxy)-biphenyl-4-yl ester

Methacrylic acid (6.02 g, 69.8 mmol) and 4-(dimethylamino)pyridine (0.36 g, 2.91 mmol) is added to a suspension of 4'-(2,2-diethoxy-ethoxy)-biphenyl-4-ol (17.7 g, 58.2 mmol) in dichloromethane (130 ml). The reaction mixture is treated dropwise at 0 °C with a solution of *N*-(3-dimethylaminopropyl)-*N'-*ethylcarbodiimide (11.3 g, 72.8 mmol) in dichloromethane (20 ml) and stirred overnight at room temperature. After removing solvent in vacuo, the oily residue is purified by silica gel chromatography with dichloromethane as eluent. The obtained product is recrystallized from heptanes/ethanol solvent mixture to afford white crystals of 2-methyl-acrylic acid 4'-(2,2-diethoxyethoxy)-biphenyl-4-yl ester (19.4 g).

### 3.3 2-Methyl-acrylic acid 4'-(2-oxo-ethoxy)-biphenyl-4-yl ester

To a solution of 2-methyl-acrylic acid 4'-(2,2-diethoxy-ethoxy)-biphenyl-4-yl ester (9.0 g, 24.3 mmol) in 60 ml aceton/120 ml THF solvent mixture is added 180 ml 2 M HCl dropwise at 5°C. The reaction mixture is stirred at room temperature overnight. After removing organic solvent in vacuo, the resulted suspension is cooled to 5°C. The precipitated crude product is recrystallized from acetonitrile to provide 2-methyl-acrylic acid 4'-(2-oxo-ethoxy)-biphenyl-4-yl ester as white crystals (7.1 g).

### 3.4 2-Methyl-acrylic acid 4'-[2,2-bis-(2-methyl-acryloyloxy)-ethoxy]-biphe nyl-4-yl ester

To a suspension of methacrylic acid anhydride (3.12 g, 20.2 mmol) in 20 ml dichloromethane is added dropwise 0.1 ml conc. HCl at 0°C. The solution of of 2-methyl-acrylic acid 4'-(2-oxo-ethoxy)-biphenyl-4-yl ester (3.0 g, 10.1 mmol) in 20 ml dichloromethane is then added dropwise. The reaction mixture is stirred at room temperature 48 hours. After removing solvent in vacuo, the oily residue is purified by silica gel chromatography with heptanes/ethylacetate as eluent. The obtained product is recrystallized from heptane to afford white crystals of 2-methyl-acrylic acid 4'-[2,2-bis-(2-methylacryloyloxy)-ethoxy]-biphenyl-4-yl ester (0.6 g, m.p. 85°C).
¹H-NMR (CDCl₃, 500 MHz): δ (ppm) = 7.54 (d, *J* = 8.5 Hz, 2 H, Ar-H), 7.50 (d, *J* = 8.5 Hz, 2 H, Ar-H), 7.24 (tr., *J* = 5 Hz, 1 H, OCHO), 7.16 (d, *J* = 8.5 Hz, 2 H, Ar-H), 7.02 (d, *J* = 8.5 Hz, 2 H, Ar-H), 6.37 (m, 1 H, H_{olefin}), 6.20 (m, 2 H, H_{olefin}), 5.77 (m, 1 H, H_{olefin}), 5.67 (m, 2 H, H_{olefin}), 4.31 (d, *J* = 5 Hz, 2 H, OCH₂), 2.08 (br. s, 3 H, CH₃), 1.97 (m, 6H, 2 x CH₃).

### Comparative Example 4

Polymerisable mesogenic compound (RM) **4** is prepared as follows. 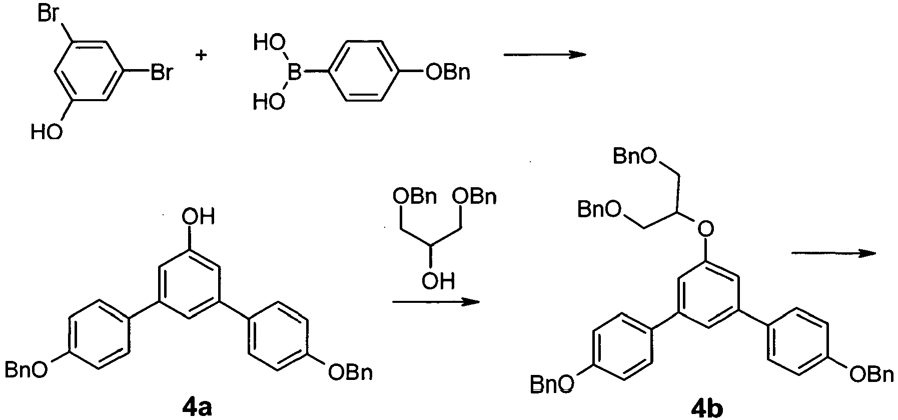 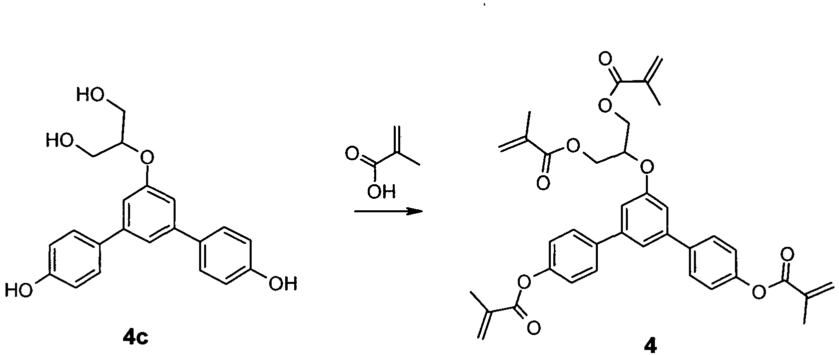
**4a:** To a solution of 4-benzyloxyl-phenylboronic acid (20.42 g, 89.5 mol) and 3-Benzyloxy-propionic acid 4-bromo-phenyl ester (29.00 g; 86.5 mmo) in 600 ml 1,4-dioxane was added 65.7 g (447.0 mmol) potassium phosphate. The resulted suspension is degassed carefully with argon. Tris(dibenzylidene acetone)dipalladium(0) (1.64 g; 1.8 mmol) and 2-dicyclohexylphosphine-2',6'-dimethoxylbiphenyl (SPhos) (3.03 g, 7.2 mmol) is then added. The reaction mixture is heated to reflux and stirred overnight. After cooling to room temperature 800 ml dist. water and 400 ml ethylacetate are added, and the mixture is neutralized carefully with 6 M HCl acid under cooling to pH∼4. The aqueous phase is extracted with ethylacetate. The organic phase is combined and washed with sat. aq. NaCl solution, dried over sodium sulfate. After removing solvent in vacuo, the solid residue is purified by recrystallization with ethanol to provide **4a** as white solid (16 g).
**4b**: To a solution of **4a** (5.5 g, 12.0 mmol), 1,3-bis-benzyloxy-propan-2-ol (3.27 g, 12.0 mmol), and triphenylphosphine (3.57 g, 13.6 mmol) in 40 ml THF is added diisopropylazodicarboxylate (2.78 ml, 14.1 mmol) dropwise at room temperature. The resulted suspension is stirred at room temperature overnight. The solvent is then removed in vacuo. The oily residue is purified by silica gel chromatography with heptane/ethylacetate as eluent to provide **4b** as colorless oil (6.5 g).
**4c**: A suspension of **4b** (6.3 g, 9.0 mmol) in tetrahydrofuran (70 ml) is treated with palladium (5%) on activated charcoal (2.0 g) and submitted to hydrogenation for 18 hs. The catalyst is then filtered off. After removing solvent *in vacuo,* the solid residue is recrystallized with dichloromethane to provide **4c** as white solid (2.5 g).
**4**: Methacrylic acid (2.75 g, 32.0 mmol) and 4-(dimethylamino)pyridine (0.087 g, 32.0 mmol) is added to a suspension of **4c** (2.5 g, 7.1 mmol) in 100 ml dichloromethane. The reaction mixture is treated dropwise at 0 °C with a solution of *N*-(3-dimethylaminopropyl)-*N'*-ethylcarbodiimide (4.96 g, 32.0 mmol) in 25 ml dichloromethane and stirred for 20 h at room temperature. After removing solvent in vacuo, the oily residue is purified by silica gel chromatography with heptane/ethyl acetate as eluent. The obtained product is recrystallized from ethanol to afford white crystals of **4** (2.5 g, m.p. 79 °C).

### Comparative Example 5

Polymerisable mesogenic compound (RM) 5 is prepared as follows. 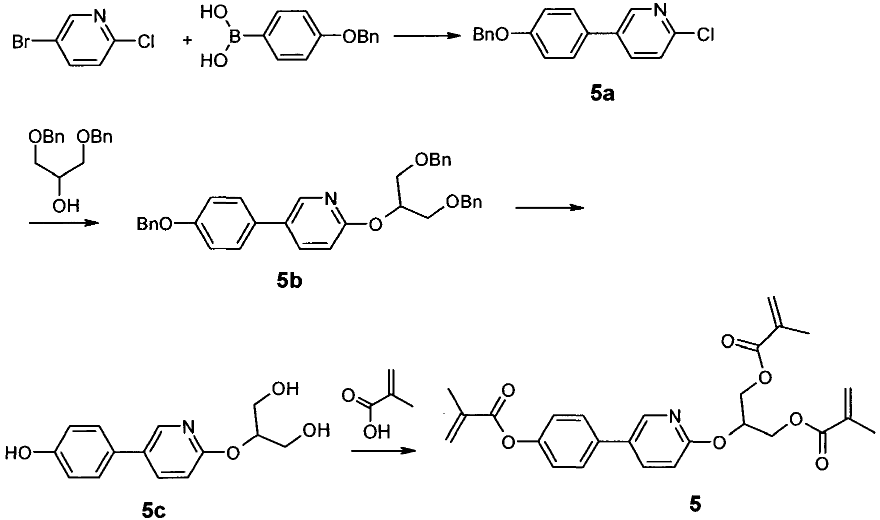
**5a:** To a solution of 4-benzyloxyl-phenylboronic acid (22.67 g, 99.4 mmol) and 5-bromo-2-chloropyridine (19.13 g, 99.4 mmol) in 200 ml toluene was added 100 ml dist. water and 100 ml ethanol. Sodium carbonate (35.00 g, 330.0 mmol) is added. The resulted suspension is degassed carefully with argon. Tetrakis(triphenylphosphine)palladium(0) (1.10 g, 0.95 mmol) is then added. The reaction mixture is heated to reflux and stirred for 4hs. After cooling to room temperature, the reaction mixture is neutralized carefully with 6 M HCl acid under cooling to pH∼7. The aqueous phase is extracted with ethyl acetate. The organic phase is combined and washed with sat. aq. NaCl solution, dried over sodium sulfate. After removing solvent in vacuo, the solid residue is purified by recrystallized from ethyl acetate to provide 5a as white solid (18.0 g).
**5b:** To a suspension of sodium hydrid (2.22 g, 55.4 mmol, 60% suspension in paraffin oil) in 40 ml dry THF was added dropwise the solution of 1,3-bis-benzyloxy-propan-2-ol (14.01 g, 51.4 mmol) under cooling. The reaction mixture was allowed to warm up slowly to RT and stirred further for 30 min. The solution of **5a** (13.7 g, 46.3 mmol) in 40 ml dry THF is then added at RT. The reaction mixture is stirred at RT for 1h, then poured into 300 ml ice-water mixture. The aqueous phase is extracted with ethyl acetate. The organic phase is combined and washed with sat. aq. NaCl solution, dried over sodium sulfate. After removing solvent in vacuo, the oily residue is purified by silica gel chromatography with heptane/ethylacetate as eluent to provide **5b** as colorless oil (6.5 g).
**5c**: A solution of **5b** (6.5 g, 12.3 mmol) and 0.4 ml conc. HCl acid in 150 ml methanol/20 ml THF solvent mixture is treated with palladium (5%) on activated charcoal (6.5 g) and submitted to hydrogenation under 2 bar at 30°C for 4hs. The catalyst is then filtered off, and the remaining solution is concentrated in vacuo. The oily residue is purified by silica gel chromatography with DCM/methanol solvent mixture as eluent to provide **5c** as yellowish oil (2.4 g).
**5:** Methacrylic acid (1.13 g, 13.0 mmol) and 4-(dimethylamino)pyridine (0.028 g, 0.23 mmol) is added to a suspension of **5c** (0.6 g, 2.3 mmol) in 50 ml dichloromethane. The reaction mixture is treated dropwise at 0 °C with a solution of *N*-(3-dimethylaminopropyl)-*N'*-ethylcarbodiimide (2.03 g, 13.1 mmol) in 10 ml dichloromethane and stirred for 20 h at room temperature. After removing solvent in vacuo, the oily residue is purified by silica gel chromatography with heptanes/ethylacetate as eluent. The obtained product is recrystallized from ethanol to afford **5** as white crystals (0.15 g, m.p. 68 °C).

### Mixture Examples

### A) Host Mixtures

### Example 1

The nematic LC mixture N1 is formulated as follows.

| | | | |
|---|---|---|---|
| CCH-501 | 9.00% | cl.p. | + 70.0 °C |
| CCH-35 | 14.00% | Δn | 0.0825 |
| PCH-53 | 8.00% | Δε | - 3.5 |
| PCH-304FF | 14.00% | ε_{∥} | 3,5 |
| PCH-504 | 13.00% | K₃/K₁ | 1.00 |
| CCP-302FF | 8.00% | γ₁ | 141 mPa s |
| CCP-503FF | 8.00% | V₀ | 2.6 V |
| CCP-21FF | 9.00% | | |
| CCP-31FF | 9.00% | | |
| CPY-2-O2 | 8.00% | | |

### Example 2

The nematic LC mixture N2 is formulated as follows.

| | | | |
|---|---|---|---|
| CY-3-O2 | 18.00% | cl.p. | + 74.5 °C |
| CPY-2-O2 | 10.00% | Δn | 0.1021 |
| CPY-3-O2 | 10.00% | Δε | - 3.1 |
| CCY-3-O2 | 9.00% | ε_{∥} | 3.5 |
| CCY-4-O2 | 4.00% | K₃/K₁ | 1.16 |
| CC-3-V | 40.00% | γ₁ | 86 mPa s |
| PYP-2-3 | 9.00% | V₀ | 2.29 V |

### Example 3

The nematic LC mixture N3 is formulated as follows.

| | | | |
|---|---|---|---|
| CC-3-V | 20.00% | cl.p. | + 74.5 °C |
| CC-3-V1 | 10.00% | Δn | 0.1084 |
| CCH-34 | 8.00% | Δε | - 3.2 |
| CCH-35 | 4.00% | ε_{∥} | 3.5 |
| CCY-3-O1 | 5.50% | K₃/K₁ | 1.04 |
| CCY-3-O2 | 12.00% | γ₁ | 94 mPa s |
| CPY-2-O2 | 2.00% | V₀ | 2.33 V |
| CPY-3-O2 | 12.00% | | |
| PY-3-O2 | 15.00% | | |
| PY-4-O2 | 8.50% | | |
| PYP-2-3 | 3.00% | | |

### Example 4

The nematic LC mixture N4 is formulated as follows.

| | | | |
|---|---|---|---|
| CC-3-V | 27.50% | cl.p. | + 74.8 °C |
| CC-3-V1 | 7.50% | Δn | 0.0986 |
| CCH-23 | 3.00% | Δε | - 3.4 |
| CCP-3-1 | 3.75% | ε_{∥} | |
| CCY-3-02 | 12.50% | K₃/K₁ | 1.16 |
| CPY-2-02 | 11.50% | γ₁ | 95 mPa s |
| CPY-3-02 | 10.50% | V₀ | 2.26 V |
| CY-3-02 | 15.50% | | |
| PY-3-02 | 3.00% | | |
| PY-4-02 | 5.25% | | |

### Example 5

The nematic LC mixture N5 is formulated as follows.

| | | | |
|---|---|---|---|
| CC-3-V | 41.50% | cl.p. | + 74.6 °C |
| CCP-3-1 | 2.00% | Δn | 0.0983 |
| CCY-3-01 | 5.25% | Δε | - 3.1 |
| CCY-3-02 | 12.50% | ε_{∥} | |
| CPY-2-02 | 12.25% | K₃/K₁ | 1.11 |
| CPY-3-02 | 7.50% | γ₁ | 85 mPa s |
| CY-3-02 | 5.50% | V₀ | 2.29 V |
| PY-3-02 | 3.50% | | |
| PY-4-02 | 10.00% | | |

### Example 6

The nematic LC mixture N6 is formulated as follows.

| | | | |
|---|---|---|---|
| CC-3-V | 27.50% | cl.p. | + 75.6 °C |
| CC-3-V1 | 8.00% | Δn | 0.0989 |
| CCH-23 | 2.50% | Δε | - 3.4 |
| CCP-3-1 | 3.00% | ε_{∥} | |
| CCY-3-02 | 12.00% | K₃/K₁ | 1.16 |
| CCY-4-02 | 2.00% | γ₁ | 94 mPa s |
| CPY-2-02 | 10.00% | V₀ | 2.28 V |
| CPY-3-02 | 10.50% | | |
| CY-3-02 | 15.50% | | |
| CY-3-04 | 1.00% | | |
| PY-3-02 | 7.00% | | |
| PYP-2-3 | 1.00% | | |

### Example 7

The nematic LC mixture N7 is formulated as follows.

| | | | |
|---|---|---|---|
| CC-3-V | 41.50% | cl.p. | + 74.5 °C |
| CCY-3-01 | 2.50% | Δn | 0.0984 |
| CCY-3-02 | 11.50% | Δε | -3.3 |
| CCY-3-03 | 5.00% | ε_{∥} | |
| CPY-2-02 | 5.00% | K₃/K₁ | 1.15 |
| CPY-3-02 | 12.00% | γ₁ | 89 mPa s |
| CY-3-02 | 9.50% | V₀ | 2.29 V |
| PY-3-02 | 7.00% | | |
| PY-4-02 | 3.00% | | |
| PYP-2-3 | 3.00% | | |

### Example 8

The nematic LC mixture N8 is formulated as follows.

| | | | |
|---|---|---|---|
| CC-3-V | 28.00% | cl.p. | + 74.9 °C |
| CC-3-V1 | 10.00% | Δn | 0.1026 |
| CCH-35 | 1.00% | Δε | - 3.0 |
| CCP-3-1 | 6.00% | ε_{∥} | |
| CCY-3-02 | 12.00% | K₃/K₁ | 1.19 |
| CCY-4-02 | 3.00% | γ₁ | 90 mPa s |
| CPY-3-02 | 12.00% | V₀ | 2.47 V |
| CY-3-02 | 10.00% | | |
| PY-3-02 | 15.00% | | |
| PYP-2-3 | 3.00% | | |

### Example 9

The nematic LC mixture N9 is formulated as follows.

| | | | |
|---|---|---|---|
| CC-3-V | 15.00% | cl.p. | + 74.4 °C |
| CC-3-V1 | 9.00% | Δn | 0.1086 |
| CCH-23 | 8.00% | Δε | - 3.2 |
| CCH-34 | 7.50% | ε_{∥} | |
| CCY-3-02 | 10.00% | K₃/K₁ | 1.10 |
| CCY-5-02 | 8.00% | γ₁ | 102 mPa s |
| CPY-2-02 | 3.00% | V₀ | 2.33 V |
| CPY-3-02 | 8.50% | | |
| CY-3-02 | 7.00% | | |
| PY-3-02 | 16.00% | | |
| PYP-2-3 | 8.00% | | |

### Example 10

The nematic LC mixture N10 is formulated as follows.

| | | | |
|---|---|---|---|
| CC-3-V | 42.00% | cl.p. | + 73.5 °C |
| CCY-3-01 | 5.00% | Δn | 0.1007 |
| CCY-3-02 | 10.00% | Δε | - 3.5 |
| CCY-4-02 | 2.50% | ε_{∥} | |
| CPY-2-02 | 10.00% | K₃/K₁ | 1.13 |
| CPY-3-02 | 10.00% | γ₁ | 85 mPa s |
| CY-3-02 | 6.50% | V₀ | 2.15 V |
| PY-3-02 | 11.00% | | |
| IS-18566 | 3.00% | | |

### Example 11

The nematic LC mixture N11 is formulated as follows.

| | | | |
|---|---|---|---|
| CC-3-V | 45.50% | cl.p. | + 73.0 °C |
| CCY-3-01 | 3.00% | Δn | 0.1011 |
| CCY-3-02 | 11.00% | Δε | - 3.5 |
| CCY-4-02 | 3.50% | ε_{∥} | |
| CPY-2-02 | 7.50% | K₃/K₁ | 1.09 |
| CPY-3-02 | 10.00% | γ₁ | 79 mPa s |
| CY-3-02 | 2.00% | V₀ | 2.15 V |
| PY-3-02 | 11.50% | | |
| IS-18566 | 6.00% | | |

### Example 12

The nematic LC mixture N12 is formulated as follows.

| | | | |
|---|---|---|---|
| CC-3-V | 34.50% | cl.p. | + 75.0 °C |
| CC-3-V1 | 8.00% | Δn | 0.1075 |
| CCY-3-01 | 7.00% | Δε | - 3.1 |
| CCY-3-02 | 11.50% | ε_{∥} | |
| CCY-4-02 | 3.50% | K₃/K₁ | 1.12 |
| CPY-3-02 | 11.50% | γ₁ | 84 mPa s |
| PY-3-02 | 13.00% | V₀ | 2.41 V |
| PP-1-2V1 | 6.00% | | |
| IS-18566 | 5.00% | | |

### Example 13

The nematic LC mixture N13 is formulated as follows.

| | | | |
|---|---|---|---|
| CY-3-02 | 16.50% | cl.p. | + 74.0 °C |
| CCY-4-02 | 10.50% | Δn | 0.1069 |
| CCY-5-02 | 6.00% | Δε | - 3.2 |
| CPY-2-02 | 9.00% | ε_{∥} | |
| CPY-3-02 | 9.00% | K₃/K₁ | 1.06 |
| CCH-34 | 9.00% | γ₁ | 117 mPa s |
| CCH-31 | 20.00% | V₀ | 2.18 V |
| CCP-3-1 | 2.00% | | |
| PYP-2-3 | 6.50% | | |
| PYP-2-4 | 6.50% | | |
| PCH-301 | 5.00% | | |

### Example 14

The nematic LC mixture N14 is formulated as follows.

| | | | |
|---|---|---|---|
| CY-3-02 | 12.00% | cl.p. | + 74.0 °C |
| CY-3-04 | 10.00% | Δn | 0.1064 |
| CCY-3-02 | 6.00% | Δε | - 3.2 |
| CCY-4-02 | 6.50% | ε_{∥} | |
| CCH-34 | 9.00% | K₃/K₁ | 0.99 |
| CCH-35 | 5.00% | γ₁ | 119 mPa s |
| CCP-3-1 | 14.50% | V₀ | 2.19 V |
| CCP-3-3 | 11.00% | | |
| PYP-2-3 | 9.00% | | |
| PYP-2-4 | 8.00% | | |
| Y-40-04 | 9.00% | | |

### Example 15

The nematic LC mixture N15 is formulated as follows.

| | | | |
|---|---|---|---|
| CC-3-V | 28.50% | cl.p. | +74.5 °C |
| CCP-31 | 12.50% | Δn | 0.1077 |
| CCOY-2-02 | 19.00% | Δε | -3.2 |
| CCOY-3-02 | 11.50% | ε_{∥} | 3.6 |
| PY-3-02 | 13.50% | K₃/K₁ | 0.91 |
| PP-1-3 | 10.00% | γ₁ | 99 |
| PYP-2-3 | 5.00% | V₀ | 2.34 V |

### B) Polymerisable Mixtures

### Examples P1.1-P3.15

Polymerisable mixtures P1.1, P1.2, P1.3, P1.4, P1.5, P1.6, P1.7, P1.8, P1.9, P1.10, P1.11, P1.12, P1.13, P1.14 and P1.15 according to the invention are prepared by adding RM 1 of Example 1 to each of LC mixtures N1-N15, respectively, at a concentration of 0.3% by weight.

Comparative polymerisable mixtures P2.1, P2.2, P2.3, P2.4, P2.5, P2.6, P2.7, P2.8, P2.9, P2.10, P2.11, P2.12, P2.13, P2.14 and P2.15 according to the invention are prepared by adding RM 2 of Comparative example 2 to each of LC mixtures N1-N15, respectively, at a concentration of 0.3% by weight.

Polymerisable mixtures P3.1, P3.2, P3.3, P3.4, P3.5, P3.6, P3.7, P3.8, P3.9, P3.10, P3.11, P3.12, P3.13, P3.14 and P3.15 according to the invention are prepared by adding RM 3 of Example 3 to each of LC mixtures N1-N15, respectively, at a concentration of 0.3% by weight.

Comparative polymerisable mixtures P4.1, P4.2 and P4.3 according to the invention are prepared by adding RM 4 of Comparative example 4 to each of LC mixtures N1-N3, respectively, at a concentration of 0.3% by weight.

Comparative polymerisable mixtures P5.1, P5.2 and P5.3 according to the invention are prepared by adding RM 5 of Comparative example 5 to each of LC mixtures N1-N3, respectively, at a concentration of 0.3% by weight.

The mixture compositions are shown in Table 1 below.

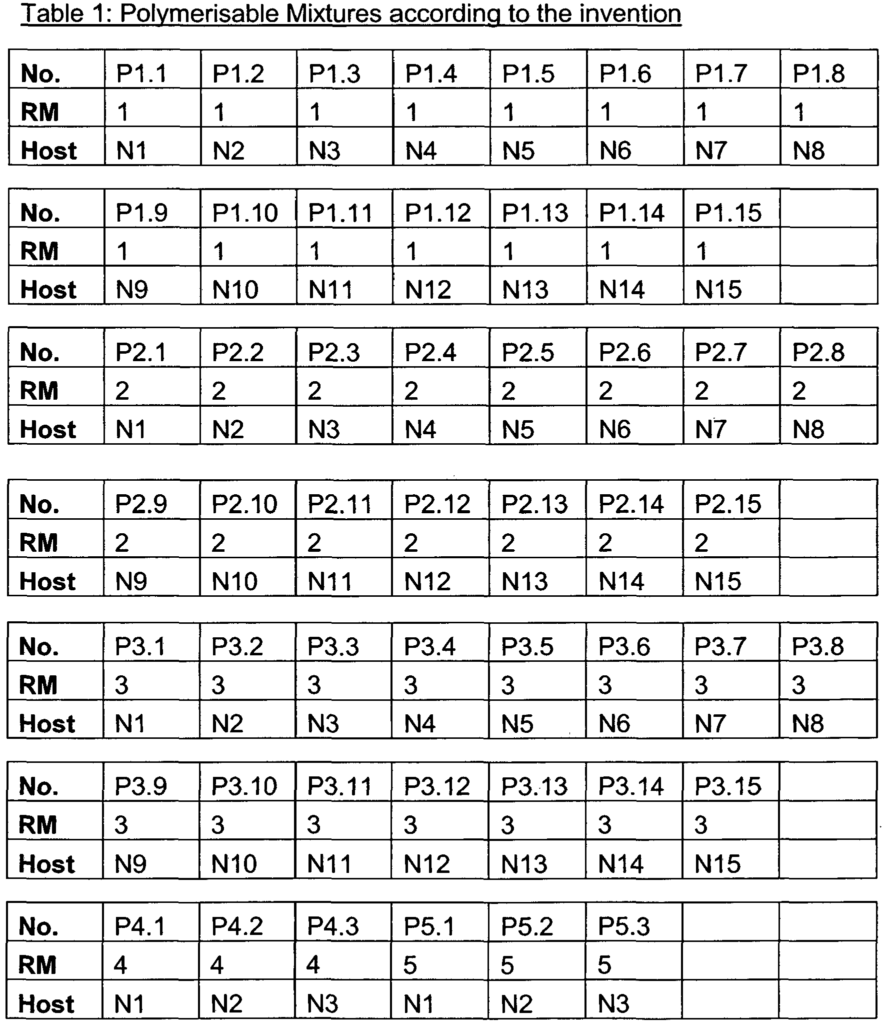

### Comparison Examples C1.1-C2.3

For comparison purposes, polymerisable mixtures C1.1, C1.2 and C1.3 are prepared by adding RM C1 of prior art to each of LC mixtures N1, N2 and N3, respectively, at a concentration of 0.3% by weight, and polymerisable mixtures C2.1, C2.2 and C2.3 are prepared by adding RM C2 of prior art to each of LC mixtures N1, N2 and N3, respectively, at a concentration of 0.3% by weight.

The mixture compositions are shown in Table 2 below.

**Table 2: Polymerisable Comparison Mixtures**

| **No.** | C1.1 | C1.2 | C1.3 | C2.1 | C2.2 | C2.3 | | |
|---|---|---|---|---|---|---|---|---|
| **RM** | C1 | C1 | C1 | C2 | C2 | C2 | | |
| **Host** | N1 | N2 | N3 | N1 | N2 | N3 | | |

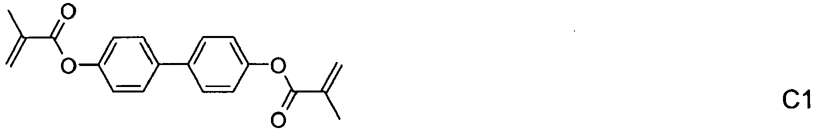

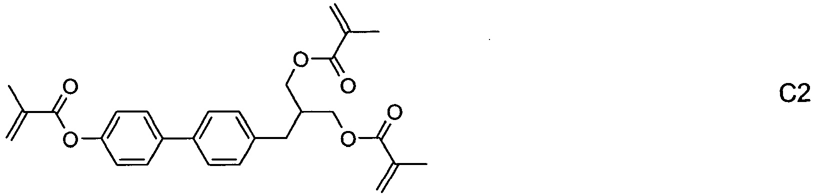

### Use Examples

The polymerisable mixtures according to the invention and the polymerisable comparison mixtures are each inserted into a VA e/o test cell, respectively. The test cells comprise a VA-polyimide alignment layer (JALS-2096-R1) which is rubbed antiparallel (for the test cells with host mixture N3 the polyimide AL64101was used). The LC-layer thickness d is approx. 4 µm. Each test cell is irradiated with UV light having an intensity of 100 mW/cm² for the time indicated with application of a voltage of 24 Vᵣₘₛ (alternating current), which causes polymerisation of the RM.

The VHR values of the polymerisable mixtures before and after UV exposure are measured as described above. "Suntest" means a second irradiation step with lower UV intensity but longer exposure time than the first step.

The residual content of unpolymerised RM (in % by weight) in the test cells is measured by HPLC after various exposure times. For this purpose each mixture is polymerised in the test cell under the conditions stated above. The mixture is then rinsed out of the test cell using MEK (methyl ethyl ketone) and measured.

The tilt angle is measured before and after UV irradiation by a crystal rotation experiment (Autronic-Melchers TBA-105).

The results are shown below.

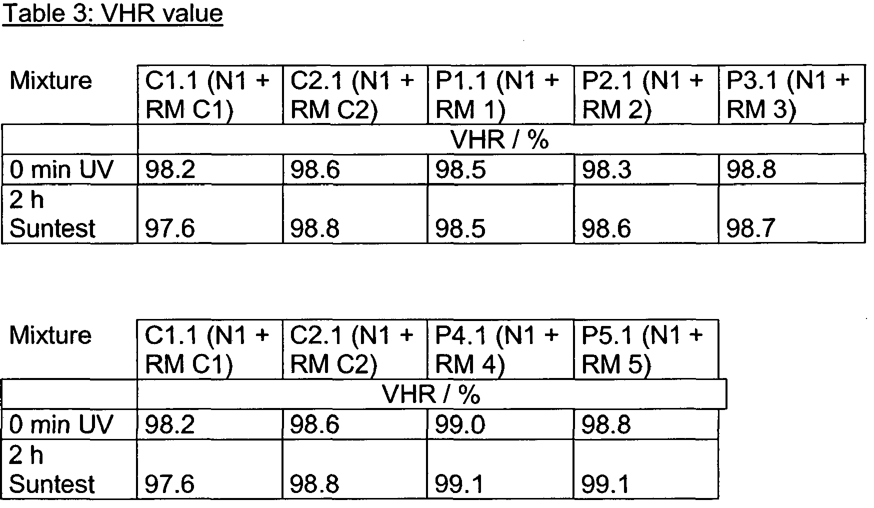

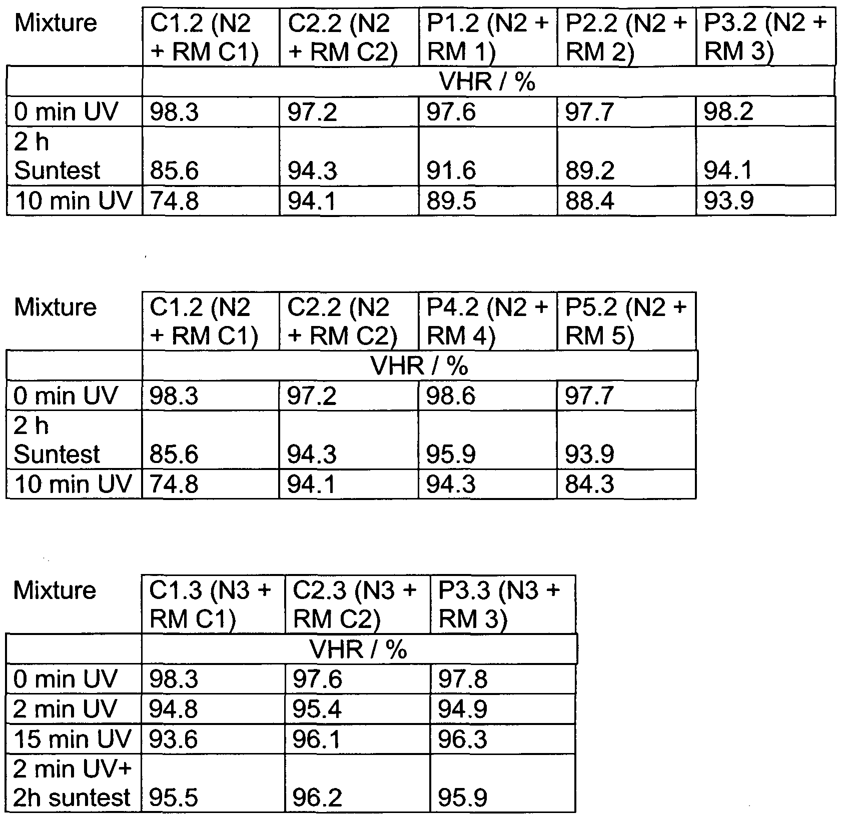

In the host mixture N1 without an alkenyl compound all RMs show high VHR values after suntest. In the host mixture N2 containing an alkenyl compound (CC-3-V) the RMs 1-5 according to the present invention show higher VHR values after suntest compared to the RM C1. Even the VHR values for RM1 and RM2 are kept on a high level, RMs 3-5 have the highest VHR after the UV load and similar or higher values than RM C2 of prior art. In the host mixture N3 containing an alkenyl compound (CC-3-V), the trireactive RMs 3-5 and RM C2 show higher VHR values after suntest than direactive RM C1.

**Table 4: Residual RM content**

| Mixture | C1.1 (N1 + RM C1) | C1.2 (N1 + RM C2) | P1.1 (N1 + RM 1) | P2.1 (N1 + RM 2) | P3.1 (N1 + RM 3) |
|---|---|---|---|---|---|
| Time / min | Residual RM / % | | | | |
| 0 | 0.3 | 0.3 | 0.3 | 0.3 | 0.3 |
| 2 | 0.264 | 0.296 | 0.242 | 0.242 | 0.275 |
| 4 | 0.203 | 0.222 | 0.165 | 0.142 | 0.207 |
| 6 | 0.173 | 0.197 | 0.136 | 0.097 | 0.162 |

| | | | | | |
|---|---|---|---|---|---|
| Mixture | C1.1 (N1 + RM C1) | C1.2 (N1 + RM C2) | P4.1 (N1 + RM 4) | P5.1 (N1 + RM 5) | |
| Time / min | Residual RM / % | | | | |
| 0 | 0.3 | 0.3 | 0.3 | 0.3 | |
| 2 | 0.264 | 0.296 | 0.137 | 0.264 | |
| 4 | 0.203 | 0.222 | 0.033 | 0.157 | |
| 6 | 0.173 | 0.197 | 0.010 | 0.113 | |

| | | | | | |
|---|---|---|---|---|---|
| Mixture | C1.2 (N2 + RM C1) | C2.2 (N2 + RM C2) | P1.2 (N2 + RM 1) | P2.2 (N2 + RM 2) | P3.2 (N2 + RM 3) |
| Time / min | Residual RM / % | | | | |
| 0 | 0.3 | 0.3 | 0.3 | 0.3 | 0.3 |
| 2 | 0.185 | 0.215 | 0.147 | 0.144 | 0.153 |
| 6 | 0.067 | 0.110 | 0.061 | 0.055 | 0.055 |

| | | | | | |
|---|---|---|---|---|---|
| Mixture | C1.2 (N2 + RM C1) | C2.2 (N2 + RM C2) | P4.2 (N2 + RM 4) | P5.2 (N2 + RM 5) | |
| Time / min | Residual RM / % | | | | |
| 0 | 0.3 | 0.3 | 0.3 | 0.3 | |
| 2 | 0.185 | 0.215 | 0.102 | 0.173 | |
| 6 | 0.067 | 0.110 | 0.022 | 0.062 | |

| Mixture | C1.3 (N3 + RM C1) | C2.3 (N3 + RM C2) | P3.3 (N3 + RM 3) | | |
|---|---|---|---|---|---|
| Time / min | Residual RM / % | | | | |
| 0 | 0.3 | 0.3 | 0.3 | | |
| 0.5 | 0.256 | 0.273 | 0.269 | | |
| 1 | 0.199 | 0.205 | 0.189 | | |
| 2 | 0.142 | 0.131 | 0.105 | | |
| 5 | 0.05 | 0.05 | 0.046 | | |

In all host mixtures N1, N2 and N3 the RMs 1-5 according to the present invention show faster polymerisation with a lower residual RM content, compared to the RMs C1 and C2 of prior art.

**Table 5: Pretilt Angle**

| Mixture | C1.1 (N1 + RM C1) | C1.2 (N1 + RM C2) | P1.1 (N1 + RM 1) | P1.2 (N1 + RM 2) | P1.3 (N1 + RM 3) |
|---|---|---|---|---|---|
| UV-Time / sec | Pretilt Angle / ° | | | | |
| 0 | 89.6 | 88.1 | 88.6 | 88.6 | 88.6 |
| 30 | 89.0 | 87.9 | 88.1 | 88.0 | 87.1 |
| 60 | 88.2 | 85.8 | 85.3 | 85.9 | 84.6 |
| 120 | 84.9 | 82.9 | 80.1 | 79.9 | 81.1 |

| | | | | | |
|---|---|---|---|---|---|
| Mixture | C1.1 (N1 + RM C1) | C1.2 (N1 + RM C2) | P4.1 (N1 + RM 4) | P5.1 (N1 RM 5) + | |
| UV-Time / sec | Pretilt Angle / ° | | | | |
| 0 | 89.6 | 88.1 | 89.3 | 88.3 | |
| 30 | 89.0 | 87.9 | 88.1 | 87.9 | |
| 60 | 88.2 | 85.8 | 86.5 | 84.6 | |
| 120 | 84.9 | 82.9 | 80.5 | 79.6 | |

| | | | | | |
|---|---|---|---|---|---|
| Mixture | C1.2 (N2 + RM C1) | C2.2 (N2 + RM C2) | P1.2 (N2 + RM 1) | P2.2 (N2 + RM 2) | P3.2 (N2 + RM 3) |
| UV-Time / sec | Pretilt Angle / ° | | | | |
| 0 | 88.8 | 88.2 | 88.6 | 88.8 | 88.9 |
| 120 | 77.2 | 77.6 | 77.3 | 76.3 | 77.7 |

| Mixture | C1.2 (N2 + RM C1) | C2.2 (N2 + RM C2) | P4.2 (N2 + RM 4) | P5.2 (N2 + RM 5) | |
|---|---|---|---|---|---|
| UV-Time / sec | Pretilt Angle / ° | | | | |
| 0 | 88.8 | 88.2 | 89.2 | 88.8 | |
| 120 | 77.2 | 77.6 | 78.4 | 76.7 | |

| | | | | | |
|---|---|---|---|---|---|
| Mixture | C1.3 (N3 + RM C1) | C2.3 (N3 + RM C2) | P3.3 (N3 + RM 3) | | |
| UV-Time / sec | Pretilt Angle / ° | | | | |
| 0 | 89.1 | 89.1 | 89.0 | | |
| 60 | 86.4 | 84.1 | 76.5 | | |
| 120 | 78.0 | 79.0 | 70.8 | | |
| 180 | 76.1 | 76.8 | 66.0 | | |
| 300 | 73.5 | 74.3 | 66.1 | | |

The tilt angle measurements confirm the results of the residual RM content measurements. Thus, in all host mixtures N1, N2 and N3 the RM1 and RM3 according to the present invention show faster generation of a higher tilt angle compared to the RMs C1 and C2 of prior art.

For the tilt stability investigation the tilt generation has been performed in a 2-step process (2 min UV light, 100 mW/cm² + 2 h suntest). Then the testcells are subjected to a voltage of 10 V_{RMS} (1 kHz) for a time of 7 days at 40°C. After a relaxation time of 10-20 min the tilt angle after stress was measured.

The pretilt angle values and the change of the pretilt angle after the stress for the individual samples are summarized in Table 6.

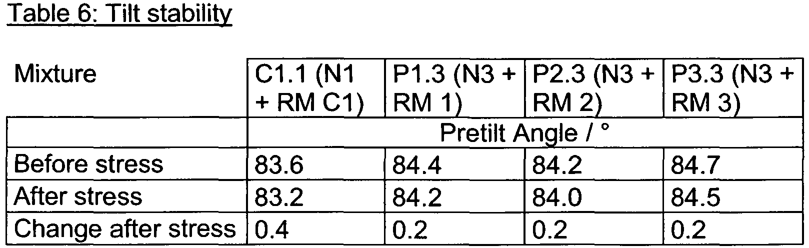

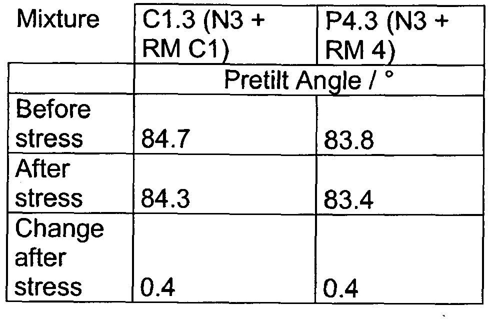

The change of the pretilt after stress is smaller with the RM1 and RM3 according to the invention, compared to the RM C1. Also RM1 and RM3 according to the invention show a better tilt stability than RM C1.

## Claims

1. A compound of formula I
P¹-Sp¹-(A¹-Z¹)ₙ-A²-O-Sp⁴-CH(Sp²-P²)(Sp³-P³) I
in which the individual radicals have the following meanings:
P¹, P², P³ independently of each other denote a vinyloxy, acrylate, methacrylate, fluoroacrylate, chloroacrylate, oxetane or epoxide group,
Sp¹ is a single bond, or Sp¹ is -(CH₂)ₚ₁- or -(CH₂)ₚ₁-O-, in which p1 is 1, 2 or 3, Sp² and Sp³ denote -(CH₂)ₚ₂-, in which p2 is 1, 2 or 3, and Sp⁴ is -(CH₂)ₚ₄-, in which p4 is 1, 2 or 3,
A¹, A² independently of each other, and on each occurrence identically or differently, denote an aromatic, heteroaromatic, alicyclic or heterocyclic group having 4 to 25 C atoms, which may also contain fused rings, and which is optionally mono- or polysubstituted by L,
Z¹ denotes, on each occurrence identically or differently, -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -CR⁰⁰R⁰⁰⁰-, or a single bond,
L denotes P¹-, P¹-Sp¹-, F, Cl, Br, I, -CN, -NO₂, -NCO, -NCS,-OCN, -SCN, -C(=O)N(R^{x})₂, -C(=O)Y¹, -C(=O)R^{x}, -N(R^{x})₂, optionally substituted silyl, optionally substituted aryl or heteroaryl having 5 to 20 ring atoms, or straight-chain or branched alkyl having 1 to 25, particularly preferably 1 to 10, , C atoms, in which, in addition, one or more non-adjacent CH₂ groups may each be replaced, independently of one another, by -C(R⁰⁰)=C(R⁰⁰⁰)-, -C≡C-, -N(R⁰⁰)-, -O-, -S-, -CO-, -CO-O--O-CO-, -O-CO-O- in such a way that O and/or S atoms are not linked directly to one another, and in which, in addition, one or more H atoms may be replaced by F, Cl, CN, P¹- or P¹-Sp¹-,
R⁰⁰ and R⁰⁰⁰ each, independently of one another, denote H or alkyl having 1 to 12 C atoms,
Y¹ is halogen,
R^{x} denotes P¹-, P¹-Sp¹-, H, halogen, straight chain, branched or cyclic alkyl having 1 to 25 C atoms, wherein one or more non-adjacent CH₂-groups are optionally replaced by -O-, -S-, -CO-, -CO-O-, -O-CO-, -O-CO-O- in such a manner that O- and/or S-atoms are not directly connected with each other, and wherein one or more H atoms are optionally replaced by F, Cl, P¹- or P¹-Sp¹-, optionally substituted aryl, aryloxy, heteroaryl or heteroaryloxy having 5 to 20 ring atoms,
n is 1, 2, 3 or 4.

2. The compound of Claim 1, **characterized in that** A¹, A² each, independently of one another, denote 1,4-phenylene, naphthalene-1,4-diyl or naphthalene-2,6-diyl, where one or more CH groups in these groups are optionally replaced by N, cyclohexane-1,4-diyl, in which, in addition, one or more non-adjacent CH₂ groups are optionally replaced by O and/or S, 1,4-cyclohexenylene, bicyclo[1.1.1]pentane-1,3-diyl, bicyclo[2.2.2]octane-1,4-diyl, spiro[3.3]heptane-2,6-diyl, piperidine-1,4-diyl, decahydronaphthalene-2,6-diyl, 1,2,3,4-tetrahydronaphthalene-2,6-diyl, indane-2,5-diyl, octahydro-4,7-methanoindane-2,5-diyl, anthracene-2,7-diyl, fluorene-2,7-diyl, phenanthrene-2,7-diyl or 9,10-dihydro-phenanthrene-2,7-diyl, where all these groups are unsubstituted or mono- or polysubstituted by L as defined in claim 1.

3. The compound of Claim 1 or 2, **characterized in that** it is selected from the group consisting of the following sub-formulae: 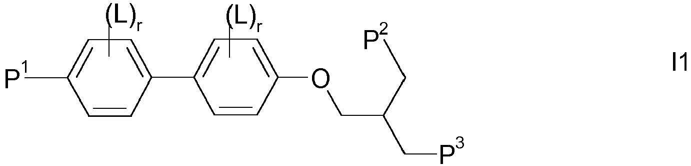 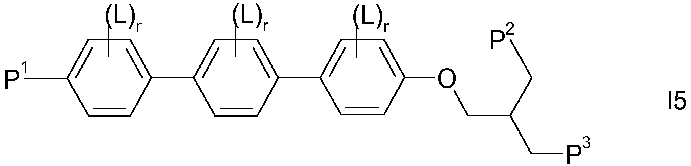 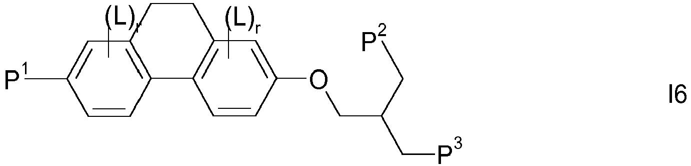 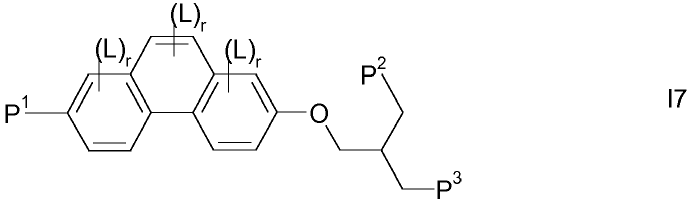 wherein P¹, P², P³ and L are as defined in claim 1 and r is 0, 1, 2, 3 or 4.

4. A liquid crystal (LC) medium comprising one or more polymerisable compounds formula I as defined in any one of Claims 1 to 3.

5. The LC medium of Claim 4, **characterized in that** it comprises
- a polymerisable component A) comprising one or more polymerisable compounds of formula I as defined in any one or Claims 1 to 3, and
- a liquid-crystalline LC component B) comprising one or more mesogenic or liquid-crystalline compounds.

6. The LC medium of Claim 4 or 5, **characterized in that** it comprises one or more compounds of the formulae CY and/or PY: 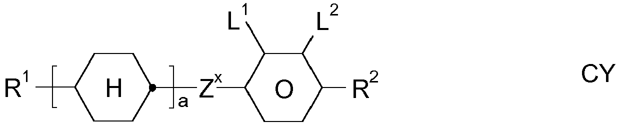 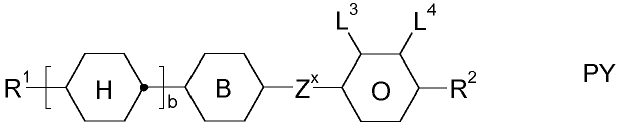 in which the individual radicals have the following meanings:
a denotes 1 or 2,
b denotes 0 or 1, 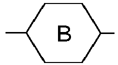 denotes 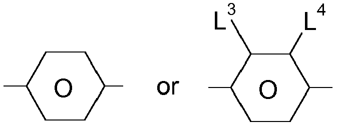
R¹ and R² each, independently of one another, denote alkyl having 1 to 12 C atoms, where, in addition, one or two non-adjacent CH₂ groups may be replaced by -O-, -CH=CH-, -CO-, -O-CO- or-CO-O- in such a way that O atoms are not linked directly to one another,
Z^{x} denotes -CH=CH-, -CH₂O-, -OCH₂-, -CF₂O-, -OCF₂-, -O-,-CH₂-, -CH₂CH₂- or a single bond, preferably a single bond,
L¹⁻⁴ each, independently of one another, denote F, Cl, OCF₃, CF₃, CH₃, CH₂F, CHF₂.

7. The LC medium of any one of Claims 5 to 6, **characterized in that** it comprises one or more compounds selected from the following formulae: 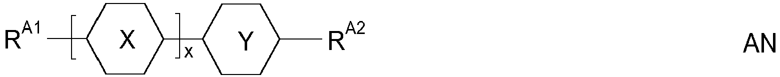 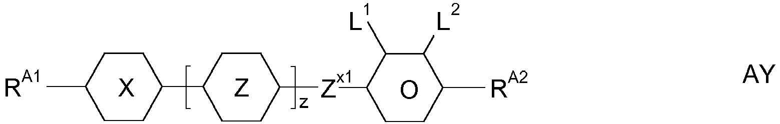 in which the individual radicals, on each occurrence identically or differently, each, independently of one another, have the following meaning: 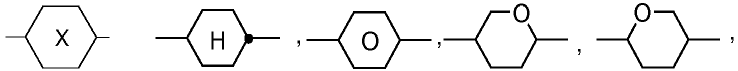 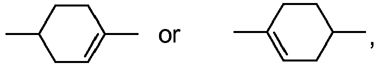 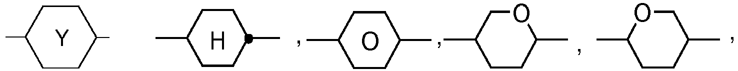 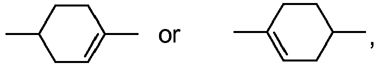 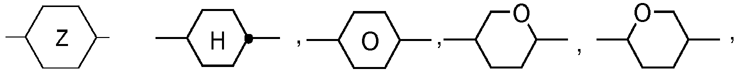 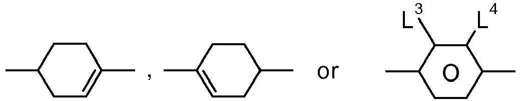
R^{A1} alkenyl having 2 to 9 C atoms or, if at least one of the rings X, Y and Z denotes cyclohexenyl, also one of the meanings of R^{A2},
R^{A2} alkyl having 1 to 12 C atoms, in which, in addition, one or two non-adjacent CH₂ groups may be replaced by -O-, -CH=CH-, -CO-, -OCO- or -COO- in such a way that O atoms are not linked directly to one another,
Z^{x1} -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, or a single bond, preferably a single bond,
L¹⁻⁴ each, independently of one another, H, F, Cl, OCF₃, CF₃, CH₃, CH₂F or CHF₂H, preferably H, F or Cl,
x 1 or 2,
z 0 or 1.

8. The LC medium of any one of Claims 4 to 7, **characterized in that** it comprises one or more compounds of the following formula: 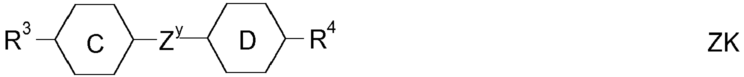 in which the individual radicals have the following meanings: 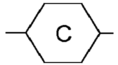 denotes 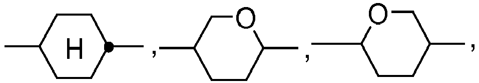 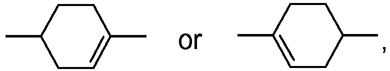 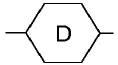 denotes 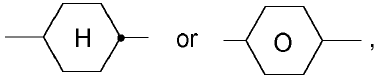
R³ and R⁴ each, independently of one another, denote alkyl having 1 to 12 C atoms, in which, in addition, one or two non-adjacent CH₂ groups may be replaced by -O-, -CH=CH-, -CO-, -O-CO- or -CO-O- in such a way that O atoms are not linked directly to one another,
Z^{y} denotes -CH₂CH₂-, -CH=CH-, -CF₂O-, -OCF₂-, -CH₂O-, -O CH₂-, -COO-, -OCO-, -C₂F₄-, -CF=CF- or a single bond.

9. The LC medium of any one of Claims 4 to 8, **characterized in that** the polymerisable compounds of formula I are polymerised.

10. An LC display comprising one or more compounds of formula I as defined in one or more of Claims 1 to 3 or comprising an LC medium as defined in any one of Claims 4 to 9.

11. The LC display of Claim 10, which is a PSA type display.

12. The LC display of Claim 11, which is a PS-VA, PS-OCB, PS-IPS, PS-FFS, PS-UB-FFS, PS-posi-VA or PS-TN display.

13. The LC display of Claim 11 or 12, **characterized in that** it comprises two substrates, at least one which is transparent to light, an electrode provided on each substrate or two electrodes provided on only one of the substrates, and located between the substrates a layer of an LC medium, comprising one or more polymerisable compounds, as defined in any one of Claims 4 to 9, wherein the polymerisable compounds are polymerised between the substrates of the display.

14. A process for the production of an LC display according to Claim 13, comprising the steps of filling or otherwise providing an LC medium, comprising one or more polymerisable compounds, as defined in any one of Claims 4 to 9, between the substrates of the display, and polymerising the polymerisable compounds.

15. A process of preparing an LC medium of any one of Claims 4 to 9, comprising the steps of mixing one or more mesogenic or liquid-crystalline compounds, or a liquid-crystalline component B) as defined in Claim 5, with one or more compounds of formula I as defined in one or more of Claims 1 to 3, and optionally with further liquid-crystalline compounds and/or additives.

## Patentansprüche

1. Verbindung der Formel I
P¹-Sp¹-(A¹-Z¹)ₙ-A²-O-Sp⁴-CH(Sp²-P²)(Sp³-P³) I
in der die einzelnen Reste die folgenden Bedeutungen besitzen:
P¹, P², P³ bedeuten unabhängig voneinander eine Vinyloxy-, Acrylat-, Methacrylat-, Fluoracrylat-, Chloracrylat-, Oxetan- oder Epoxidgruppe,
Sp¹ ist eine Einfachbindung, oder Sp¹ ist -(CH₂)ₚ₁ oder -(CH₂)ₚ₁-O-, in denen p1 1, 2 oder 3 ist, Sp² und Sp³ bedeuten -(CH₂)ₚ₂-, in dem p2 1, 2 oder 3 ist, und Sp⁴ ist -(CH₂)ₚ₄-, in dem p4 1, 2 oder 3 ist,
A¹, A² bedeuten unabhängig voneinander und bei jedem Auftreten gleich oder verschieden eine aromatische, heteroaromatische, alicyclische oder heterocyclische Gruppe mit 4 bis 25 C-Atomen, die auch anellierte Ringe enthalten kann und die gegebenenfalls ein- oder mehrfach durch L substituiert ist,
Z¹ bedeutet bei jedem Auftreten gleich oder verschieden -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -CR⁰⁰R⁰⁰⁰- oder eine Einfachbindung,
L bedeutet P¹-, P¹-Sp¹-, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, gegebenenfalls substituiertes Silyl, gegebenenfalls substituiertes Aryl oder Heteroaryl mit 5 bis 20 Ringatomen oder geradkettiges oder verzweigtes Alkyl mit 1 bis 25, besonders bevorzugt 1 bis 10 C-Atomen, in dem zusätzlich eine oder mehrere nicht benachbarte CH₂-Gruppen jeweils unabhängig voneinander so durch -C(R⁰⁰)=C(R⁰⁰⁰)-, -C≡C-, -N(R⁰⁰)-, -O-, -S-, -CO-, -CO-O-, -O-CO-, -O-CO-O- ersetzt sein können, dass O- und/oder S-Atome nicht direkt miteinander verknüpft sind, und in dem zusätzlich ein oder mehrere H-Atome durch F, Cl, CN, P¹- oder P¹-Sp¹- ersetzt sein können,
R⁰⁰ und R⁰⁰⁰ bedeuten jeweils unabhängig voneinander H oder Alkyl mit 1 bis 12 C-Atomen,
Y¹ ist Halogen,
R^{x} bedeutet P¹-, P¹-Sp¹-, H, Halogen, geradkettiges, verzweigtes oder cyclisches Alkyl mit 1 bis 25 C-Atomen, bei dem gegebenenfalls eine oder mehrere nicht benachbarte CH₂-Gruppen so durch -O-, -S-, -CO-, -CO-O-, -O-CO-, -O-CO-O-ersetzt sind, dass O- und/oder S-Atome nicht direkt miteinander verbunden sind, und bei dem gegebenenfalls ein oder mehrere H-Atome durch F, Cl, P¹- oder P¹-Sp¹- ersetzt sind, gegebenenfalls substituiertes Aryl, Aryloxy, Heteroaryl oder Heteroaryloxy mit 5 bis 20 Ringatomen,
n ist 1, 2, 3 oder 4.

2. Verbindung des Anspruchs 1, **dadurch gekennzeichnet, dass** A¹, A² jeweils unabhängig voneinander 1,4-Phenylen, Naphthalin-1,4-diyl oder Naphthalin-2,6-diyl, wobei eine oder mehrere CH-Gruppen in diesen Gruppen gegebenenfalls durch N ersetzt sind, Cyclohexan-1,4-diyl, in dem zusätzlich eine oder mehrere nicht benachbarte CH₂-Gruppen gegebenenfalls durch O und/oder S ersetzt sind, 1,4-Cyclohexenylen, Bicyclo[1.1.1]pentan-1,3-diyl, Bicyclo[2.2.2]octan-1,4-diyl, Spiro[3.3]-heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, Anthracen-2,7-diyl, Fluoren-2,7-diyl, Phenanthren-2,7-diyl oder 9,10-Dihydro-phenanthren-2,7-diyl bedeuten, wobei alle diese Gruppen unsubstituiert oder ein- oder mehrfach durch L wie in Anspruch 1 definiert substituiert sind.

3. Verbindung nach Anspruch 1 oder 2, **dadurch gekennzeichnet, dass** sie aus der Gruppe bestehend aus den folgenden Unterformeln ausgewählt ist: 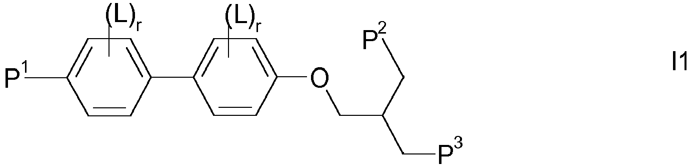 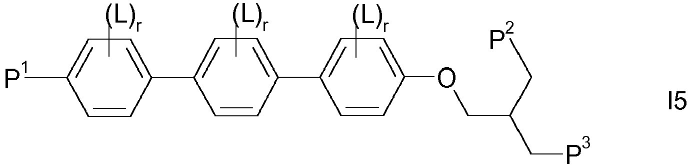 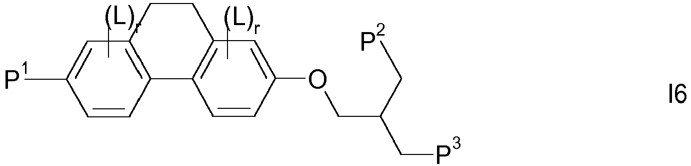 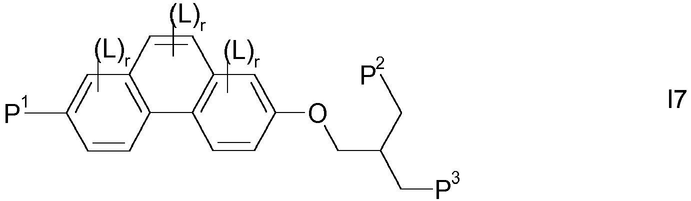 bei denen P¹, P², P³ und L wie in Anspruch 1 definiert sind und r 0, 1, 2, 3 oder 4 ist.

4. Flüssigkristall(FK)-Medium enthaltend eine oder mehrere polymerisierbare Verbindungen der Formel I wie in einem beliebigen der Ansprüche 1 bis 3 definiert.

5. FK-Medium des Anspruchs 4, **dadurch gekennzeichnet, dass** es
- eine polymerisierbare Komponente A) enthaltend eine oder mehrere polymerisierbare Verbindungen der Formel I wie in einem beliebigen der Ansprüche 1 bis 3 definiert und
- eine flüssigkristalline(FK)-Komponente B) enthaltend eine oder mehrere mesogene oder flüssigkristalline Verbindungen enthält.

6. FK-Medium des Anspruchs 4 oder 5, **dadurch gekennzeichnet, dass** es eine oder mehrere Verbindungen der Formeln CY und/oder PY enthält: 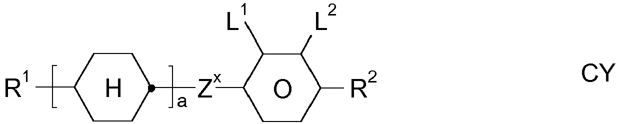 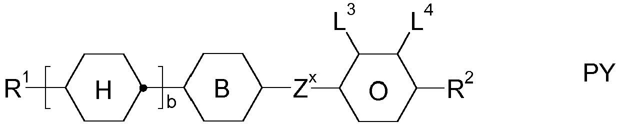 in denen die einzelnen Reste die folgenden Bedeutungen besitzen:
a bedeutet 1 oder 2,
b bedeutet 0 oder 1, 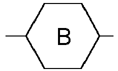 bedeutet 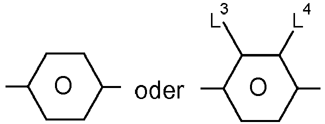
R¹ und R² bedeuten jeweils unabhängig voneinander Alkyl mit 1 bis 12 C-Atomen, wobei zusätzlich eine oder zwei nicht benachbarte CH₂-Gruppen so durch -O-, -CH=CH-, -CO-, -O-CO-oder -CO-O- ersetzt sein können, dass O-Atome nicht direkt miteinander verknüpft sind,
Z^{x} bedeutet -CH=CH-, -CH₂O-, -OCH₂-, -CF₂O-, -OCF₂-, -O-, -CH₂-, -CH₂CH₂- oder eine Einfachbindung, vorzugsweise eine Einfachbindung,
L¹⁻⁴ bedeuten jeweils unabhängig voneinander F, Cl, OCF₃, CF₃, CH₃, CH₂F, CHF₂.

7. FK-Medium nach einem beliebigen der Ansprüche 5 bis 6, **dadurch gekennzeichnet, dass** es eine oder mehrere Verbindungen enthält, die aus den folgenden Formeln ausgewählt sind: 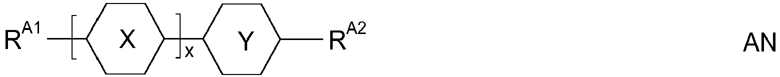 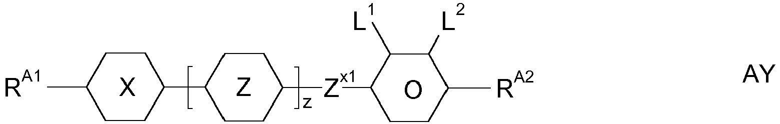 in denen die einzelnen Reste bei jedem Auftreten gleich oder verschieden und jeweils unabhängig voneinander die folgende Bedeutung besitzen: 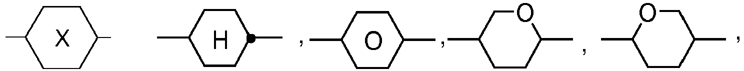 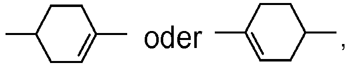 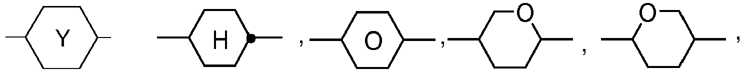 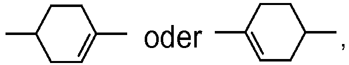 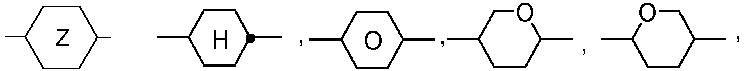 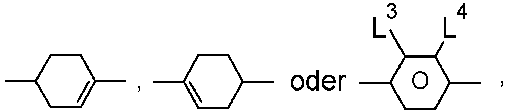
R^{A1} Alkenyl mit 2 bis 9 C-Atomen oder, wenn mindestens einer der Ringe X, Y und Z Cyclohexenyl bedeutet, auch eine der Bedeutungen von R^{A2}
R^{A2} Alkyl mit 1 bis 12 C-Atomen, in dem zusätzlich eine oder zwei nicht benachbarte CH₂-Gruppen so durch -O-, -CH=CH-, -CO-, -OCO- oder -COO- ersetzt sein können, dass O-Atome nicht direkt miteinander verknüpft sind,
Z^{x1} -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O- oder eine Einfachbindung, vorzugsweise eine Einfachbindung,
L¹⁻⁴ jeweils unabhängig voneinander H, F, Cl, OCF₃, CF₃, CH₃, CH₂F oder CF₂H, vorzugsweise H, F oder Cl,
x 1 oder 2,
z 0 oder 1.

8. FK-Medium nach einem beliebigen der Ansprüche 4 bis 7, **dadurch gekennzeichnet, dass** es eine oder mehrere Verbindungen der folgenden Formel enthält: 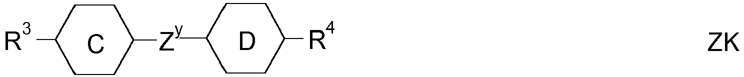 in der die einzelnen Reste die folgenden Bedeutungen besitzen: 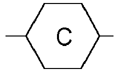 bedeutet 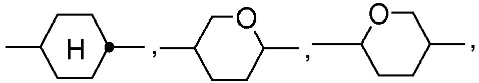 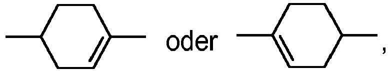 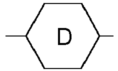 bedeutet 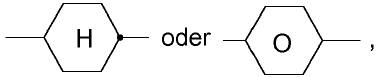
R³ und R⁴ bedeuten jeweils unabhängig voneinander Alkyl mit 1 bis 12 C-Atomen, in dem zusätzlich eine oder zwei nicht benachbarte CH₂-Gruppen so durch -O-, -CH=CH-, -CO-, -O-CO-oder -CO-O- ersetzt sein können, dass O-Atome nicht direkt miteinander verknüpft sind,
Z^{y} bedeutet -CH₂CH₂-, -CH=CH-, -CF₂O-, -OCF₂-, -CH₂O-, -OCH₂-, -COO-, -OCO-, -C₂F₄-, -CF=CF- oder eine Einfachbindung.

9. FK-Medium nach einem beliebigen der Ansprüche 4 bis 8, **dadurch gekennzeichnet, dass** die polymerisierbaren Verbindungen der Formel I polymerisiert werden.

10. FK-Anzeige enthaltend eine oder mehrere Verbindungen der Formel I wie in einem oder mehreren der Ansprüche 1 bis 3 definiert oder enthaltend ein FK-Medium wie in einem beliebigen der Ansprüche 4 bis 9 definiert.

11. FK-Anzeige des Anspruchs 10, bei der es sich um eine Anzeige des PSA-Typs handelt.

12. FK-Anzeige des Anspruchs 11, bei der es sich um eine PS-VA-, PS-OCB-, PS-IPS-, PS-FFS-, PS-UB-FFS-, PS-posi-VA- oder PS-TN-Anzeige handelt.

13. FK-Anzeige des Anspruchs 11 oder 12, **dadurch gekennzeichnet, dass** sie zwei Substrate, von denen mindestens eines lichtdurchlässig ist, eine auf jedem Substrat vorgesehene Elektrode oder zwei auf nur einem der Substrate vorgesehene Elektroden und zwischen den Substraten angeordnet eine Schicht eines FK-Mediums enthaltend eine oder mehrere polymerisierbare Verbindungen wie in einem beliebigen der Ansprüche 4 bis 9 definiert enthält, bei der die polymerisierbaren Verbindungen zwischen den Substraten der Anzeige polymerisiert werden.

14. Verfahren zur Herstellung einer FK-Anzeige nach Anspruch 13, umfassend die Schritte des Einfüllens oder anderweitigen Bereitstellens eines FK-Mediums enthaltend eine oder mehrere polymerisierbare Verbindungen wie in einem beliebigen der Ansprüche 4 bis 9 definiert, zwischen den Substraten der Anzeige und Polymerisieren der polymerisierbaren Verbindungen.

15. Verfahren zur Herstellung eines FK-Mediums nach einem beliebigen der Ansprüche 4 bis 9, umfassend die Schritte Mischen einer oder mehrerer mesogener oder flüssigkristalliner Verbindungen oder einer flüssigkristallinen Komponente B) wie in Anspruch 5 definiert mit einer oder mehreren Verbindungen der Formel I wie in einem oder mehreren der Ansprüche 1 bis 3 definiert und gegebenenfalls mit weiteren flüssigkristallinen Verbindungen und/oder Zusatzstoffen.

## Revendications

1. Composé de la formule I :
P¹-Sp¹-(A¹-Z¹)ₙ-A²-Sp⁴-O-CH(Sp²-P²)(Sp³-P³) I
dans laquelle les radicaux individuels présentent les significations qui suivent :
P¹, P², P³ représentent, de manière indépendante les uns des autres, un groupe vinyloxy, acrylate, méthacrylate, fluoroacrylate, chloroacrylate, oxétane ou époxyde ;
Sp¹ est une liaison simple, ou Sp¹ est -(CH₂)ₚ₁- ou -(CH₂)ₚ₁-O-, où p1 est 1, 2 ou 3, Sp² et Sp³ représentent -(CH₂)ₚ₂-, où p2 est 1, 2 ou 3, et Sp⁴ est -(CH₂)ₚ₄-, où p4 est 1, 2 ou 3 ;
A¹, A² représentent, de manière indépendante l'un de l'autre et pour chaque occurrence, de manière identique ou différente, un groupe aromatique, hétéroaromatique, alicyclique ou hétérocyclique qui comporte de 4 à 25 atomes de C, lequel peut également contenir des cycles fusionnés, et lequel est en option mono- ou polysubstitué par L ;
Z¹ représente, pour chaque occurrence de manière identique ou différente, -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -CR⁰⁰R⁰⁰⁰-, ou une liaison simple ;
L représente P¹⁻, P¹⁻Sp¹⁻, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, silyle en option substitué, aryle ou hétéroaryle en option substitué qui comporte de 5 à 20 atomes de cycle, ou alkyle en chaîne droite ou ramifié qui comporte de 1 à 25 atome(s) de C, de façon particulièrement préférable de 1 à 10 atome(s) de C, où, en outre, un ou plusieurs groupe(s) CH₂ non adjacents peut/peuvent chacun être remplacé(s), de manière indépendante les uns des autres, par -C(R⁰⁰)=C(R⁰⁰⁰)-, -C≡C-, -N(R⁰⁰)-, -O-, -S-, -CO-, -CO-O-, -O-CO-, -O-CO-O- de telle sorte que des atomes de O et/ou des atomes de S ne soient pas liés directement les uns aux autres, et où, en outre, un ou plusieurs atome(s) de H peut/ peuvent être remplacé(s) par F, Cl, CN, P¹- ou P¹-Sp¹- ;
R⁰⁰ et R⁰⁰⁰ représentent chacun, de manière indépendante l'un de l'autre, H ou alkyle qui comporte de 1 à 12 atome(s) de C ;
Y¹ est halogène ;
R^{x} représente P¹-, P¹⁻Sp¹⁻, H, halogène, alkyle en chaîne droite, ramifié ou cyclique qui comporte de 1 à 25 atome(s) de C, où un ou plusieurs groupe(s) CH₂ non adjacents est/sont en option remplacé(s) par -O-, -S-, -CO-, -CO-O-, -O-CO-, -O-CO-O- de telle sorte que des atomes de O et/ou des atomes de S ne soient pas directement connectés les uns aux autres, et où un ou plusieurs atome(s) de H est/sont en option remplacé(s) par F, Cl, P¹- ou P¹⁻Sp¹⁻, aryle, aryloxy, hétéroaryle ou hétéroaryloxy en option substitué qui comporte de 5 à 20 atomes de cycle ;
n est 1, 2, 3 ou 4.

2. Composé selon la revendication 1, **caractérisé en ce que** A¹, A² représentent chacun, de manière indépendante l'un de l'autre, 1,4-phénylène, naphtalène-1,4-diyle ou naphtalène-2,6-diyle, où un ou plusieurs groupe(s) CH dans ces groupes est/sont remplacé(s) par N, cyclohexane-1,4-diyle, où, en outre, un ou plusieurs groupe(s) CH₂ non adjacents est/sont en option remplacé(s) par O et/ou S, 1,4-cyclohexénylène, bicyclo[1.1.1]pentane-1,3-diyle, bicyclo[2.2.2]octane-1,4-diyle, spiro[3.3]heptane-2,6-diyle, pipéridine-1,4-diyle, décahydronaphtalène-2,6-diyle, 1,2,3,4-tétrahydronaphtalène-2,6-diyle, indane-2,5-diyle, octahydro-4,7-méthanoindane-2,5-diyle, anthracène-2,7-diyle, fluorène-2,7-diyle, phénanthrène-2,7-diyle ou 9,10-dihydrophénanthrène-2,7-diyle, où tous ces groupes sont non substitués ou mono- ou polysubstitués par L, tel que défini selon la revendication 1.

3. Composé selon la revendication 1 ou 2, **caractérisé en ce qu'**il est sélectionné parmi le groupe qui est constitué par les sous-formules qui suivent : 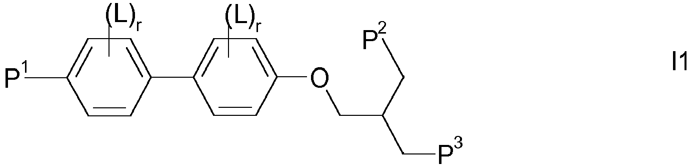 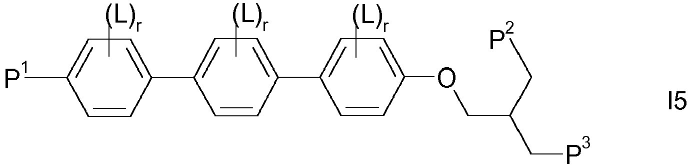 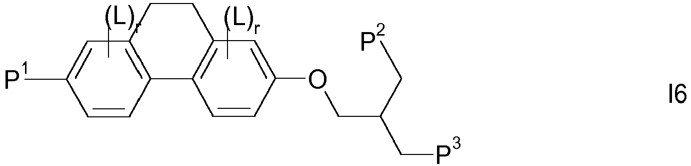 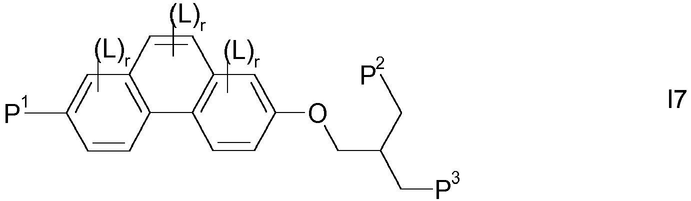 dans lesquelles P¹, P², P³ et L sont tels que définis selon la revendication 1 et r est 0, 1, 2, 3 ou 4.

4. Milieu cristallin liquide (LC) comprenant un ou plusieurs composé(s) polymérisable(s) de la formule I tels que définis selon l'une quelconque des revendications 1 à 3.

5. Milieu LC selon la revendication 4, **caractérisé en ce qu'**il comprend :
- un composant polymérisable A) qui comprend un ou plusieurs composé(s) polymérisable(s) de la formule I tels que définis selon l'une quelconque des revendications 1 à 3 ; et
- un composant cristallin liquide (LC) B) qui comprend un ou plusieurs composé(s) mésogène(s) ou cristallin(s) liquide(s).

6. Milieu LC selon la revendication 4 ou 5, **caractérisé en ce qu'**il comprend un ou plusieurs composé(s) des formules CY et/ou PY : 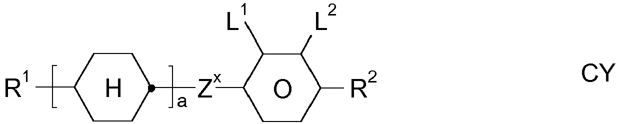 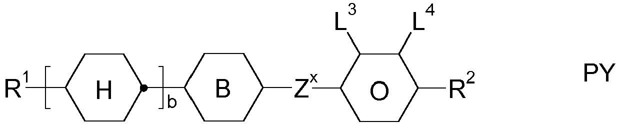 dans lesquelles les radicaux individuels présentent les significations qui suivent :
a représente 1 ou 2 ;
b représente 0 ou 1 ; 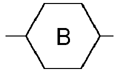 représente 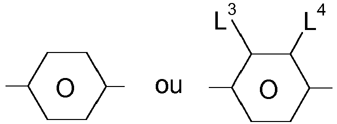
R¹ et R² représentent chacun, de manière indépendante l'un de l'autre, alkyle qui comporte de 1 à 12 atome(s) de C, où, en outre, un ou deux groupe(s) CH₂ non adjacents peut/peuvent être remplacé(s) par -O-, -CH=CH-, -CO-, -O-CO- ou -CO-O- de telle sorte que des atomes de O ne soient pas liés directement les uns aux autres ;
Z^{x} représente -CH=CH-, -CH₂O-, -OCH₂-, -CF₂O-, -OCF₂-, -O-, -CH₂-, -CH₂CH₂- ou une liaison simple, de préférence une liaison simple ;
L¹⁻⁴ représentent chacun, de manière indépendante les uns des autres, F, Cl, OCF₃, CF₃, CH₃, CH₂F, CHF₂.

7. Milieu LC selon l'une quelconque des revendications 5 et 6, **caractérisé en ce qu'**il comprend un ou plusieurs composé(s) qui est/sont sélectionné(s) parmi les formules qui suivent : 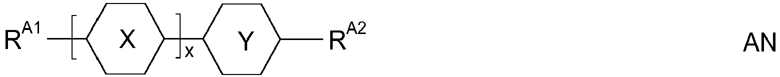 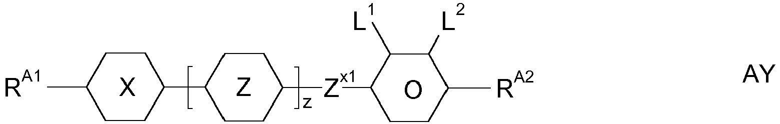 dans lesquelles les radicaux individuels présentent, pour chaque occurrence de manière identique ou différente, chacun de manière indépendante des autres, la signification qui suit :, 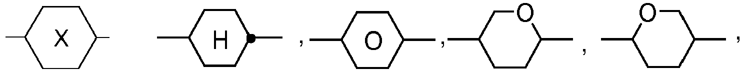 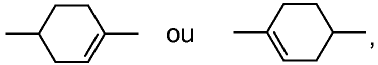 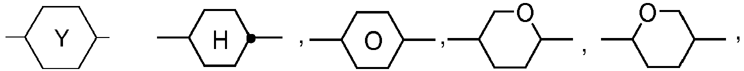 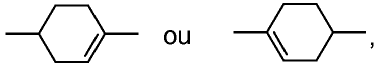 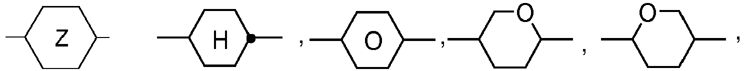 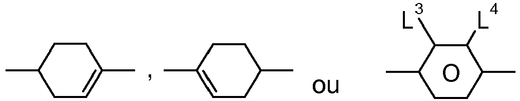
R^{A1} alkényle qui comporte de 2 à 9 atomes de C ou, si au moins l'un des cycles X, Y et Z représente cyclohexényle, également l'une des significations de R^{d} ;
R^{A2} alkyle qui comporte de 1 à 12 atome(s) de C, où, en outre, un ou deux groupe(s) CH₂ non adjacents peut/peuvent être remplacé(s) par -O-, -CH=CH-, -CO-, -OCO- ou -COO- de telle sorte que des atomes de O ne soient pas liés directement les uns aux autres ;
Z^{x1} -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, ou une liaison simple, de préférence une liaison simple ;
L¹⁻⁴ représentent chacun, de manière indépendante les uns des autres, H, F, Cl, OCF₃, CF₃, CH₃, CH₂F ou CF₂H, de préférence H, F ou Cl ;
x 1 ou 2 ;
z 0 ou 1.

8. Milieu LC selon l'une quelconque des revendications 4 à 7, **caractérisé en ce qu'**il comprend un ou plusieurs composé(s) de la formule qui suit : 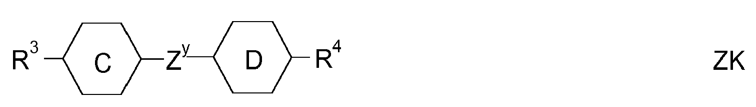 dans laquelle les radicaux individuels présentent les significations qui suivent : 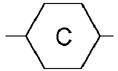 représente 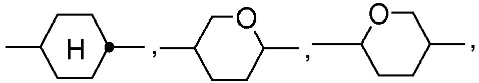 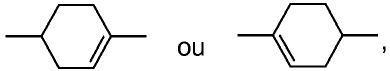 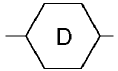 représente 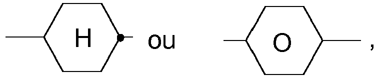
R³ et R⁴ représentent chacun, de manière indépendante l'un de l'autre, alkyle qui comporte de 1 à 12 atome(s) de C, où, en outre, un ou deux groupe(s) CH₂ non adjacents peut/peuvent être remplacé(s) par -O-, -CH=CH-, -CO-, -O-CO- ou -CO-O- de telle sorte que des atomes de O ne soient pas liés directement les uns aux autres ;
Z^{y} représente -CH₂CH₂-, -CH=CH-, -CF₂O-, -OCF₂-, -CH₂O-, -OCH₂-, -COO-, -OCO-, -C₂F₄-, -CF=CF- ou une liaison simple.

9. Milieu LC selon l'une quelconque des revendications 4 à 8, **caractérisé en ce que** les composés polymérisables de la formule I sont polymérisés.

10. Affichage LC comprenant un ou plusieurs composé(s) de la formule I tels que définis selon une ou plusieurs des revendications 1 à 3 ou comprenant un milieu LC tel que défini selon l'une quelconque des revendications 4 à 9.

11. Affichage LC selon la revendication 10, lequel est un affichage du type PSA.

12. Affichage LC selon la revendication 11, lequel est un affichage PS-VA, PS-OCB, PS-IPS, PS-FFS, PS-UB-FFS, PS-posi-VA ou PS-TN.

13. Affichage LC selon la revendication 11 ou 12, **caractérisé en ce qu'**il comprend deux substrats, dont au moins l'un est transparent vis-à-vis de la lumière, une électrode qui est prévue sur chaque substrat ou deux électrodes qui sont prévues sur seulement l'un des substrats et, localisée entre les substrats, une couche en un milieu LC, comprenant un ou plusieurs composé(s) polymérisable(s), tels que définis selon l'une quelconque des revendications 4 à 9, dans lequel les composés polymérisables sont polymérises entre les substrats de l'affichage.

14. Procédé pour la fabrication d'un affichage LC selon la revendication 13, comprenant les étapes de remplissage ou de fourniture de tout autre façon d'un milieu LC, comprenant un ou plusieurs composé(s) polymérisable(s), tels que définis selon l'une quelconque des revendications 4 à 9, entre les substrats de l'affichage, et de polymérisation des composés polymérisables.

15. Procédé de préparation d'un milieu LC selon l'une quelconque des revendications 4 à 9, comprenant les étapes de mélange d'un ou de plusieurs composé(s) mésogène(s) ou cristallin(s) liquide(s), ou d'un composant cristallin liquide B) tel que défini selon la revendication 5, avec un ou plusieurs composé(s) de la formule I tels que définis selon une ou plusieurs des revendications 1 à 3, et en option, avec d'autres composés cristallins liquides et/ou des additifs.
